# EUROPEAN PATENT SPECIFICATION

(11) **EP 1 476 136 B1**
(45) Date of publication and mention of the grant of the patent: **14.06.2006**
(21) Application number: 03700915.6
(22) Date of filing: 22.01.2003
(51) Int. Cl.: A61K 9/16, A61K 9/20, A61K 31/535

(54) **PHARMACEUTICAL COMPOSITION COMPRISING N-((1-N-BUTYL-4-PIPERIDINYL)MENTHYL)-3,4-DIHYDRO-2H-(1,3)OXAZINO(3,2-A)INDOLE-10-CARBOXAMIDE OR SALT AND PROCESS FOR PREPARING THEREOF COMPRISING DRY GRANULATION**
PHARMAZEUTISCHE FORMULIERUNG, DIE N-((1-N-BUTYL-4-PIPERIDINYL)MENTHYL)-3,4-DIHYDRO-2H-(1,3)OXAZINO(3,2-A)INDOL-10-CARBOXAMID ODER EIN ENTSPRECHENDES SALZ HIERVON ENTHÄLT, SOWIE DEREN HERSTELLUNGSPROZESS, WELCHER TROCKENGRANULIERUNG BEINHALTET
COMPOSITION PHARMACEUTIQUE COMPRENANT N- ((1-N-BUTYL-4-PIPERIDINYL) METHYL)-3, 4-DIHYDRO-2H- (1, 3) OXAZINO (3, 2-A) INDOLE-10-CARBOXAMIDE OU UN SEL DE CELUI-CI ET PROCEDE ASSOCIE COMPRENANT LA GRANULATION PAR VOIE SECHE

(30) Priority: 14.02.2002 GB 0203528; 14.02.2002 GB 0203526
(43) Date of publication of application: 17.11.2004
(73) Proprietor: GLAXO GROUP LIMITED, Greenford, Middlesex UB6 ONN (GB)
(72) Inventor: BUXTON, Philip C., GlaxoSmithKline, Harlow, Essex CM19 5AW (GB); GROVES, Sharon E., GlaxoSmithKline, Harlow, Essex CM19 5AW (GB); THOMSON, Seona, GlaxoSmithKline, Ware, Hertfordshire SG12 0DP (GB); VAN SCHIE, Dirk M.J., GlaxoSmithKline, Harlow, Essex CM19 5AW (GB); YEATES, Kenneth T., GlaxoSmithKline, Harlow, Essex CM19 5AW (GB)
(74) Representative: Breen, Anthony Paul
(86) International application number: PCT/GB2003/000217
(87) International publication number: WO 2003/068193

(56) References cited:
- EP-A- 1 093 813
- WO-A-02/11733

## Description

This invention relates to a novel process for preparing a pharmaceutical composition, for example a tablet or capsule, comprising SB 207266 or a pharmaceutically acceptable salt thereof, and a pharmaceutical composition obtainable by said process or prepared by said process.

### Introduction

WO 93/18036 (SmithKline Beecham) discloses a large number of condensed indole compounds as 5-HT4 antagonists including, as Example 3 on pages 17-18, N-[(1-ⁿbutyl-4-piperidinyl)methyl]-3,4-dihydro-2H-[1,3]oxazino[3,2-a]indole-10-carboxamide (SB 207266, generic name (International Nonproprietary Name) = piboserod) and its preferred hydrochloride salt (SB 207266-A, piboserod hydrochloride). These compounds are disclosed for use in the treatment or prophylaxis of gastrointestinal, cardiovascular and CNS disorders, in particular irritable bowel syndrome, and in the treatment of urinary incontinence. WO 93/18036 also states in the general description on pp.6-7 in general terms that: "Specific cardiac 5-HT₄ receptor antagonists which prevent atrial fibrillation and other atrial arrhythmias associated with 5-HT would also be expected to reduce the occurrence of stroke". See also US 5,852,014, EP 0 884 319 A2, L.M. Gaster et al, *J. Med. Chem*., 1995, *38*, 4760-4763 and *Drugs of the Future,* 1997, 22(12), 1325-1332 for the compound SB 207266, which is highly selective for the 5HT₄ receptor over other 5HT receptors. The structure of SB 207266 is as follows: 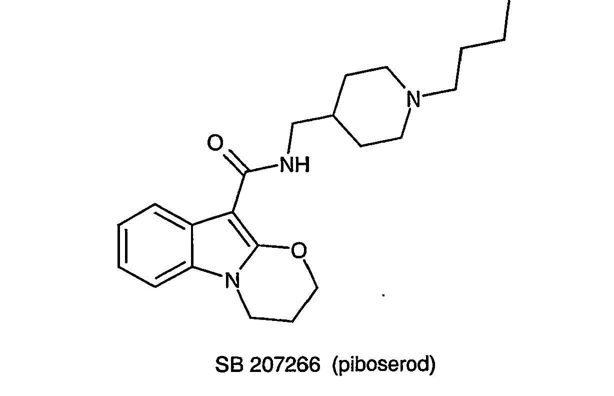 For improved syntheses of SB 207266 and/or a salt thereof, see WO 98/07728, WO 98/11067; WO 00/03983; and WO 00/03984.

There are several methods of making the SB 207266 in free base form or as a hydrochloride salt disclosed in the art. Example 3 on page 17-18 of WO 93/18036 discloses the production of SB 207266 in free base form in Methods 1 and 2. Method 2 also discloses conversion to the HCl salt and recrystallisation from ethanol/60-80 petrol to give a white solid. L. Gaster, *Drugs of the Future,* 1997, 22(12), 1325-1332 discloses a similar method involving HCL salt formation by treatment of SB 207266 free base with anhydrous HCL in ethanol. WO 98/07728 discloses three new methods for making the free base on page 6 line 5 to page 7 line 20. WO 98/07728 also discloses two methods of making the HCl salt (SB 207266-A) - Method A on page 7 line 22 to page 8 line 9, and Method B on page 8 line 10 to page 8 line19. In page 8 lines 10-19 of WO 98/07728, Method B for making the SB 207266 HCl salt is as follows: "N-[(1-Butyl-4-piperidinyl)methyl]-3,4-dihydro-2*H*-[1,3]-oxazino[3,2-*a*]indole-10-carboxamide (SB-207266) (100g, 0.27mol) was dissolved in ethanol (870ml) and the resulting solution filtered to remove particulates. Anhydrous HCl in ethanol (83ml, 3.6M, 0.30mol) was added causing the product to precipitate out of solution. The slurry was heated to redissolve the solid and hexane (550ml) was added. After cooling to room temperature, the mixture was cooled to 0 - 5°C and stirred at that temperature for about two hours. The solid was isolated by filtration and dried *in vacuo* at about 40°C to give the product, N-[(1-butyl-4-piperidinyl)methyl]-3,4-dihydro-2*H*-[1,3]-oxazino[3,2-α]indole-10-carboxamide hydrochloride, (102.8g) in 94% yield."

### The Invention

It has now been recognised that there are problems with certain processes for making the SB 207266 HCl salt, which processes are similar or identical to the process disclosed as Method B in page 8 lines 10-19 of WO 98/07728 in that the HCl salt is dissolved in ethanol, industrial methylated spirits (IMS, e.g. ethanol containing ca. 1% methanol) or similar and crystallised by addition of a C₅-C₁₀ hydrocarbon (e.g. hexane and/or heptane e.g. n-heptane) and/or a solvent containing a C₅-C₁₀ hydrocarbon (e.g. hexane and/or heptane e.g. n-heptane).

The first aspect of the newly recognised problem is that such processes produce the SB 207266 hydrochloride salt in the form of particles of extremely small particle size. For example, the following Table 1 shows the particle size data from batches of the HCl salt (SB-207266-A) made using a process similar to Method B of page 8 of WO 98/07728 but using n-heptane instead of hexane in the crystallisation step (a similar process using IMS instead of ethanol and n-heptane instead of hexane is disclosed in Description 1 hereinafter):

**Table 1**

| **Batch** | **DV 90 (µm)** | **DV 50 (µm)** | **DV 10 (µm)** |
|---|---|---|---|
| BDC-H-01C | 12.8 | 5.3 | 1.4 |
| BDC-G-02C | 13.8 | 5.7 | 1.5 |
| BDC-G-03C | 16.4 | 6.8 | 1.8 |
| BDC-G-04C | 14.4 | 5.3 | 1.4 |
| BDC-G-05C | 14.6 | 5.8 | 1.5 |
| Average | 14.4 | 5.8 | 1.5 |

DV 90, DV 50 and DV 10 respectively mean that 90%, 50% and 10% by volume of the material is less than the micron size specified.

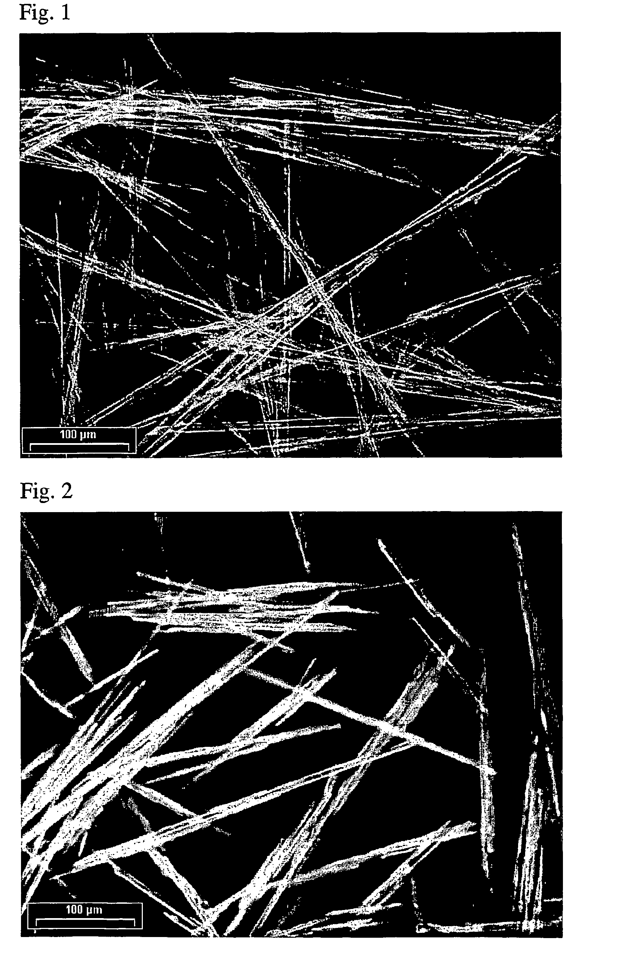

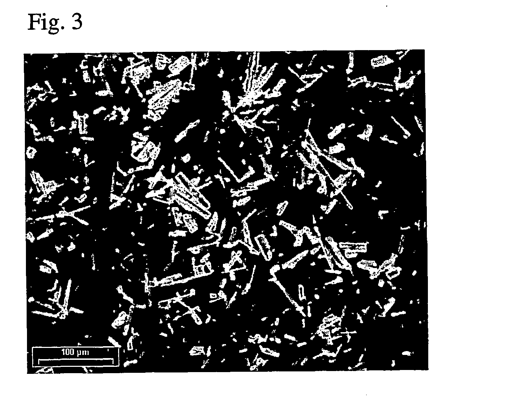

One reason for the small particle size and another aspect of the newly recognised problem is the fact that to date, under normal conditions, the SB 207266 HCl salt has only been produced in needle-shaped crystalline form. For example, as shown in Figs. 1 and 2 and as described in Description 2, when freshly-formed, the needle-shaped crystals of the SB 207266 HCl salt (e.g. >75% or >50% of them by number or by volume or weight) are usually >100 µm or >200 µm in length. However, the needle-shaped crystals crystals (e.g. >75% or >50% of them by number or by volume or weight) are usually <10 µm (e.g. Fig. 1) or <25 µm (e.g. Fig. 2) in width (lateral dimension). Attempts to increase the particle size tend to lead merely to an increase in the length of the needles rather than a more flowable less-elongated crystalline form or crystalline habit.

Processing, e.g. stirring or other movement, of these needle-shaped crystals tends to lead to crystal breakage regenerating shorter needles of small particle size. The shortened / broken needle-shaped crystals are generally (e.g. >50% of them by number or by volume or weight) needle-shaped or otherwise elongated; but usually they (e.g. >50% of the crystals by number or by volume or weight) are <75 µm or <100 µm or <200 µm in length and/or <10 µm or <25 µm in width, as shown for example in Fig. 3 and as described in Description 2.

The second aspect of the newly recognised problem, is the discovery that the SB 207266 HC1 salt produced by these processes (WO 98/07728 and/or Descriptions 1 and/or 2 herein) is very cohesive and has poor flowability / flow characteristics.

The third aspect of the newly recognised problem is that at above certain concentrations in a pharmaceutical formulation, this cohesive drug material causes the composition to be sufficiently poorly-flowing that it cannot easily be tabletted or made into capsules, when the SB-207266 HCl salt is combined with microcrystalline cellulose, mannitol and magnesium stearate excipients. It has been found that a composition for SB 207266, for human oral administration, containing: SB-207266 HCl salt (ca. 5.0 mg measured as free base), microcrystalline cellulose (30.0 mg), mannitol (112.0mg) and magnesium stearate (3.0 mg), with total tablet weight = ca. 150 mg, is possible to tablet using standard direct compression tabletting technology wherein the SB-207266 HCl salt is present at ca. 3.3% by weight of tablet. However, higher concentrations of the SB-207266 HCl salt in this type of formulation are not easily tabletted using direct compression. This is particularly relevant as the clinical maintenance dose of SB-207266 for treatment or prophylaxis of atrial fibrillation is now thought likely to be about 20, 50 or 80 mg/day (see the clinical protocol Example and the tablet Examples hereinafter); whereas the clinical doses of SB-207266 tried previously for treatment of irritable bowel syndrome were only 0.05, 0.25, 1 and 5 mg/day, and 20 mg/day as four 5 mg tablets given together per day.

The fourth aspect of the newly recognised problem is that the small-particle size SB-207266 HCl salt has a low bulk density (e.g. see table in Description 1 hereinafter), densifying on the addition of water. This means that less material can be added to processing machinery of fixed volume, leading to a less efficient manufacturing process as large volumes of equipment have to be used for relatively small volumes of drug (smaller throughput in plant).

Co-pending patent application PCT/GB01/03590 filed on 8 August 2001 by SmithKline Beecham p.l.c. et al., and published on 14 February 2002 as WO 02/11733 A1, discloses that some or all of these problems can be at least partly overcome or mitigated by the forming the SB 207266 HCl salt into granules which have a particle size larger than that of the original SB 207266 HCl salt. These granules were found to have better flow characteristics for e.g. tabletting purposes. Some or all of these advantages of granulated formulations are also expected to be gained for the free base which is believed also to have usually a small-particle size. For example, the free base is very slow to filter when crystallised by the addition of hexane to a toluene solution (e.g. as in Method A on page 6 lines 19-23 and Method C on page 7 lines 14-20 of WO 98/07728). Similarly pharmaceutically acceptable salts other than the HCl salt are thought to benefit too from granulation.

The SB 207266 granulation process disclosed in the description - in particular Examples 4 and 5 - of PCT/GB01/03590 (copending, filed on 8 August 2001, published on 14 February 2002 as WO 02/11733 A1), and also disclosed in Examples 7 and 8 of PCT/GB01/03544 (copending, filed on 7 August 2001, published on 14 February 2002 as WO 02/11766 A2), is a "wet granulation" process, that is the granules of the SB 207266 or salt thereof are formed in the presence of a granulating solvent such as water and/or ethanol. The wet granulation process disclosed in Example 4 of PCT/GB01/03590 (WO 02/11733 A1) is disclosed hereinafter as the "Comparative Example" for comparison with the present invention. As can be seen, the SB-207266 is blended with certain excipients in a high-shear mixer granulator, and water (as the granulation solvent) is added to the stirred granulator to effect the wet granulation. The resulting granules after drying have superior flow characteristics and are more dense than the pure SB207266 HCl salt, and can be compressed, e.g. together with extragranular excipient(s), at medium or high drug concentrations (% of drug by weight of the tablet) to give excellent tablets.

However, one or more of the Inventors have now encountered some occasional unexpected problems with this WO 02/11733 A1 "wet granulation" process, which forms the fifth aspect of the newly recognised problem on which the present invention is based. Surprisingly, on closer observation, non-homogeneous granulation (specifically, physically non-homogeneous granulation) and/or excessive agglomeration is occasionally found to occur during wet granulation. It has been found that as the water (as granulation solvent) is poured onto the drug-excipient mix, the water droplets are almost instantly absorbed/adsorbed by that part of the granulation mix local to the water droplet, which often leads to shrinkage and formation of a locally partly-dissolved mass which forms "balls" by attracting adjacent material. These balls, generally doughy when wet and hard when dry, stick to the walls of the mixer. The balls, especially after drying, result in localised hard lumps of semi-granulated drug-containing material. The extent of ball / lump formation tends to increase as wet granulation time increases and as the drug concentration in the granulation mix increases. The microcrystalline cellulose was used in Example 4 of WO 02/11733 A1 during wet granulation as a granulation aid to disperse the water granulating solvent to an extent during granulation. However, surprisingly, microcrystalline cellulose has been found to be not as effective at dispersing water throughout the granulation mix (to achieve a homogeneous granulation) as is normally the case in wet granulation procedures.

Without being bound by theory, it is thought that these unexpected problems are caused by a combination of the very small particle size and very high water-solubility of the SB-207266 HCl salt which leads to an extremely high rate of solubilisation of the SB 207266 or salt thereof in the water granulation solvent. The localised SB 207266 or salt thereof is thought to dissolve extremely rapidly on contact with the water droplets, leading to the localised "balls" or large hard (when dry) lumps of semi-granulated material described above. When dry, these large lumps are hard and difficult to reduce to a small size, because the SB 207266 or salt thereof which is semi-dissolved acts as a strong binder to surrounding particles of excipients or active ingredient. (This binding property also tends to lead to quite long tablet disintegration times after tabletting).

Also, because much of the water is concentrated in the balls/lumps, there remains after the granulation some unchanged SB 207266 or salt thereof which has not been granulated, densified and made more flowable; i.e. the mixture after granulation is surprisingly physically non-homogeneous.

These problems were minimal or manageable in 2.5 kg batches processed according to the Comparative Example hereinafter. However, on a larger (e.g. 14 kg) scale, ball/lump formation and unchanged SB 207266 or salt after granulation appear to occur more commonly and/or to a greater extent. Therefore, the risk of non-homogeneous mixtures might increase as the wet-granulated formulation is scaled-up to a production scale, though careful choice of large-scale granulation equipment and/or processes might mitigate this. This increased "balling" on scale-up might be at least partly caused by the optional use on scale-up of increased impeller energy in the high-shear mixer-(wet)granulator as the vessel volume increases.

It has now been found by the inventors that the unexpected non-homogeneity problems of the wet granulation process of WO 02/11733 A1 can be mitigated by employing a dry granulation process to make SB-207266 formulations (pharmaceutical compositions). Dry granulation avoids or reduces/mitigates (a) solubilisation of the drug during processing and/or (b) "ball" or hard lump formation. It is therefore surprisingly found that a dry granulation process can lead to a more robust and scalable formulation than the wet-granulated formulation of WO 02/11733 A1. The risks of expensive formulation batch failures in the factory (e.g. due to "balling") are thought to be reduced, and it is likely that fewer process-control measures and/or less care are desirable/needed to minimise the risk of batch failures, compared to wet granulation. Dry granulation can also lead to shorter distintegration times than wet granulated formulations, as explained above. Dry granulation also lends itself to continuous processing and automation, compared to wet granulation.

Therefore, **a first aspect of the invention** provides a process for preparing a pharmaceutical composition comprising N-[(1-ⁿbutyl-4-piperidinyl)methyl]-3,4-dihydro-2H-[1,3]oxazino[3,2-a]indole-10-carboxamide (SB 207266) (piboserod) or a pharmaceutically acceptable salt thereof in combination with one or more pharmaceutically acceptable excipients,
the process comprising forming part or all of the SB 207266 or the salt thereof into granules by a dry granulation process,
and wherein the SB 207266 or the salt thereof is present in the composition in at least 4 weight % by weight of the composition.

The pharmaceutical composition prepared by this process is believed to be novel per se over the wet granulated formulation disclosed in Examples 4 and 5 of PCT/GB01/03590 (WO 02/11733 Al).

Therefore, **a second aspect of the invention** provides a pharmaceutical composition obtainable by a process as defined in the first aspect of the invention.

**A third aspect of the invention** provides a pharmaceutical composition which has been prepared by a process as defined in the first aspect of the invention.

**A fourth aspect of the invention** provides a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable excipients, wherein part or all of the SB 207266 or the salt thereof is present in granules obtainable or prepared by a dry granulation process, and wherein the SB 207266 or the salt thereof is present in the composition in at least 4 weight % by weight of the composition.

By "dry granulation process" is meant a process in which granules are formed substantially in the absence of an externally applied granulating solvent such as water and/or ethanol. For example, the granules are formed in the presence of preferably less than 2% by weight, more preferably less than 1% by weight, still more preferably less than 0.3% by weight, most preferably less than 0.1% by weight, of an externally applied granulating solvent such as water and/or ethanol. In a preferable embodiment of the invention, the dry granulation process of the invention is a process in which granules are formed wholly in the absence of an externally applied granulating solvent such as water and/or ethanol. The dry granulation processes of the invention include those where a solvent such as water is present in a form bound to the drug and/or bound to any of the excipients, for example as water of crystallisation (e.g. as with CaHPO₄.2H₂O).

"Granules" have the meaning understood by a skilled formulation scientist in the context of pharmaceutical formulations (for example as understood in the context of the Handbook of Pharmaceutical Granulation Technology, ed. D.M. Parikh, 1997, Marcel Dekker Inc.). "Granules" in this context excludes particles of pure SB 207266 or salt thereof in a form which has not been subject to a particle-size-increasing process, and "granules" for example excludes SB 207266 HCl salt as directly prepared by Description 1 hereinafter and having the particle size according to Table 1.

N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof is herein often referred to as "the SB 207266 or the salt thereof', "piboserod or the salt thereof", "the active substance", "the active ingredient" or "the drug", or similar.

### Preferred process features of the first, second, third and fourth aspects of the invention

Preferably, the process of the invention also comprises mixing some or all of the SB 207266 or the salt thereof with one or more pharmaceutically acceptable excipients (intragranular excipients) before dry granulation. The one or more intragranular excipients preferably comprise a lubricant; this allows lubrication during the dry granulation step and minimises process difficulties such as adherence of the granulated ingredients to metal machinery parts in e.g. a roller compactor. The one or more intragranular excipients preferably comprise a filler (diluent) and/or a compression aid. The one or more intragranular excipients can optionally comprise a binder and/or a disintegrant. The intragranular lubricant, filler, compression aid, binder and/or disintegrant can be as defined herein.

Preferably, the dry granulation process of the invention comprises compression of (i.e. application of pressure to) and/or compaction (i.e. densification) of the SB 207266 or the salt thereof. The process preferably increases the particle size (e.g. mean, median (D50), D90, and/or D10 particle size) of the SB 207266 or the salt thereof by compression together of particles of drug, optionally also with particles of intragranular excipients, to form larger granules.

In comparison, wet granulation utilises a granulation solvent to make the granulation mix which forms granules after drying, but usually without the use of compression.

Preferably, the dry granulation process of the invention comprises roller compaction of the SB 207266 or the salt thereof. Alternatively, the dry granulation process can comprise slugging of the SB 207266 or the salt thereof. For these alternative "roller compaction" and "slugging" compaction processes, see the "Handbook of Pharmaceutical Granulation Technology", ed. D.M. Parikh, 1997, Marcel Dekker Inc., New York, Chapter 6 "Roller Compaction Technology", especially pages 119-132 (IV. Roller Compactor Design), and pages 103-107, 112, and 115-116. For roller compaction, see also for example the Fitzpatrick Company "Roll Compaction" brochure and the paper "Introduction to Roll Compaction and the Fitzpatrick Chilsonator", both of which are available from: the Fitzpatrick Company internet site at www.fitzpatrick.be; and/or available from The Fitzpatrick Company Europe N.V., Entrepotstraat 8, B-9100 Sint-Niklaas, Belgium, fax: +32/3-766-10-84, email: Fitzpatrick_Europe@compuserve.com; and/or available from The Fitzpatrick Company, 832 Industrial Drive, Elmhurst, IL 60126, USA, fax. +1-630-530-0832, www.fitzmill.com.

Roller compaction typically involves feeding by gravity and/or by a screw feed the pre-granulation powder to be compacted into the gap ("roll gap") between a pair of counterrotating rollers (rolls) having substantially parallel longitudinal axes. Both rolls can be fixed on their axes. More preferably the axes of the rolls can be moveable with respect to each other, for example the axis of one roll being fixed to the compactor frame and the other roll axis being moveable relative to the compactor frame.

The rolls can have smooth outer circumferential surfaces but preferably have textured outer circumferential surfaces such as radial sine wave rolls or more preferably interlocking knurled rolls. The textured outer circumferential surfaces of the rolls preferably comprise corrugations (peaks and valleys) aligned generally in the direction of the roll axis ("corrugated rolls"). More preferably, the corrugated rolls are interlocking knurled rolls; these rolls comprise a first and second roll wherein the outer circumferential corrugations (peaks and valleys) of the first roll are alignable with and/or partly or wholly interlockable with the outer circumferential corrugations (valleys and peaks) of the second roll when the rolls are in circumferentially-opposed relation. For the corrugated rolls (e.g. interlocking knurled rolls), the peaks of the corrugations can e.g. be sharp, rounded or flattened in cross-section, but preferably the peaks of the corrugations are rounded in cross-section. For corrugated rolls, the corrugations maximise the amount of time ("dwell time") that the drug spends in the "nip zone" (see below) of the rolls, maximising de-aeration and compaction of the SB-207266 or its salts, compared to smooth rolls, for this particular drug.

During roller compaction, pressure, e.g. hydraulic pressure, is applied to one or both of the rolls, usually to the moveable-axis roll (floating roll), in a direction so as to urge the rolls together. By this means, the compaction pressure is delivered to the rolls. The powder fed towards the roll gap rubs against the roll surfaces rotating towards the roll gap and is then drawn into the "nip angle area" or "nip zone" near the roll gap where pre-densification takes place. The powder then passes between the rolls and receives the compaction pressure delivered to the rolls; the powder is thereby compacted (densified).

During roller compaction, the pre-granulation powder to be compacted is preferably fed into the roll gap by a rotating screw feed (e.g. auger screw feed) adjacent and directed towards the rolls. In one embodiment, the rolls can be substantially horizontal and substantially level with each other and a "vertical screw feed" positioned above and directed towards the rolls can be used. In another embodiment, the rolls can be substantially horizontal and positioned one above the other, and a "horizontal screw feed" substantially level with and directed towards the roll gap can be used.

An optional preliminary screw feed, e.g. a horizontal screw feed, can be used to meter the product (the pre-granulation blend containing drug) from an inlet hopper into a pre-compression (pre-compaction) stage. The preliminary screw feed speed (e.g. horizontal preliminary screw feed speed) is typically from 0 to about 62 revolutions per minute (rpm) and/or preferably is at least about 10 rpm or at least about 15 rpm or at least about 20 rpm and/or preferably is up to about 40 rpm or up to about 62 rpm. The preliminary screw feed speed is more preferably from about 10 rpm to about 62 rpm, or from about 20 rpm to about 62 rpm; still more preferably from about 10 rpm to about 40 rpm or from about 15 rpm to about 40 rpm; for example about 30 rpm (e.g. Example 11), about 17 to about 20 rpm (e.g. Example 12), or about 20 rpm (e.g. Example 13).

A main screw feed adjacent the rolls (downstream from any optional preliminary screw feed), preferably a vertical main screw feed, is preferably used e.g. to perform pre-compression and/or de-aeration (pre-compaction) of the pre-granulation powder and to feed the powder to the rolls. The main screw feed speed (e.g. vertical main screw feed speed) can for example be up to 600 rpm but is typically from 0 to about 270 rpm, preferably from about 30 to about 270 rpm or from about 30 to about 100 rpm, more preferably from about 40 to about 90 rpm. For example, the main screw feed speed can be about 100 rpm, or about 70 rpm (e.g. Example 11), or about 49 to about 53 rpm (e.g. Examples 12 and 13). The main screw feed speed is important for good compaction as this feed delivers material directly to the rolls.

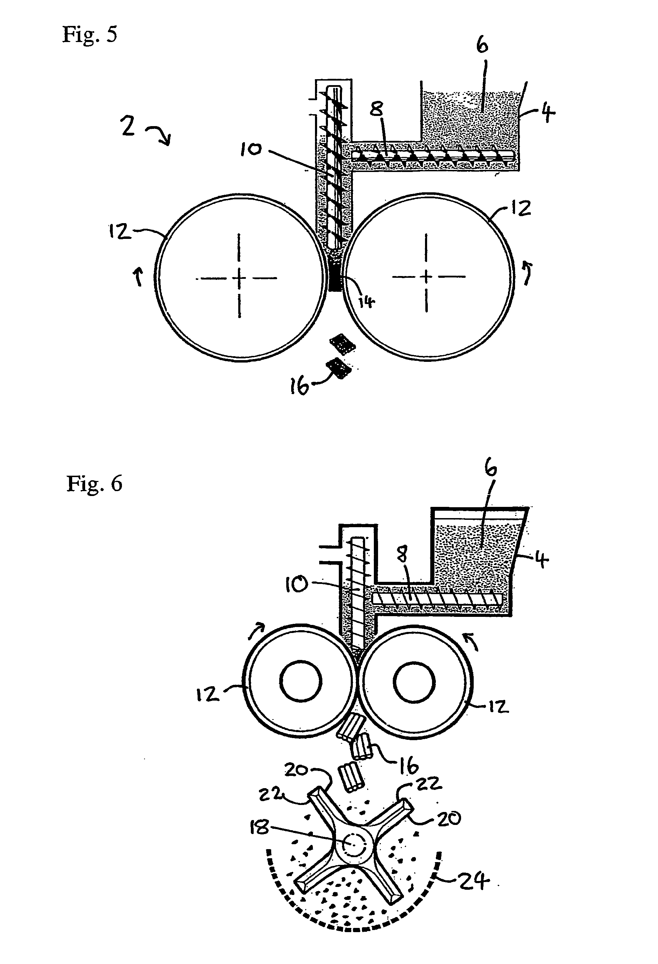

An example of a roller compactor comprising rolls which are substantially horizontal and substantially level with each other, a vertical main screw feed positioned above, adjacent and directed towards the rolls, and upstream of the vertical main feed a horizontal preliminary screw feed is: for example the Fitzpatrick Chilsonator IR220 Roller Compactor available from Fitzpatrick Company (described in more detail in step 2 of Example 11 and shown in Fig. 5).

Where a preliminary screw feed and main screw feed are both used, the ratio of the preliminary screw feed speed to the main screw feed speed is typically from 1 : 30 to 1 : 1 or from 1 : 10 to 1 : 1.2, preferably from 1 : 5 to 1 : 1.5, more preferably from 1 : 4 to 1 : 1.5, most preferably from 1 : 3.5 to 1 : 2. This ratio helps with smooth flow of material from the hopper through preliminary feed and main feed to the rolls, without too much or too little material accumulating between the two feeds.

The flow of drug-containing material thorough one or more feeds and into the rolls can optionally be assisted by formation of a vacuum, e.g. by vacuum connection to one or more of the feeds, in particular by vacuum connection to the (main) screw feed which feeds material directly to the rolls. This assists in de-aeration and pre-compaction, and e.g. can be useful for high intragranular concentrations of the present drug (the SB-207266 or the salt thereof) which has a low density.

The roll pressure can for example be from about 2000 to about 20000 pounds per linear inch (pli) (from about 3.51 to about 35.1 kN / cm), but is typically from about 2000 to about 16500 pounds per linear inch (pli) (from about 3.51 to about 28.93 kN / cm). The roll pressure is suitably from about 8000 to about 16500 pli or from about 8000 to about 20000 pli (from about 14.0 to about 28.93 kN / cm or from about 14.0 to about 35.1 kN / cm), for example about 8000 pli (about 14.0 kN / cm). More preferably, the roll pressure is from about 20 kN / cm to 28.93 kN / cm, and is ideally about 24 to about 26 kN / cm (e.g. see Examples 11-13).

The roll speed can be typically from 0 to about 17 rpm, but is suitably from about 1.5 rpm to about 17 rpm or about 1.5 rpm to about 10 rpm, for example about 10 rpm. Slow roll speeds are found to increase the dwell time and increase drug compaction desirably, but too slow speeds can risk jamming the drug-containing material in the rolls. So, preferably, the roll speed is about 1.5 rpm to about 7 rpm, more preferably about 2 rpm to about 6 rpm, still more preferably about 2.5 rpm to about 5.5 rpm, most preferably about 3 to about 5 rpm (e.g. see Examples 11-13).

Perhaps due to the low density of the drug (SB207266 or salt), it has been found (e.g. in going from Example 12 to Example 11) that as the drug concentration in the pre-granulation blend increases, the flow rate through the screw feed(s) decreases. In this case, to obtain an ideal compact it is preferable to increase the preliminary screw feed speed and/or the main screw feed speed to provide an ideal material feed rate to the rolls, and/or to increase the dwell time in the rolls. Therefore, in particular when the SB-207266 or the salt thereof is present in the granules (i.e. during dry granulation) in at least 22% or at least 27% or at least 32% or at least 35% of the granules (intragranular ingredients), then:
- preferably the preliminary screw feed speed, if such a feed is present, is at least about 20 rpm or at least about 25 rpm or at least about 30 rpm, e.g. from about 20 rpm to about 62 rpm, still more preferably from about 25 rpm to about 62 rpm or from about 25 rpm to about 40 rpm, for example about 30 rpm (e.g. Example 11 and 14); and/or
- preferably the main screw feed speed (e.g. vertical main screw feed speed) is at least about 56 rpm or at least about 60 rpm or at least about 65 rpm or or at least about 70 rpm, for example from about 56 rpm either to about 600 rpm or to about 270 rpm, and is more preferably from about 56 to about 100 rpm or from about 60 to about 90 rpm, and most preferably is from about 60 to about 80 rpm, for example from about 60 to about 70 rpm (e.g. Example 11 and 14); and/or
- preferably the roll speed is up to about 5 rpm or up to about 4 rpm or up to about 3.5 rpm and/or preferably is at least about 1.5 rpm; more preferably the roll speed is from about 1.5 rpm to about 4 rpm, still more preferably about 2 rpm to about 4 rpm, most preferably about 2.5 rpm to about 3.5 rpm, e.g. about 3 rpm (e.g. see Examples 11 and 14).

The roll gap can for example be about 0.5 to about 2 mm, e.g. about 0.7 to about 1.3 mm.

The roller-compacted mixture as it exits the rollers is called a "compact", "flake" or "ribbon".

Preferably, the ribbon density is from about 1.0 to about 1.5 g / cc (g/ml).

The granules formed by the dry granulation process are preferably milled to a particle size suitable for use in tablets or capsules. Preferably, e.g. for roller compaction, the compact (flake, ribbon) exiting from the rollers (rolls) is milled to a particle size suitable for use in tablets or capsules, e.g. using a comminuting mill. For example, the granules can be milled such that they pass through sieve or screen with a 0.110 inch (2.80 mm), 0.097 inch (ca. 2.46 mm), 0.093 inch (2.36 mm), 1.70 mm, 0.065 inch (1.65 mm), 0.063 inch (1.60 mm), 0.055 inch (1.40 mm), 0.032 inch (0.81 mm), 500 µm, or 250 µm hole size. The granules can be passed through such a sieve or screen during or after milling. Preferably, the sieve has a 0.110 inch (2.80 mm) to a 0.032 inch (0.81 mm) hole size, more preferably a 0.097 inch (ca. 2.46 mm) to 0.055 inch (1.40 mm) hole size or a 0.097 inch (ca. 2.46 mm) to 0.063 inch (1.60 mm) hole size; this is best e.g. for tablets incorporating the granules.

In use, the mill, e.g. comminuting mill, can for example be rotating at 1000 to 10000 rpm or 3000 to 8000 rpm, e.g. about 5000 rpm.

A preferable comminuting mill is one with hammers and/or knives. As known to the skilled person, this type of comminuting mill comprises a chamber with an entry port (usually upwardly positioned) for entry of the granules, a sieve or screen (usually downwardly-positioned) for exit of milled granules, and disposed therebetween in the chamber a rotatable spindle projecting from which are a plurality of generally radially disposed blades which generally have a sharp or "knife" edge as one rotationally-directed edge and a flat or "hammer" edge as the other opposed rotationally-directed edge. One example is shown in the bottom part of Fig. 6, wherein the spindle (rotatable in either direction) is 18, the knives are 20, the hammers 22, and the sieve / screen is 24. Depending on the direction of rotation of the spindle, the "knife" edges ("knives") and/or the "hammer" edges ("hammers") of the spindle blades are "forward" (i.e. form the leading edge which during rotation impacts the granules/particles in the mill chamber) - usually it is knives forward or hammers forward. Optionally the mill can be a comminuting mill with "hammers forward", but preferably the mill is a comminuting mill with "knives forward". The "knives forward" mode tends to control the granule particle size more (e.g. less fines) and is generally better for tablets. For example, a FitzMill L1A (as described in Example 1) comminuting mill (e.g. available from Fitzpatrick, see Example 1 for address), with hammers and/or knives forward, preferably using a 0.065 inch or 0.093 inch (2.36 mm) screen, and preferably rotating at 5000 rpm, can be used.

Another alternative type of mill is a "rasping mill". This mill comprises a rotor comprising a central rotatable or oscillatable shaft attached (e.g. by generally radial struts) to a plurality of bars or members directed generally in the direction of the central shaft axis but spaced apart from the central shaft. The bars are generally all spaced apart from the central shaft axis by approximately the same distance. This mill also comprises usually a curved "rasping screen" which is spaced apart from and radially-outside the rotor bars (usually spaced closely to the bars) and capable in use of sifting or rasping the granules between the rotating or oscillating bars and the rasping screen thereby to reduce their particle size. The rasping screen usually has generally part-circular cross-section and is co-axial with the central rotor shaft; and/or can be of the hole size as described elsewhere.

Another alternative type of mill is a rotor-granulator (oscillating granulator).

Another alternative type of comminuting mill can comprise a frustoconical screen (e.g. 0.093 inch (2.36 mm), 1.70 mm, 0.065 inch (1.65 mm), 0.055 inch (1.40 mm), 0.032 inch (0.81 mm), or 500 µm hole size) forming the wall of the mill and a coaxial axially-rotatable frustoconical impeller closely-spaced to the screen to crush granules poured into the gap between screen and the rotating impeller. Once crushed to the necessary size, the granules can escape through the holes in the screen.

Optionally, the mill can be integral with the roller compactor, e.g. as shown in Fig. 6.

Whatever type of mill is used, it is preferable that the tapped density of the dry granulated (e.g. roller compacted) and milled granules is about 1.0 to about 1.5 g/cc (g/ml) and/or about 0.8 to about 1.3 g/ml, more preferably (e.g. for better dissolution) about 0.8 to about 1.2 g/ml, still more preferably about 1.0 to about 1.2 g/ml.

Optionally, all the milled material can be used during formation of the pharmaceutical composition (e.g. tablet or capsule). Alternatively and preferably, the milled granules can be "classified", that is a portion thereof with a predetermined particular particle size range can be separated (selected) e.g. for incorporation into the composition, by excluding material above a predetermined particle size (e.g. by sieving) and/or by excluding material below a predetermined particle size (e.g. by sieving). For example, the milled granules can be passed through two (or more) sieves of progressively decreasing hole size, and e.g. for 2 sieves the material passing through the first sieve (e.g. a 2.00 or 1.00 or 0.85 or 0.81 mm sieve) but retained by the second sieve (e.g. a 75 or 106 or 150 or 180 micron (µm) sieve) can be selected e.g. for incorporation into the composition. The large granules retained by the first sieve and/or the small granules passing through the second sieve can optionally be recycled e.g. by feeding them into the pre-granulation stage / hopper.

The milled and/or "classified" granules can have the particle size distribution as hereinafter described.

Preferably, after formation of the granules (e.g. after roller compaction) and optional milling to a suitable size, the granules are then (i) optionally mixed with one or more pharmaceutically acceptable excipients (extragranular excipients) and (ii) optionally compressed into tablets or filled into capsules. Such extragranular excipient(s) preferably include a disintegrant and/or a lubricant, and/or optionally include a compression aid and/or a filler (diluent) and/or a binder, e.g. as defined herein.

### Preferred composition features of the first, second, third and fourth aspects of the invention

Preferably, the granules contain one or more pharmaceutically acceptable intragranular excipients.

Preferably, 90% or more or 95% or more by weight, or substantially all or all, of the SB 207266 or the salt thereof is present in the granules obtainable or prepared (formed) by the dry granulation process.

Preferably the pharmaceutical composition is orally administrable (capable of being administered orally), e.g. orally administrable to a human.

Preferably the pharmaceutical composition is a tablet(s) (for example a swallow tablet), or the invention can be a capsule containing the pharmaceutical composition. The tablet(s) can for example be round in major cross-section, but preferably, the tablet(s) is/are oval in major cross-section (longitudinal cross-section).

Preferably, 50% or more by weight or by volume of the granules including the SB 207266 or the salt thereof have a particle size of: ≥ 75 microns (micrometres) e.g. 75 to 1000 or 75 to 500 microns, more preferably ≥ 100 microns (micrometres) e.g. 100 to 1000 or 100 to 500 microns, more preferably ≥ 106 microns e.g. 106 to 1000 or 106 to 500 microns, more preferably ≥ 150 microns e.g. 150 to 1000 or 150 to 500 microns, still more preferably ≥ 200 microns e.g. 200 to 1000 or 200 to 500 microns. Preferably, the granules including the SB 207266 or the salt thereof have a particle size defined by a "D50", or median particle size, e.g. by weight (DM50) or by volume (DV50), of ≥ 100 microns or one of the other above-specified preferred size ranges.

Preferably, 70% or more by weight or by volume of the granules including the SB 207266 or the salt thereof have a particle size of: ≥ 40 microns e.g. 40 to 1000 or 40 to 500 microns, preferably ≥ 50 microns e.g. 50 to 1000 or 50 to 500 microns, more preferably ≥ 53 microns e.g. 53 to 1000 or 53 to 500 microns, still more preferably ≥ 63 microns e.g. 63 to 1000 or 63 to 500 microns, most preferably ≥ 75 microns e.g. 75 to 1000 or 75 to 500 microns.

Preferably, 90% or more by weight or by volume of the granules including the SB 207266 or the salt thereof have a particle size of: ≥ 10 microns (micrometres) e.g. 10 to 1000 microns, more preferably ≥ 20 microns e.g. 20 to 1000 or 20 to 500 microns, still more preferably ≥ 50 microns e.g. 50 to 1000 or 50 to 500 microns, yet more preferably ≥ 53 microns e.g. 53 to 1000 or 53 to 500 microns, most preferably ≥ 75 microns e.g. 75 to 500 microns. Preferably, the granules including the SB 207266 or the salt thereof have a particle size defined by a "D10", e.g. by weight (DM10) or by volume (DV10), of ≥ 10 microns or one of the other above-specified preferred size ranges. As an alternative definition, preferably 10% or less by weight or by volume of the granules including the SB 207266 or the salt thereof have a particle size of: ≤ 10 microns (micrometres), more preferably ≤ 50 microns, still more preferably ≤ 75 microns.

Compositions of the invention containing granules with the above-mentioned medium to large particle sizes are generally less cohesive, flow better, and usually are well or acceptably well compressible into tablets, and are thus less likely to cause the above-mentioned formulation problems. A large percentage of particles with particle sizes of less than 75 or 100 microns might for example lead to tablet-compression and/or uniformity problems, and a large percentage of particles with particle sizes of more than 1000 microns might cause dissolution problems.

Preferably, 50% or more by weight or by volume of the particles of the SB 207266 or the salt thereof (e.g. before forming into granules and/or after granule formation; e.g. within the granules) have a particle size of ≤ 75 microns (micrometres), more preferably ≤ 50 or ≤ 53 or ≤ 63 microns, still more preferably ≤ 20 microns, even more preferably ≤ 10 microns, most preferably ≤ 8 microns. In other words, this means that preferably the particles of the SB 207266 or the salt thereof (e.g. before forming into granules and/or after granule formation; e.g. within the granules) have a particle size defined by a "D50", or median particle size, e.g. by weight (DM50) or by volume (DV50), of ≤ 75 microns or ≤ 50 or ≤ 53 or ≤ 63 microns or one of the other above-specified preferred size ranges.

Preferably, 10% or more by weight or by volume of the particles of the SB 207266 or the salt thereof (e.g. before forming into granules and/or after granule formation; e.g. within the granules) have a particle size of ≤ 20 microns (micrometres), more preferably ≤ 10 microns, still more preferably ≤ 5 microns, even more preferably ≤ 2.5 microns, most preferably ≤ 2 microns. In other words, this means that preferably the particles of the SB 207266 or the salt thereof (e.g. before forming into granules and/or after granule formation; e.g. within the granules) have a particle size defined by a "D10", e.g. by weight (DM10) or by volume (DV10), of ≤ 20 microns or one of the other above-specified preferred size ranges.

Preferably, 90% or more by weight or by volume of the particles of the SB 207266 or the salt thereof (e.g. before forming into granules and/or after granule formation; e.g. within the granules) have a particle size of ≤ 100 microns (micrometres), more preferably ≤ 75 or ≤ 53 or ≤ 50 microns, still more preferably ≤ 20 microns. In other words, this means that preferably the particles of the SB 207266 or the salt thereof (e.g. before forming into granules and/or after granule formation; e.g. within the granules) have a particle size defined by a "D90", e.g. by weight (DM90) or by volume (DV90), of ≤ 100 microns, more preferably ≤ 75 or ≤ 53 or ≤ 50 microns, still more preferably ≤ 20 microns.

As discussed above, SB 207266 or salts with such small particle sizes are the ones most likely to give the problems above-mentioned, and are most likely to benefit from the present invention.

In general, particle sizes (D50, D10, D90, et al.) can be measured by sieving with one or more sieves (e.g. for granules before further processing into tablets, and/or for measuring the powder inside capsules). Suitable sieves include 53, 63, 75, 90, 106, 125, 150, 180, 212, 250, 300, 355, 425, 500, 600, 630, 710, 810, or 850 micron (µm) sieves, or 1.00, 1.18, 1.40, 1.60, 1.65, 1.70, 2.00, 2.36, 2.46, 2.80, 3.35, or 4.00 mm sieves.

Alternatively, particle sizes can be measured by laser diffraction, also known as low angled laser light scattering (LALLS). Laser diffraction is based on the angular distribution of scattered light. Laser diffraction is known to the skilled person and can use an algorithm based on a Fraunhoefer or Mie optical model also known to the skilled person. Further details of the laser diffraction technique can be found in: Clive Washington, "Particle Size Analysis in Pharmaceutics and Other Industries, Theory and Practice", Ellis Horwood Limited, 1992, see in particular Chapter 6, p.109-133. The Fraunhoefer calculation is described therein and is commonly performed by the software analysis package provided as part of commercially available laser diffraction apparatus e.g. as now described. Suitable laser diffraction apparatus include (a) the Malvern Mastersizer S, obtainable from Malvern Instruments Limited, Enigma Business Park, Grovewood Road, Malvem, Worcestershire WR14 1XZ, United Kingdom, email: www.malvern.co.uk; and (b) the Sympatec HELOS/QUIXEL, obtainable from Sympatec UK and Ireland, Bury Business Centre, Kay Street, Bury BL9 6BU, United Kingdom, email: sympatec.uk@btinternet.com.

Alternatively, particle sizes can be measured directly, (for example optically e.g. by microscope, or otherwise), particularly in a tablet. For example, particle sizes can be so measured in a section through the tablet (for example obtained by breaking or cutting a tablet into 2 pieces and observing the cross-sectional face); diameters of specific particles can be measured which enables an estimation of the particle size distribution by volume and thence by weight.

Particle size analysis methods typically assume sphericity of particles in the calculation of the distribution. In cases where non-spherical particles are analysed, skilled interpretation is required to understand the influence that shape may have on skewing the size distribution. Particle sizing techniques that utilise images of the particles such as microscopy can, however, accurately infer particle shape and size, though typically size would still be expressed assuming sphericity.

Preferably, the SB 207266 or the salt thereof (e.g. the HCl salt) is of a form obtainable by, e.g. preferably is made by, a process in which the SB 207266 or the salt (e.g. HCl salt) is dissolved in a C₁₋₄alcohol to form a solution and is crystallised from the solution by addition of a C₅-C₁₀ hydrocarbon (e.g. hexane and/or heptane e.g. n-heptane) and/or a solvent containing a C₅-C₁₀ hydrocarbon (e.g. hexane and/or heptane e.g. n-heptane). The C₁₋₄alcohol can comprise or be: methanol, propanol (e.g. isopropanol), butanol (e.g. n-butanol or t-butanol), and/or ethanol or an ethanol-containing solvent such as industrial methylated spirits (IMS, e.g. ethanol containing ca. 1% methanol). Ethanol or an ethanol-containing solvent is preferred. Such processes often form SB 207266 or salts with small particle sizes - at least for the HCl salt - which products are most likely to give the problems above-mentioned, and are which most likely to benefit from the present invention.

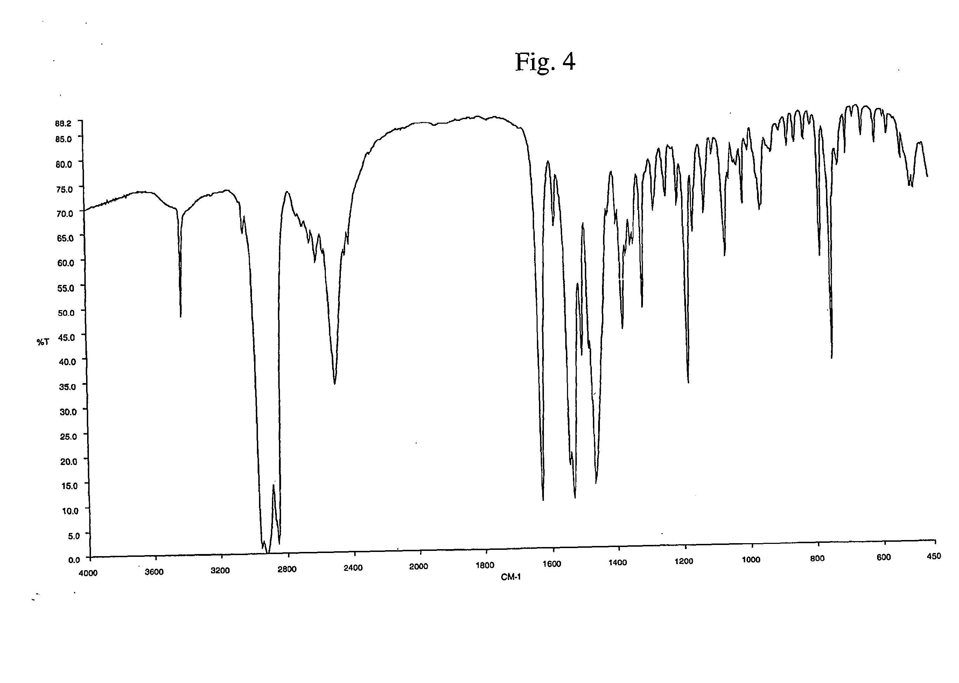

Preferably, the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises (e.g. is) the hydrochloride salt of SB 207266 (SB 207266-A), more preferably the needle-shaped crystalline form of the hydrochloride salt of SB 207266 and/or a or the substantially anhydrous crystalline form of the hydrochloride salt of SB 207266. The needle-shaped crystalline form can e.g. optionally be as described above, and/or can e.g. be substantially as shown in one or more of Figures 1, 2 and 3 in particular Fig. 3 and/or substantially as described in Description 2, and/or can e.g. be defined by >50% of the crystals by number or by volume or weight being needle-shaped or otherwise elongated; and/or can e.g. be defined by >50% of the crystals by number or by volume or weight being <75 µm or <100 µm or <200 µm in length and/or <10 µm or <25 µm in width. The needle-shaped crystalline form is a substantially anhydrous (e.g. anhydrous) crystal form of SB-207266-A. So the SB-207266-A preferably comprises (e.g. 70% or more or 80% or more or 90% or more is), or consists essentially of or is, a substantially anhydrous or anhydrous crystalline form thereof. The water of hydration content can be measured by known methods such as the Karl Fischer method (e.g. as described in the US Pharmacopeia, (e.g. 1990 edition, pages 1619-1621) or as described in the European Pharmacopeia (e.g. 2nd edition, 1992, part 2, 16th fascicule at v. 3.5.6-1). The hydrochloride salt of SB 207266 (e.g. the needle-shaped and/or anhydrous crystalline form) preferably has an infrared (IR) spectrum (nujol mull) substantially as shown in Fig. 4; and/or has an infrared (IR) spectrum (nujol mull) with three, four, five, six or more (e.g. all) of the following peaks: 3423, 3044, 2502, 1628, 1582, 1502, 1531, 1184, 748 cm⁻¹ (some peak variation is allowed, e.g. ± about 2 cm⁻¹ or ± about 1 cm⁻¹); and/or has an infrared (IR) spectrum (nujol mull) with three, four, five or more (e.g. all) of the following peaks: 1628, 1582, 1502, 1531, 1184, 748 cm⁻¹ (some peak variation is allowed, e.g. ± about 2 cm⁻¹ or ± about 1 cm⁻¹). See e.g. Description 2 for a description of Fig. 4.

Preferably, the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises (e.g. is) the crystalline form of the hydrochloride salt of SB 207266 characterised by having an infrared (IR) spectrum (nujol mull) substantially as shown in Fig. 4; and/or having an infrared (IR) spectrum (nujol mull) with three, four, five, six or more of the following peaks 3423, 3044, 2502, 1628, 1582, 1502, 1531, 1184, 748 cm⁻¹ (± about 2 cm⁻¹).

The SB 207266 or the salt thereof is present in the composition in at least 4 weight % by weight of the composition. Preferably, the SB 207266 or the salt thereof is present in the composition and/or in the granules in at least 4 weight % or at least 4.4 weight% or at least 5 weight % or at least 6 weight % or at least 7 weight % or at least 8 weight % or at least 11 weight % or at least 15 weight % or at least 20 weight %, by weight of the composition and/or by weight of the granules respectively. Preferably, the SB 207266 or the salt thereof is present in the composition and/or in the granules in up to 95 weight %, more preferably up to 70 weight % or up to 60 weight %, most preferably up to 50 weight % or up to 40 weight %, by weight of the composition and/or by weight of the granules respectively. Most preferably, the SB 207266 or the salt thereof is present in the composition and/or in the granules in 4.4-95 or 6-95 or 6-88% or 11-70% or 11-60% or 15-60%, by weight of the composition and/or by weight of the granules respectively. For example, e.g. from Examples 1-9, the SB 207266 or the salt thereof is present in the composition in from 4.4 wt% to 35.2 wt%. These preferred percentages are calculated as the actual weight of the SB 207266 or the salt thereof including any counterions or added-acids (e.g. HC1 for the HCI salt) which are present.

For example, for every 250mg of weight of composition (e.g. for every 250 mg coated or uncoated tablet weight), the composition ideally comprises: about 10 to about 150 mg or about 11 to about 150 mg (e.g. 10, 11, 22, 27.5, 33, 44, 55, 82.5, 88, 110 or 132 mg) of the SB 207266 or the salt thereof such as the hydrochloride salt (calculated as the actual weight including counterions or added-acids); or about 10 to about 120 mg (e.g. 10, 20, 25, 30, 40, 50, 75, 80, 100 or 120 mg) of the SB 207266 or the salt thereof (calculated as the free base).

Preferably, the composition is in a unit dose form (preferably a tablet or capsule, e.g. a 250 mg tablet by uncoated tablet weight). In this case, preferably a unit dose form (e.g. a tablet or a capsule) being or comprising the composition comprises: about 10 to about 150 mg, or about 11 to about 150 mg, or 44 to 132 mg, or 44 to 110 mg, or 82.5 to 110 mg (e.g. 10, 11, 22, 27.5, 33, 44, 55, 82.5, 88, 110 or 132 mg, preferably 55 or 88 mg) of the SB 207266 or the salt thereof such as the hydrochloride salt (calculated as the actual weight including counterions or added-acids). Preferably, a unit dose form (e.g. a tablet or a capsule) being or comprising the composition comprises: about 10 to about 120 mg, or 40 to 120 mg, or 40 to 100 mg, or 75 to 100 mg (e.g. 10, 20, 25, 30, 40, 50, 75, 80, 100 or 120 mg, preferably 50 or 80 mg) of the SB 207266 or the salt thereof (calculated as the free base).

Preferably, the granules containing the SB 207266 or the salt thereof also contain a filler (diluent). Mixing the filler with the SB 207266 or the salt thereof before granulation often aids formation of granules. Granulating pure SB 207266 or a salt can be difficult.

Preferably, the filler (diluent) is abrasive. This helps to alleviate the cohesiveness of the SB 207266 or the salt, and aids the flowability of the granules.

Preferably, the filler is brittle (as opposed to elastic or plastic). Brittleness can be determined by tests known to the skilled man such as compaction simulation tests which for example determine Young's modulus of the filler.

Preferably, the filler (diluent) is insoluble, practically insoluble, very slightly soluble or slightly soluble (more preferably insoluble or practically insoluble) in water and/or ethanol and/or isopropanol. The terms "practically insoluble", "very slightly soluble" and/or "slightly soluble" can be as defined in the British Pharmacopoeia, the European Pharmacopoeia and/or the US Pharmacopoeia. "Practically insoluble" according to the British Pharmacopoeia 1999 (page 11) means that at least 10 litres of the solvent is required to dissolve 1 gram of the filler / solute (e.g. at ambient temperature, e.g. 15 or 20 or preferably 25 °C). "Very slightly soluble" according to the British Pharmacopoeia means that at least 1 litre and up to 10 litres of the solvent is required to dissolve 1 gram of the filler / solute (e.g. at 25 °C). "Slightly soluble" according to the British Pharmacopoeia means that at least 100 ml and up to 1 litre of the solvent is required to dissolve 1 gram of the filler / solute (e.g. at 25 °C). "Soluble" according to the British Pharmacopoeia 1999 means that from 10 to 30 ml of the solvent is required to dissolve 1 gram of the solute at ambient temperature (e.g. 15 to 25 °C). "Freely soluble" according to the British Pharmacopoeia means that from 1 to 10 ml of the solvent is required to dissolve 1 gram of the solute (e.g. at 25 °C). "Very soluble" according to the British Pharmacopoeia means that less than 1 ml of the solvent is required to dissolve 1 gram of the solute (e.g. at 25 °C).

Preferably, the filler comprises (e.g. is) any pharmaceutically acceptable metal (e.g. calcium or magnesium) salt which is insoluble, practically insoluble, very slightly soluble or slightly soluble (preferably insoluble) in water and/or ethanol. The salt can for example be a phosphate, hydrogen phosphate, carbonate, hydrogen carbonate or lactate salt. Such insoluble-to-slightly soluble salts include calcium phosphate, dibasic calcium phosphate (calcium hydrogen phosphate), calcium carbonate, magnesium carbonate, magnesium phosphate, calcium lactate (e.g. the pentahydrate), etc.; so the filler can comprise (e.g. is) one or more of these salts.

Preferably, the filler comprises (e.g. is) dibasic calcium phosphate (i.e. dicalcium phosphate, calcium hydrogen phosphate, CaHPO₄). More preferably, the filler comprises (e.g. is) dibasic calcium phosphate hydrate e.g. dihydrate (i.e. calcium hydrogen phosphate hydrate, e.g. the dihydrate or CaHPO₄.2H₂O). Anhydrous dibasic calcium phosphate can also be used. CaHPO₄, e.g. hydrated or anhydrous, is abrasive and helps to alleviate the cohesiveness of the SB 207266 or salt; and it is insoluble in water. Alternatively or additionally, the filler can comprise calcium phosphate, i.e. tribasic calcium phosphate, Ca₃(PO₄)₂. Calcium hydrogen phosphate can act as a flow aid e.g. during dry granulation; it generally aids roller compaction, and it is dense.

Optionally, a fine grade filler (having a mean or median particle size of ca. 5 to 30 µm, for example 5 to 20 µm or about 9 to about 15 µm) can be used. For example, fine grade CaHPO₄ (dihydrate or anhydrous) such as Calipharm ^{TM}, e.g. Calipharm D (dihydrate) or Calipharm A (anhydrous), as disclosed e.g. in the Handbook of Pharmaceutical Excipients, 3rd edn, 2000; or fine grade Ca₃(PO₄)₂ can optionally be used.

However, it is preferable to use a coarse grade filler, i.e. filler having a mean or median particle size of: ≥ 50 or ≥ 53 or ≥ 100 or ≥ 106 µm, and/or ≤ 425 or ≤ 300 or ≤ 250 or ≤ 200 µm, e.g. 53-300 µm or 106-300 µm or 106-250 µm), for example: coarse grade CaHPO₄ dihydrate such as Emcompress ™ or DI-TAB ™ (average particle size = ca. 180 µm), or coarse grade CaHPO₄ anhydrous such as Emcompress Anhydrous ™ or A-TAB ™ (average particle size = ca. 136 and ca. 180 µm respectively), as disclosed e.g. in the Handbook of Pharmaceutical Excipients, 3rd edn, 2000. Emcompress ™ and Emcompress Anhydrous ™ is available from Penwest Pharmaceuticals Co. at 801 First Street S.W., P.O. Box 99, Cedar Rapids, IA., USA, or at 2981 Route 22, Patterson, N.Y. 12563, USA. DI-TAB ™ and Calipharm ™ is available from Rodia at: Rhodia Inc., 259 Prospect Plains Road, CN 7500, Cranbury, USA 08512-7500, or at Rhodia Organique, 190 avenue Thiers, 69457 Etoile Part-Dieu, France.

The filler is preferably present in up to 95% by weight of the granules, and/or up to 85% or up to 70% or up to 60% by weight of the composition and/or by weight of the granules. Preferably, the filler is present in ≥ 15 wt% or ≥ 20 wt% or ≥ 30 wt% of the composition and/or by weight of the granules. For example, the filler is preferably present in from 15 to 85% or from 15 to 70% by weight of the composition and/or by weight of the granules. For example (e.g. from Examples 1-8 or Example 9), the filler can be present in from 33.8% to 64.6% and/or from 38.8% to 69.6% by weight of the composition. Preferably, the filler comprises from about 10 to about 90% by weight of the granules. Preferably, the filler is at least partly (e.g. wholly) intragranular.

Preferably, the weight ratio of the filler (e.g. as herein defined) to the drug (i.e. the SB-207266 or the pharmaceutically acceptable salt thereof) in the composition and/or in the granules is at least 1:3, preferably at least 1:2.5 or at least 1:2 or at least 2:3; and/or is preferably ≤ about 10:1 or ≤ 5:1 or ≤ 3:1. For example, the weight ratio of the filler to the drug in the granules can preferably be from 1:3 to about 10:1 (e.g. from 1:3 to 10:1) or from 1:2 to about 10:1 (e.g. from 1:2 to 10:1), e.g. from 2:3 to 10:1 or from 1:3 to 3:1 or from 1:2 to 3:1 or from 2:3 to 3:1. Suitably, the weight ratio of the filler to the drug in the composition and/or in the granules is as hereinabove defined, wherein the filler comprises (e.g. is) one or more of calcium phosphate, calcium hydrogen phosphate (e.g. hydrate and/or anhydrous), calcium carbonate, magnesium carbonate, magnesium phosphate and calcium lactate (e.g. pentahydrate); more preferably the filler comprises (e.g. is) calcium phosphate and/or calcium hydrogen phosphate (e.g. hydrate and/or anhydrous).

The filler (e.g. as herein defined, e.g. CaHPO₄) can be intragranular, extragranular, or part-intragranular and part- extragranular; see e.g. Examples 1-3 for CaHPO₄. Preferably, at least part of (i.e. some or all of, e.g. 50% or more or 70% or more or 90% or more thereof) the filler is intragranular, e.g. see Examples 2-9. More preferably the filler is wholly intragranular, e.g. see Example 2, Examples 4-10 modifying Example 2, and Examples 11-16. If the filler is at least partly present intragranularly (i.e. during the dry granulation process), then preferably the intragranular ratio of filler to drug (i.e. the ratio during dry granulation) is from about 1:10 to about 10:1, preferably at least 1:3 or at least 1:2 or at least 1:2.5 or at least 2:3 or ≥ 1:1, and/or is preferably ≤ 5:1 or ≤ 3:1. More preferably, the intragranular ratio of filler to drug, i.e. the ratio during dry granulation, is from 1:3 to about 10:1 (e.g. from 1:3 to 10:1) or from 1:2 to about 10:1 (e.g. from 1:2 to 10:1) or from 1:3 to 3:1, for example from 1:1 to about 10:1 or from 1:2 to 3:1 or from 1:1 to 3:1.

Preferably, the composition includes an excipient which acts as a compression aid, for example comprising or being microcrystalline cellulose (MCC). The compression aid is preferably present in at least 3 weight % (wt%) or at least 5 wt% and/or ≤ 60 wt% or ≤ 50 wt% or ≤ 30 wt% by weight of the composition and/or by weight of the granules, more preferably at least 10 wt% or at least 15 wt% by weight of the composition and/or by weight of the granules, still more preferably 10-50 wt% or 15-50 wt% or 15-30 wt% (e.g. about 20 wt%) by weight of the composition and/or by weight of the granules.

Preferably, the compression aid comprises (e.g. is) microcrystalline cellulose (MCC) having a nominal mean particle size of about 25 µm to about 150 µm, more preferably about 50 µm to about 100 µm. Suitable grades of MCC include Avicel PH-102 (nominal mean particle size of about 100 µm) and Avicel PH-101 (nominal mean particle size of about 50 µm) available from FMC Corporation.

Alternatively, mannitol and/or lactose (e.g. compressible lactose, e.g. anhydrous lactose or spray-dried lactose) could be used as the compression aid. Such compression aids, which could also be classified as fillers, are classified as compression aids for the purposes of this patent specification. CaHPO₄ and similar metal salts (e.g. as described above) are classified as fillers.

"Compression aid" means an excipient which aids in overall compressibility, for example during the dry granulation process and/or during any compression into tablets. For example, MCC acts to help plastic deformation when tabletting, and aids roller compaction.

The compression aid can be present inside the granules (i.e. intragranular) and/or outside the granules (i.e. extragranular). The compression aid can be present inside (intragranular) and/or outside (extragranular) the granules of the composition. Preferably, the compression aid is at least partly (e.g. wholly) intragranular.

Preferably, the intragranular weight ratio, i.e. the weight ratio during the dry granulation process, of the filler (e.g. as herein defined e.g. calcium hydrogen phosphate) to the compression aid (e.g. microcrystalline cellulose such as Avicel PH-102 or PH-101) is ≥ 15:1 or ≥ 7:1 or ≥ 5:1 or ≥ 4:1 or ≥ 3:1 or ≥ 5:2, and/or is preferably ≤ 1:3 or ≤ 1:2 or ≤ 2:3 or ≤ 1:1 or ≤ 3:2. For example, the intragranular weight ratio of the filler to the compression aid can be from 7:1 to 1:2, preferably from 5:1 to 2:3, more preferably from 4:1 to 1:1, still more preferably from 3:1 to 3:2 or from 5:2 to 3:2, and most preferably about 2 : 1 (e.g. from 2.2 : 1 to 1.8 : 1, from 2.1 : 1 to 2.0 : 1, or from 2.07 : 1 to 2.04 : 1).

Optionally, the composition can include a binder. The binder acts to bind the drug (SB207266 or a salt thereof) onto the other intragranular ingredients, increasing the strength of the granules so that for example when compressed they form stronger bonds. The binder is preferably a cellulosic binder for example comprising or being hydroxypropylmethylcellulose (HPMC) (e.g. low viscosity HPMC such as Pharmacoat 603, made by Shinogi, Japan). Other possible cellulosic binders can include hydroxypropylcellulose (HPC), hydroxyethylcellulose (HEC), hydroxymethylcellulose (HMC), methyl cellulose (e.g. low to medium viscosity), ethyl cellulose, etc. Another suitable binder includes povidone (polyvinylpyrrolidone, PVP; this is an essentially linear, non-crosslinked polymer, see Handbook of Pharmaceutical Excipients, 3rd edn, 2000), for example K25, K30, K60 or K90 grade povidone and/or povidone having about 50,000 to about 1,000,000 molecular weight. The binder can preferably be present in about 1 to about 10 weight % of the composition, for example about 2.5 to about 10 weight % or about 1 to about 5 weight % (e.g. about 5 wt%) of the composition. HPMC is preferably present in about 5 wt%. The binder can be present inside (intragranular) and/or outside (extragranular) the granules of the composition (the intragranular and extragranular options do not exclude the possibility that a portion of the binder is present in the non-stated region).

Preferably, however, the composition includes no HPMC binder; more preferably the composition includes no binder (e.g. see Examples 9 and 11-16).

Preferably, the composition includes a disintegrant (e.g. tablet disintegrant) such as sodium starch glycollate (e.g. Primojel or Explotab ™, the latter being available from Penwest Pharmaceuticals Co. at 801 First Street S.W., P.O. Box 99, Cedar Rapids, IA., USA, or at 2981 Route 22, Patterson, N.Y. 12563, USA), croscarmellose sodium (e.g. Ac-Di-Sol ™), or crospovidone (cross-linked polyvinylpyrrolidone). The disintegrant can be preferably present in about 1 to about 10 weight % of the composition, for example about 2.5 to about 10 weight % or or about 3.7 to about 10 weight % or about 5 to about 10 weight % or about 1 to about 5 weight % (e.g. about 5 wt%) of the composition. Sodium starch glycollate is preferably present in about 5 wt%. The disintegrant can be present inside (intragranular) and/or outside (extragranular) the granules of the composition (the intragranular option and the extragranular option do not exclude the possibility that a portion of the disintegrant is present in the non-stated region). Preferably, the disintegrant is present at least partly (e.g. wholly) extragranularly.

Preferably, the composition includes a lubricant, for example comprising or being an alkaline earth metal stearate such as calcium stearate or more preferably magnesium stearate. The lubricant can be present in preferably about 0.2 to about 5 weight % or more preferably about 0.2 to about 2 weight % or about 0.5 to about 2 weight % (e.g. about 1 wt% or about 2 wt%) by weight of the composition and/or by weight of the granules. Preferably, at least part of the lubricant is present inside the granules (intragranular) (which does not exclude the possibility that a portion of the lubricant is present outside the granules). This allows lubrication during the dry granulation step and minimises process difficulties such as adherence of the granulated ingredients to metal machinery parts in e.g. a roller compactor. More preferably, the lubricant can be present both intragranularly and extragranularly. Optionally, the intragranular lubricant is present in about 1% to about 35%, or about 1% to about 25%, or more preferably about 1% to about 12%, by weight of the granules.

The granules (i.e. the intragranular ingredients) can optionally form about 2% to 99.8% by weight or about 2% to about 99% by weight, for example about 4% to about 95 % by weight or about 4% to about 75% by weight or about 2% to about 75% by weight, of the composition. The granules (i.e. the intragranular ingredients) preferably form from about 50% to 99.8% or from about 75% to 99.8% by weight of the composition. More preferably, the granules form from about 50% to about 99% or from about 75% to about 99% by weight of the composition. For example the granules can form from about 90% to 99.8%, or from about 90% to about 99%, or from about 95% to about 99% by weight of the composition. Alternatively, the granules can form 100% by weight of the composition (i.e. no extragranular excipients).

**A fifth aspect of the invention** provides a process for preparing a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable excipients (carriers), wherein the SB 207266 or the salt thereof is present in the composition in at least 4 weight % by weight of the composition,
the process comprising:
(a) dissolving the SB 207266 or the salt thereof in ethanol or an ethanol-containing solvent to form a solution,
(b) crystallising the SB 207266 or the salt thereof from the solution by addition of a C₅-C₁₀ hydrocarbon (e.g. hexane and/or heptane) and/or a solvent containing a C₅-C₁₀ hydrocarbon (e.g. hexane and/or heptane), and
(c) forming at least some of the SB 207266 or the salt thereof into granules by a dry granulation process.

The ethanol-containing solvent can be industrial methylated spirits (IMS, e.g. ethanol containing ca. 1% methanol).

The dry granulation process and/or the excipient(s) can be as described herein.

In this fifth aspect of the invention, it is particularly preferable that the SB 207266 or the salt thereof comprises (e.g. is) the hydrochloride salt of SB 207266, e.g. the needle-shaped crystalline form thereof.

SB 207266 or the salt thereof may conveniently be administered by any of the routes conventionally used for drug administration, for instance, parenterally, orally, topically or by inhalation.

Procedures for making the composition and/or tablet and/or capsule may involve mixing, granulating and compressing the ingredients as appropriate to the desired preparation.

The excipient(s)/carriers used in the composition should be "pharmaceutically acceptable" in the sense of being compatible with the other ingredients of the formulation and not deleterious to the recipient thereof.

The pharmaceutically acceptable carrier employed may be, for example, a solid. Exemplary of solid carriers are lactose, terra alba, sucrose, talc, gelatin, agar, pectin, acacia, magnesium stearate, stearic acid and the like. Similarly, the carrier may include time delay material well known to the art, such as glyceryl mono-stearate or glyceryl distearate alone or with a wax.

A wide variety of pharmaceutical forms can be employed. Thus, if a solid carrier is used, the preparation can be tableted, or can be placed in a hard gelatin capsule in powder or pellet form or in the form of a troche or lozenge. The amount of solid carrier will vary widely but preferably is from about 25 mg to about 1 g.

### Utility / industrial application

The pharmaceutical composition containing SB 207266 or a salt thereof obtainable or produced by the process of the present invention can be used in the treatment or prophylaxis of atrial arrhythmias such as atrial fibrillation (AF), and/or in the treatment or prophylaxis of atrial remodelling. Atrial fibrillation is preferred. In particular, it is thought that compositions such as tablets containing SB 207266 or a salt thereof can be administered to patients with symptomatic persistent atrial fibrillation (AF) in order to inhibit symptomatic recurrences of atrial fibrillation in these patients. A proposed clinical protocol is given in Example 17 hereinafter.

Therefore the invention also describes a method of treatment or prophylaxis of atrial arrhythmia, such as atrial fibrillation, compising administering to a mammal (e.g. human) in need of such treatment or prophylaxis an effective amount of a pharmaceutical composition as defined herein or obtainable or produced by the process as defined herein. The invention also describes a method of inhibiting symptomatic recurrences of atrial fibrillation in a mammal (e.g. human) with symptomatic persistent atrial fibrillation compising administering to the mammal an effective amount of a pharmaceutical composition as defined herein or obtainable or produced by the process as defined herein.

SB 207266 compositions might also reduce the occurrence of stroke in AF patients. SB 207266 compositions might also be useful in the treatment and/or prophylaxis of urinary incontinence, and/or other uses as disclosed in WO 93/18036.

### EXAMPLES

The invention will now be described by reference to the following Descriptions and Examples which are merely illustrative and which are not to be construed as a limitation of the scope of the present invention which is defined in particular by the Claims.

The Descriptions exemplify some non-limiting methods by which SB 207266 and/or its hydrochloride salt can be made; other methods are possible. The non-limiting Examples (other than the Comparative Example) exemplify dry granulation processes for preparing a pharmaceutical composition comprising SB 207266 or a pharmaceutically acceptable salt thereof, starting from the SB 207266 or the pharmaceutically acceptable salt thereof, and exemplify the dry granulated pharmaceutical compositions so prepared, according to embodiments of the invention.

The Examples and/or Descriptions are described partly by reference to the Figures, in which:
Fig. 1 is a scaled micrograph (photograph) showing the initial stages of formation of needle-shaped crystals of N-[(1-ⁿbutyl-4-pipendinyl)methyl]-3,4-dihydro-2*H*-[1,3]-oxazino[3,2-*a*]indole-10-carboxamide hydrochloride (SB-207266-A), during the crystallisation step of Description 2. Long thin needles are evident.
Fig. 2 is a scaled micrograph showing the final stages of formation of the needle-shaped crystals of SB-207266-A shown in Fig. 1, later on in the crystallisation step of Description 2. The needles have grown wider.
Fig. 3 is a scaled micrograph showing the SB-207266-A crystals of Fig. 2 after they have been stirred, washed and transferred to a vacuum oven for drying in Description 2. The long needles of Fig. 2 have been broken into shorter crystals.
Figs. 1 to 3 are illustrative and, unless otherwise indicated, are not to be construed as a limitation of the scope of the present invention which is defined in particular by the Claims. Different crystal formation and/or crystal transformation results, and different crystal dimensions, are possible and are within the scope of the invention unless indicated otherwise.
Fig. 4 is an infrared (IR) nujol mull spectrum of the needle-shaped substantially anhydrous crystalline form of SB-207266 hydrochloride salt (SB-207266-A) as produced by Descriptions 1 and 2, and described in Description 2.
Fig. 5 is a cross-sectional drawing of certain parts of a Fitzpatrick Chilsonator IR220 Roller Compactor of the sort used in Examples 1 and 11 and as described in more detail in step 2 of Example 11
Fig. 6 is a cross-sectional drawing of certain parts of a Fitzpatrick Chilsonator IR220 Roller Compactor (above) and (below) a FitzMill L1A comminuting mill having a central shaft / spindle 18 with projecting knives 20 and hammers 22 and a sieve / screen 24, and as described in detail in the description hereinbefore.

### Descriptions

SB 207266 - N-[(1-ⁿbutyl-4-piperidinyl)methyl]-3,4-dihydro-2H-[1,3]oxazino[3,2-a]indole-10-carboxamide - can be made using the synthetic methods described in the introduction, e.g. preferably as described in one or more of WO 98/07728, WO 98/11067; WO 00/03983; and/or WO 00/03984.

For a method of making the SB 207266 hydrochloride salt from the free base, see in particular Method B in page 8 lines 10-19 of WO 98/07728 and minor variations thereof which are described in full in the "Introduction" and "The Invention" sections above (e.g. see page 2 hereinabove). One minor and wholly equivalent variation of the WO 98/07728 Method B is given in detail in the following Description 1, in which industrial methylated spirits (IMS) is used instead of ethanol and n-heptane is used instead of hexane in the crystallisation step. In Descriptions 1 and 2, the specific type of IMS used was ethanol containing ca. 1% methanol. Description 2 gives an alternative method of making the SB 207266 hydrochloride salt from the free base, using acetyl chloride instead of anhydrous HCl.

### Description 1: N-[(1-ⁿbutyl-4-piperidinyl)methyl]-3,4-dihydro-2H-[1,3]-oxazino[3,2-a]indole-10-carboxamide hydrochloride; anhydrous HCl method

N-[(1-ⁿButyl-4-piperidinyl)methyl]-3,4-dihydro-2*H*-[1,3]-oxazino[3,2-*a*]indole-10-carboxamide (SB-207266) (100g, 0.27mol) was dissolved in industrial methylated spirits (IMS) (825ml) and the resulting solution filtered to remove particulates. Anhydrous HCI in IMS (174ml, 1.7M, 0.29mol) was added causing the product to precipitate out of solution. The slurry was heated to redissolve the solid and n-heptane (550ml) was added. After cooling to room temperature, the mixture was cooled to 0 - 5°C and stirred at that temperature for about one hour. The solid was isolated by filtration and dried *in vacuo* at about 40°C to give the product, N-[(1-ⁿbutyl-4-piperidinyl)methyl]-3,4-dihydro-2*H*-[1,3]-oxazino[3,2-*a*]indole-10-carboxamide hydrochloride (SB-207266-A), (98g) in 89% yield. SB-207266-A Bulk Densities from Description 1 (and/or a variant using ethanol in place of IMS) were low, and were measured as:

| | | |
|---|---|---|
| **Batch** | **Aerated bulk density g/ml** | **Tapped bulk Density g/ml** |
| BDC-H-01c | 0.136 | 0.250 |
| BDC-G-02c | 0.142 | 0.300 |
| BDC-G-03c | 0.173 | 0.339 |
| BDC-G-04c | 0.146 | 0.310 |
| BDC-G-05c | 0.152 | 0.308 |

### Description 2: N-[(1-ⁿbutyl-4-piperidinyl)methyl]-3,4-dihydro-2H-[1,3]-oxazino[3,2-a]indole-10-carboxamide hydrochloride; acetyl chloride method and particle analysis

1. N-[(1-ⁿButyl-4-piperidinyl)methyl]-3,4-dihydro-2H-[1,3]-oxazino[3,2-*a*]indole-10-carboxamide (SB-207266) (20 g, 54 mmol, 1 mole equivalent) is dissolved in industrial methylated spirits (IMS) (160 ml) and the solution filtered into a 500 ml vessel. [It is desirable to use a filter aid to remove inorganics which may be present as a fine powder.] The residue is washed with IMS (20 ml).
2. Filtered acetyl chloride (4.6 ml, 64 mmol, 1.2 mole equivalents) is added slowly at <45°C (e.g. at room temperature e.g. about 15-25 °C) to the filtered solution of SB-207266 in IMS which is also at <45°C (e.g. room temperature). The reaction is exothermic, so the rate of addition of acetyl chloride should be controlled such that the temperature of the reaction mixture remains below the boiling point of acetyl chloride (50°C) and preferably below 45°C. In this embodiment, the acetyl chloride is added over 30 minutes. [Contact of the acetyl chloride with ferrous containing metals should be avoided since this can cause blue colouration of the liquors.] The vessel residues of acetyl chloride are rinsed into the reaction mixture with filtered IMS (20 ml).
3. The resulting mixture is heated to 70-80°C to give a solution. Step 4 can then follow immediately, as soon as a solution has been obtained; or alternatively the hot solution can be held and stirred at 70-80°C for 10 minutes before step 4.
4. Filtered n-heptane (100 ml) at room temperature is added to the hot (60-80°C, e.g. 60-70°C) solution over approximately 1 hour, maintaining the solution temperature at 60-80°C, e.g. 60-70°C. Step 5 can then follow immediately or after a short (e.g. 10 minute) time lag.
5. The mixture is cooled to 0-5 °C, over a time period dependent on the size and cooling capacity of the vessel (e.g. can be cooled over a period of about 30 mins to about 2 hours). The cooled crystal-containing mixture is stirred for 1 hour.
6. The crystalline product is separated from the cooled solvent by filtration, for example using a Buchner funnel, and washed with filtered 1:1 IMS : n-heptane (20 ml). The product is a fine solid which is fairly slow to filter. The product is dried under vacuum at 40°C, for example in a vacuum oven, to give the product, N-[(1-ⁿbutyl-4-piperidinyl)methyl]-3,4-dihydro-2*H*-[1,3]-oxazino[3,2-*a*]indole-10-carboxamide hydrochloride (SB-207266-A, 20.4g, 93% yield) as white crystals. Yields can vary, and can typically be about 85 to about 95%. Typical density values: Aerated bulk density = ca. 0.14 g/ml, tapped bulk density = ca. 0.31 g/ml.

Particle analysis: Figs. 1, 2 and 3 illustrate the crystal formation and transformation steps for the hydrochloride salt SB-207266-A, during the various steps of Description 2. It is to be noted that Figs. 1 to 3 are illustrative and, unless otherwise indicated, are not to be construed as a limitation of the scope of the present invention which is defined in particular by the Claims. Different crystal formation and/or crystal transformation results, and different crystal dimensions, are possible and are within the scope of the invention unless indicated otherwise.

Fig. 1 illustrates the initial stages of crystal formation, which happens during and/or after the 1-hour addition of n-heptane at 60-70°C (step 4), and/or during the cooling from 60-70°C to 0-5 °C (step 5). It can be seen that thin long (generally >100 µm) needle-shaped crystals of SB-207266-A are initially produced.

Fig. 2 illustrates the final stages of crystal formation, at the end of the 1-hour stirring at 0-5 °C in step 5. Long (>100 µm) needle-shaped crystals of SB-207266-A are still evident, but these have a slightly greater width (diameter) due to crystal growth.

As shown in Figs. 1 and 2, the needle-shaped crystals of of SB-207266-A formed (e.g. >75% of them by number or by volume or weight) are generally >100 µm or >200 µm in length. However, the needle-shaped crystals (e.g. >75% of them by number or by volume or weight) are usually <10 µm (e.g. Fig. 1) or <25 µm (e.g. Fig. 2) in width (lateral dimension)

Fig. 3 illustrates the SB-207266-A crystals after they have been stirred, washed and transferred to the vacuum oven for drying. It can be seen that the long needles of Fig. 2 have been broken into much shorter crystals which generally (e.g. >50% of them by number or by volume or weight) are still needle-shaped or otherwise elongated but <75 µm or <100 µm or <200 µm in length. Some very small crystals are evident in Fig. 3.

The particle size analysis of the broken / shortened crystals of Fig. 3 is thought to be similar to that shown in Table 1.

Spectroscopic data: Fig. 4 shows an infrared (IR) spectrum (a nujol mull spectrum) for the crystalline hydrochloride salt SB-207266-A formed from Descriptions 1 and/or 2. This is a substantially anhydrous crystal form of SB-207266-A. IR peaks due to the SB-207266-A are seen at wavenumber 3423, 3044, 2502, 1628, 1582, 1502, 1531, 1184, 748 cm⁻¹ (accurate to about: ± 2 cm⁻¹ or ± 1 cm⁻¹). The peaks at ca. 2960-2850, ca. 1466, ca. 1327 and ca. 721 cm⁻¹ are thought to be due to the nujol. The tentative assignment of the IR peaks of the SB-207266-A is as follows:

| Wavenumber (cm⁻¹) | Assignment |
|---|---|
| 3423 | N-H amide bond stretch |
| 3044 | C-H (Aromatic) bond stretch |
| 2502 | N⁺-H bond stretch |
| 1628 | C=O bond stretch |
| 1582 and 1502 | ring mode |
| 1531 | Amide II |
| 1184 | C-O bond stretch |
| 748 | C-H (4H) out of plane bond deformation |

Modification of Description 2: In an alternative embodiment of Description 2, especially preferable on a larger scale, instead of using a Buchner funnel and vaccum oven, the crystalline product is separated by filtration, washed, and vacuum-dried in a single piece of apparatus (namely a filter-dryer), optionally with slow agitation (e.g. mechanical stirring) of the crystalline product e.g. during drying.

### Comparative Example - tablets made by wet granulation

Tablets containing the hydrochloride salt of SB 207266 (SB 207266-A) in amounts of 10, 25 or 40 mg (measured as the free base) were made according to the composition in the table below. This process and composition is not according to the present invention, and is Example 4 of WO 02/11733 A1.

| **Comparative Example Composition** | | | | |
|---|---|---|---|---|
| *Ingredient* | *Function* | *Quantity (mg*/*tablet)* | | |
| | | 10 mg tablet strength | 25 mg tablet strength | 40 mg tablet strength |
| *Active Ingredient* | | | | |
| SB-207266-A (hydrochloride) | API | 11.0* | 27.5* | 44.0* |
| *Other Ingredients* | | | | |
| Microcrystalline Cellulose (e.g. Ph. Eur. or NF) | Compression & granulation aid | 50.0 | 50.0 | 50.0 |
| Hydroxypropylmethyl cellulose (e.g. USP) (e.g. Pharmacoat 603) | Binder | 12.5 | 12.5 | 12.5 |
| Sodium starch glycollate (e.g. NF or Ph Eur) | Disintegrant | 12.5 | 12.5 | 12.5 |
| Calcium hydrogen phosphate dihydrate (Dibasic Calcium Phosphate dihydrate) (e.g. Ph. Eur. or USP) | Major diluent | 161.5 | 145.0 | 128.5 |
| Magnesium Stearate (e.g. Ph.Eur. or NF) | Lubricant | 2.5 | 2.5 | 2.5 |
| Purified Water ** (e.g. Ph. Eur. or USP) | Granulating solvent | ** | ** | ** |
| | | | | |
| Opadry White YS-1-7003 | Film Coat | 6.25 | 6.25 | 6.25 |
| Purified Water ** | | ** | ** | ** * |
| Total Tablet Weight | | 256.25 | 256.25 | 256.25 |

| | | | | |
|---|---|---|---|---|
| * Equivalent to 10, 25, 40 mg respectively of pure free base | | | | |
| ** Removed during processing | | | | |

The SB-207266-A tablets of this Comparative Example are packed into high density polyethylene (HDPE) bottles with plastic, child-resistant, induction seal caps.

The formulation of this Comparative Example used a wet granulation process using an insoluble major excipient, Dibasic calcium Phosphate dihydrate (or Dicalcium phosphate). Dibasic calcium Phosphate dihydrate is the major diluent together with microcrystalline cellulose which is added to disperse the granulating solvent and to aid in the overall compressibility. The binding agent added is hydroxypropylmethyl cellulose and the granulation is carried out in a conventional mixer granulator. The granule mix is dried, screened and mixed with sodium starch glycollate as a disintegrant and magnesium stearate as a lubricant to form the compression mix. Tablets are produced on a suitable rotary tablet press, and can be either oval or round in shape.

### Comparative Example - Detailed Manufacturing Process, In-process Controls, and Assembly Process

SB-207266-A, microcrystalline cellulose, dibasic calcium phosphate dihydrate, and hydroxypropylmethyl cellulose are blended together. Purified water is added to the blended powders while mixing in a high shear mixer-granulator. The granules are dried in a fluid bed drier and are then transferred to a mixer, where they are blended with sodium starch glycollate and magnesium stearate. The lubricated mix is compressed into tablet cores using a rotary tablet press. The tablet cores are film coated using an aqueous dispersion of Opadry White YS-1-7003.

| *Procedure:* | | |
|---|---|---|
| 1.0 | Granulation. | |
| | 1.1 | Blend the SB-207266, microcrystalline cellulose, hydroxypropylmethyl cellulose and dibasic calcium phosphate dihydrate in a suitable high shear mixer-granulator. |
| | 1.2 | Add the purified water to effect the granulation. |
| | 1.3 | Dry the granules in a fluid bed drier. |
| | 1.4 | Pass the dried granules through a stainless steel screen using a suitable mill. |
| | 1.5 | Determine the yield of the granules. |
| 2.0 | Manufacture of Compression Mix. | |
| | 2.1 | Blend the required quantities of sodium starch glycollate and magnesium stearate with the dried granules |
| | 2.2 | Determine the yield of compression mix. |
| 3.0 | Tablet Compression. | |
| | 3.1 | Transfer the compression mix to a suitable tablet machine. |
| | 3.2 | Compress the tablets. |
| | 3.3 | Determine the yield of the compressed tablets. |
| 4.0 | Film Coating. | |
| | 4.1 | Transfer the tablet cores to a suitable coating machine. |
| | 4.2 | Rotate the cores and spray on aqueous dispersion of Opadry. |
| | 4.3 | Release test samples are taken randomly from the batch and appropriately labelled. |
| 5.0 | Bottle filling | |
| | 5.1 | HDPE bottles are filled to the appropriate fill count, induction sealed and fitted with a child resistant cap using suitably automated equipment. |

The following data were recorded for some film-coated tablets according to the Comparative Example:

| | |
|---|---|
| Mean Hardness (Kp) | 13.7 |
| (the core hardness (without coating) is likely to be very approximately 2 Kp lower) | |
| Disintegration Time (mins) | 15 |
| Dissolution Time T75 (mins) | 14.8 |

### EXAMPLES according to the present invention - SB 207266 dry-granulated pharmaceutical compositions

### Example 1 - SB-207266-A tablets made by dry granulation process - SB-207266-A and part of magnesium stearate intragranular

Tablets containing the hydrochloride salt of SB 207266 (SB 207266-A) in amounts of 10, 20, 25, 40, 50 or 80 mg (calculated as the free base) were made according to the composition in Table 2 below.

| **Table 2: Example 1 composition** | | | | | | | |
|---|---|---|---|---|---|---|---|
| *Ingredient* | *Function* | *Quantity - mg*/*tablet (% by weight of uncoated tablet given in brackets)* | | | | | |
| | | 10 mg tablet strength | 20 mg tablet strength | 25 mg tablet strength | 40 mg tablet strength | 50 mg tablet strength | 80 mg tablet strength |
| *Intragranular Ingredients (subject to roller compaction)* | | | | | | | |
| SB-207266-A (hydrochloride salt) | Active ingredient | 11.0 mg* | 22.0 mg* | 27.5 mg* | 44.0 mg* | 55.0 mg* | 88.0 mg* |
| | | (4.4 wt%) | (8.8 wt%) | (11 wt%) | (17.6 wt%) | (22 wt%) | (35.2 wt%) |
| Magnesium stearate (intragranular) | Lubricant | 1.25 mg (0.5wt%) | 1.25 mg | 1.25 mg | 1.25 mg | 1.25 mg | 1.25 mg |
| *Extragranular Ingredients* | | | | | | | |
| Microcrystalline cellulose (e.g. Avicel PH-102) | Compression aid | 50.0 mg (20 wt%) | 50.0 mg | 50.0 mg | 50.0 mg | 50.0 mg | 50.0 mg |
| Hydroxypropylmethyl cellulose | Binder | 12.5 mg (5 wt%) | 12.5 mg | 12.5 mg | 12.5 mg | 12.5 mg | 12.5 mg |
| (HPMC) (e.g. Pharmacoat 603) | | | | | | | |
| Sodium starch glycollate | Tablet disintegrant | 12.5 mg (5 wt%) | 12.5 mg | 12.5 mg | 12.5 mg | 12.5 mg | 12.5 mg |
| Calcium hydrogen phosphate | Diluent | 161.5 mg | 150.5 mg | 145.0 mg | 128.5 mg | 117.5 mg | 84.5 mg |
| dihydrate, coarse grade (e.g. DI-TAB or Emcompress ™) | | (64.6 wt%) | (60.2 wt%) | (58 wt%) | (51.4 wt%) | (47.0 wt%) | (33.8 wt%) |
| Magnesium stearate (extragranular) | Lubricant | 1.25 mg (0.5wt%) | 1.25 mg | 1.25 mg | 1.25 mg | 1.25 mg | 1.25 mg |
| Total uncoated tablet weight | | 250 mg | 250 mg | 250 mg | 250 mg | 250 mg | 250 mg |
| Opadry White YS-1-7003 | Film Coat | 6.25 mg | 6.25 mg | 6.25 mg | 6.25 mg | 6.25 mg | 6.25 mg |
| Total coated tablet Weight | | 256.25 mg | 256.25 mg | 256.25 mg | 256.25 mg | 256.25 mg | 256.25 mg |

| | | | | | | | |
|---|---|---|---|---|---|---|---|
| * Equivalent to 10, 20, 25, 40, 50, 80 mg respectively of pure free base. | | | | | | | |
| All ingredients are in accordance with Ph. Eur. or NF or USP where appropriate. | | | | | | | |

The process used to make the tablets of Example 1 is now described.

The equipment used (for small scale development work up to approximately 1 kg) includes:
- Fitzpatrick Chilsonator IR220 Roller Compactor (described in more detail in step 2 of Example 11 and shown in Fig. 5 and 6)
- FitzMill L1A comminuting mill with knives or hammers and screens (e.g. as described in the description hereinbefore and as shown in the bottom half of Fig. 6, it can be separate from or integral with the roller compactor)

This equipment is made by Fitzpatrick, whose headquarters are in US. The equipment is also available from their European division at: The Fitzpatrick Company Europe N.V., Entrepotstraat 8, B-9100 Sint-Niklaas, Belgium; fax: +32/3-766-10-84; email: Fitzpatrick_Europe@compuserve.com; www.fitzpatrick.be.

### Example 1 - Detailed Process

1. Pass the SB-207266-A and the to-be-intragranular portion of the magnesium stearate through a nominal 1250 micron screen using a vibratory sieve, if required, into a suitable mixer.
2. Blend for 5 minutes at about 17 revolutions per minute (rpm).
3. Load the blend into the hopper of the roller compactor (Fitzpatrick Chilsonator IR220 Roller Compactor) and commence roller compaction. The following parameters are recommended for roller compaction operation:
   - Smooth rolls (counter-rotating, pressure applied to floating roller)
   - Horizontal screw feed (meters the product from the hopper into the pre-compression stage): screw feed speed from 0 to about 62 rpm, for example about 20 rpm
   - Vertical screw feed (performs pre-compression and de-aeration of materials and forces material to the rolls where actual compaction and final densification takes place in the nip area of rolls): screw feed speed from 0 to about 270 rpm, for example about 100 rpm
   - Roll pressure: from about 2000 to about 16,500 pounds per linear inch (pli), for example about 8000 pli
   - Roll speed: from 0 to about 17 rpm, for example about 10 rpm
4. Record the following in-process measurements:
   - Ribbon thickness (mm)
   - Ribbon density (g/cc) - target density from about 1.0 to about 1.5 g/cc
   - Ribbon width (mm)
   - Ribbon length (mm)
   - Ribbon weight (g)

   The method for determining ribbon density is as follows. Using a pychnometer, add 70 ml of oil (preferably liquid paraffin) and add ribbons of compacted materials up to 80 ml. The density of the ribbons can then be calculated by weight/10mL.
5. Collect the ribbons in a suitable container.
6. Mill the roller compacted ribbons using a suitable mill such as a comminuting mill with hammers and/or knives, preferably a FitzMill L1A with hammers forward using a 0.065 inch screen.
7. Determine the bulk density of the milled granules.
8. Screen the extragranular excipients (here: calcium hydrogen phosphate dihydrate, microcrystalline cellulose, sodium starch glycollate and HPMC), excluding the extragranular magnesium stearate, through a nominal 500 micron screen using a vibratory sieve, if required, into a suitable blender.
9. Blend these extragranular excipients with the milled granules at approximately 17 rpm for 15 minutes.
10. Screen the extragranular portion of the magnesium stearate through a nominal 500 micron screen into the blender which contains the mixture of extragranular excipients and granules.
11. Blend at approximately 17 rpm for 2 minutes.
12. Compress the resultant compression mix on a suitable tablet press, for example a Picolla rotary press or Kilian T100 rotary press, to make tablets. These tablets can be round or oval in major cross-section.

### Example 2 - calcium hydrogen phosphate dihydrate wholly intragranular

This uses the same ingredients list and process as Example 1, but all of the calcium hydrogen phosphate dihydrate is blended with the SB-207266-A and the to-be-intragranular portion of the magnesium stearate in step 2 of the process and the resulting blend roller compacted in step 3 of the process. The calcium hydrogen phosphate dihydrate is therefore wholly intragranular in the tablet.

In Example 2, the weight ratio of the CaHPO₄.2H₂O filler to the drug in the granules is for example: 2.14 : 1 (when 50 mg tablet strength/dose); or 0.96 : 1 = 1 : 1.04 (when 80 mg tablet strength).

### Example 3 - calcium hydrogen phosphate dihydrate part-intragranular and part-extragranular

This uses the same ingredients list and process as Example 1, but half of the calcium hydrogen phosphate dihydrate is blended with the SB-207266-A and the to-be-intragranular portion of the magnesium stearate in step 2 of the process and the resulting blend roller compacted in step 3 of the process. The remaining half of the calcium hydrogen phosphate dihydrate is screened and blended with the milled granules in steps 8 and 9 of the process. The calcium hydrogen phosphate dihydrate is therefore part-intragranular and part-extragranular in the tablet.

### Example 4 - HPMC wholly intragranular

This uses the same ingredients list and process as either Example 2 or Example 3 (calcium hydrogen phosphate dihydrate either wholly intragranular, or part-intragranular and part-extragranular), but all of the HPMC binder is blended with the SB-207266-A and the to-be-intragranular portions of the magnesium stearate and calcium hydrogen phosphate dihydrate in step 2 and the resulting blend roller compacted in step 3. The HPMC is therefore wholly intragranular in the tablet.

### Example 5 - Microcrystalline cellulose wholly intragranular

This uses the same ingredients list and process as either Example 2 or Example 3 (calcium hydrogen phosphate dihydrate either wholly intragranular, or part-intragranular and part-extragranular), but all of the microcrystalline cellulose is blended with the SB-207266-A and the to-be-intragranular portions of the magnesium stearate and calcium hydrogen phosphate dihydrate in step 2 and the resulting blend roller compacted in step 3. The microcrystalline cellulose is therefore wholly intragranular in the tablet.

### Example 6 - Microcrystalline cellulose part-intragranular, part-extragranular

This uses the same ingredients list and process as either Example 2 or Example 3 (calcium hydrogen phosphate dihydrate either wholly intragranular, or part-intragranular and part-extragranular), but half of the microcrystalline cellulose is blended with the SB-207266-A and the to-be-intragranular portions of the magnesium stearate and calcium hydrogen phosphate dihydrate in step 2 and the resulting blend roller compacted in step 3. The remaining half of the microcrystalline cellulose is screened and blended with the milled granules in steps 8 and 9. The microcrystalline cellulose is therefore part-intragranular, part-extragranular in the tablet.

### Example 7 - sodium starch glycollate wholly intragranular

This uses the same ingredients list and process as either Example 2 or Example 3 (calcium hydrogen phosphate dihydrate either wholly intragranular, or part-intragranular and part-extragranular), but all of the sodium starch glycollate is blended with the SB-207266-A and the to-be-intragranular portions of the magnesium stearate and calcium hydrogen phosphate dihydrate in step 2 and the resulting blend roller compacted in step 3. The sodium starch glycollate is therefore wholly intragranular in the tablet.

### Example 8 - sodium starch glycollate part-intragranular, part-extragranular

This uses the same ingredients list and process as either Example 2 or Example 3 (calcium hydrogen phosphate dihydrate either wholly intragranular, or part-intragranular and part-extragranular), but half of the sodium starch glycollate is blended with the SB-207266-A and the to-be-intragranular portions of the magnesium stearate and calcium hydrogen phosphate dihydrate in step 2 and the resulting blend roller compacted in step 3. The remaining half of the sodium starch glycollate is screened and blended with the milled granules in steps 8 and 9. The sodium starch glycollate is therefore part-intragranular, part-extragranular in the tablet.

### Example 9 - no HPMC binder

This uses the same ingredients list and process as any of Examples 1 to 3 or 5 to 8 but the HPMC binder is absent from the formulation. The loss of HPMC binder is compensated by an equivalent rise in the amount of calcium hydrogen phosphate dihydrate (either intragranular or extragranular), so as to maintain unchanged (a) the total coated tablet weight of 256.25 mg, (b) the total pre-coating tablet weight of 250 mg and (c) the amounts of the other excipients.

Therefore, for example, in Example 9, the amount and weight % of the composition of the calcium hydrogen phosphate dihydrate is as follows for the following tablet strengths (dose, calculated as SB-207266 free base): 174.0 mg or 69.6 wt% (when 10 mg tablet strength/dose); 163.0 mg or 65.2 wt% (20 mg strength); 130.0 mg or 52.0 wt% (50 mg strength); 97.0 mg or 38.8 wt% (80 mg strength). Where Example 9 modifies Example 2 or another Example itself modifying Example 2 (i.e. CaHPO₄.2H₂O wholly intragranular, and assuming remains so), then the weight ratio of the CaHPO₄.2H₂O filler to the drug in the granules is for example: 15.8 : 1 (when 10 mg tablet strength/dose); 6.84 : 1 (20 mg tablet strength); 2.36 : 1 (50 mg tablet strength); or 1.10 : 1 (80 mg tablet strength).

### Example 10 - dose modification

In a modification of any of Examples 1 to 9, formulations containing 30 mg, 75 mg, or 120 mg of SB-207266 per tablet (present as the hydrochloride salt, but the dose stated here being calculated as the free base) can be used to make tablets; instead of the 10, 25 and 40 mg per tablet amounts given in Examples 1-8. These formulations maintain unchanged (a) the total coated tablet weight of 256.25 mg, (b) the total pre-coating tablet weight of 250 mg and (c) the other excipient amounts in the Example 1 compositions, but adjust the amount of calcium hydrogen phosphate dihydrate used as the amount of SB 207266 varies.

### Examples 11 to 16

More preferred examples of the process and composition of the invention are given in the following Examples 11-16, none of which contain any added binder such as HPMC. Examples 11-16 summarise the processes carried out for the manufacture of: a 10 kg batch of tablets with 80 mg SB-207266 (measured as the pure free base) where the disintegrant was wholly extragranular (Example 11); a 10 kg batch of tablets as with Example 11 but with 40 mg SB-207266 (measured as the pure free base) (Example 12); a 10 kg batch of tablets with 40 mg SB-207266 (measured as the pure free base) where the disintegrant was 50% extragranular (Example 13). Example 14 is a modification of Example 11 using a 12-kg batch containing additional extragranular microcrystalline cellulose. Example 15 provides an optional film coating to the tablets. Example 16 varies the dose of SB-207266 in the tablets of Examples 11-15.

### Example 11 - tablets with 80 mg SB-207266, with no binder, with microcrystalline cellulose and calcium hydrogen phosphate wholly intragranular, with part-intragranular part-extragranular lubricant, and with disintegrant wholly extragranular

| **Ingredient** | **Unit Formula (weight per tablet, mg)** | **Batch quantity (g)** | **% by weight of uncoated tablet** | **Notes** |
|---|---|---|---|---|
| SB-207266-A (hydrochloride salt) | 87.90 mg | 3516 g | 35.16 % | 1 |
| Microcrystalline cellulose | 48.12 mg | 1925 g | 19.25 % | 2 |
| Calcium hydrogen phosphate dihydrate | 98.98 mg | 3959 g | 39.59 % | 3 |
| Sodium starch glycollate | 12.50 mg | 500 g | 5.00 % | 4 |
| Magnesium stearate | 2.50 mg | 100 g | 1.00 % | 5 |
| **Total** | **250.0 mg** | **10000 g (10 kg)** | 100 % | 6 |

| | | | | |
|---|---|---|---|---|
| **Notes :** | | | | |
| 1. The SB-207266-A, formed according to Description 1 or 2, is equivalent to 80.0mg of pure SB-207266 free base, and is wholly intragranular, being 37.21% by weight of the granules. | | | | |
| 2. The grade of microcrystalline cellulose is Avicel PH102 ™ (about 100 µm nominal mean particle size), wholly intragranular, 20.37% by weight of the granules. | | | | |
| 3. The grade of calcium hydrogen phosphate dihydrate is Emcompress TM, and is wholly intragranular, being 41.89% by weight of the granules. | | | | |
| 4. The sodium starch glycollate is Explotab ™, wholly extragranular. | | | | |
| 5. The magnesium stearate is split into 50% intragranular (which is 0.53% by weight of the granules) and 50% extragranular. | | | | |
| 6. Total granules (intragranular ingredients) = 9450 g per 10 kg of tablets = 236.25 mg per 250 mg tablet = 94.5% by weight of the composition | | | | |

### Process :

1. SB-207266-A was passed through a 1 mm stainless steel screen into a bin blender. Microcrystalline cellulose, calcium hydrogen phosphate dihydrate and one half of the quantity of magnesium stearate (50g) was similarly passed through a 630 µm stainless steel screen into the blender and the mix blended at 15 rpm (rpm = revolutions per minute) for 10 minutes.
2. The blend from step 1 was fed through a roller compactor (Fitzpatrick Chilsonator IR220) equipped with interlocking knurled rolls.
   With reference to **Fig. 5**, this particular roller compactor 2 comprises: a hopper 4 for holding the intragranular ingredients 6 blended in step 1, a preliminary horizontal screw feed 8 which in use meters the blend 6 from the hopper 4 into a pre-compression stage; and a main vertical screw feed 10 directly downstream of and in use fed directly by the preliminary horizontal screw feed 8, the main vertical screw feed 10 in use performing pre-compression / pre-compaction and de-aeration of the blend and feeding material vertically directly down into the nip angle area (nip zone) 14 of the rolls 12. The rolls 12 are horizontal and substantially level with each other. The compact (flake or ribbon) 16 is released from below the rolls 12.
   In this Example, the compactor roll speed was set at 3 rpm, the (preliminary) horizontal feed screw speed was 30 rpm, the (main) vertical feed screw speed was about 60-62 rpm or 70 rpm, and the roll force (roll pressure) was set to 24 kN/cm.
   The compact formed from the roller compactor was screened using a comminuting mill (Fitzmill L1A, e.g. as described in the description hereinbefore and as shown in the bottom half of **Fig. 6**, it can be separate from or integral with the roller compactor) in the knives forward mode with a speed of 5000 rpm and using a screen size of 0.093 inches (2.36 mm). The resultant yield was 9173 g of granule mix with a tapped bulk density of 1.06 g/ml. A portion was subject to particle size analysis using sieves.
3. The granule mix from step 2 (9061 g) was passed into a bin blender. Proportional quantities of sodium starch glycollate (467 g) and magnesium stearate (46.7 g) were passed into the blender (preferably passed through a 630 µm stainless steel screen into the blender) and the mix was blended for 5 minutes at 15 rpm.
4. A portion of the compression mix was compacted into tablets using a single punch tablet press (Manesty F3) equipped with 10.5 mm x 5.0 mm oval normal concave punches. The target uncoated tablet weight was 250 mg and the target hardness range 9 to 15 Kp. The tablets were tested for hardness, thickness, weight variation, disintegration and dissolution.

The following data was recorded for the intermediates used in the above process:

| | |
|---|---|
| Bulk Density of powder blend from step 1 prior to roller compaction | 0.36 g/ml |
| Tapped Density of blend from step 1 prior to roller compaction | 0.68 g/ml |
| Bulk Density of granule mix after roller compaction and milling (end of step 2) | 0.69 g/ml |
| Tapped Density of granule mix after roller compaction and milling (end of step 2) | 1.06 g/ml |

The sieve analysis (particle size analysis using sieves) of a portion of the roller compacted and milled granule mix (as at the end of step 2) was as follows:

| Sieve Size (µm) | Weight Retained (g) | Cumulative Weight Retained (% w/w) |
|---|---|---|
| 1000 | 0.483 | 5.0 |
| 710 | 0.799 | 13.3 |
| 500 | 0.920 | 22.9 |
| 355 | 0.841 | 31.7 |
| 250 | 1.191 | 44.1 |
| 180 | 1.372 | 58.4 |
| 63 | 2.356 | 83.0 |
| Base | 1.633 | 100.0 |

The following data was recorded for the tablets produced in step 4:

| | |
|---|---|
| Mean Hardness (Kp) | 12.4 |
| Mean thickness (mm) | 4.6 |
| Mean weight (mg) | 248.2 |
| Uniformity of weight | Complied with Ph Eur |
| Disintegration Time (mins) | 16 |
| Dissolution Time T75 (mins) | 17.9 |

### Example 12 - 40-mg-dose tablets with no binder, with microcrystalline cellulose and calcium hydrogen phosphate wholly intragranular, with part-intragranular part-extragranular lubricant, and with disintegrant wholly extragranular

| **Ingredient** | **Unit Formula (weight per tablet, mg)** | **Batch quantity (g)** | **% by weight of uncoated tablet** | **Notes** |
|---|---|---|---|---|
| SB-207266-A (hydrochloride salt) | 43.95 mg | 1758 g | 17.58 % | 1 |
| Microcrystalline cellulose | 62.50 mg | 2500 g | 25.00 % | 2 |
| Calcium hydrogen phosphate dihydrate | 128.55 mg | 5142 g | 51.42 % | 3 |
| Sodium starch glycollate | 12.50 mg | 500 g | 5.00 % | 4 |
| Magnesium stearate | 2.50 mg | 100 g | 1.00 % | 5 |
| **Total** | **250.0 mg** | **10000 g (10 kg)** | 100 % | 6 |

| | | | | |
|---|---|---|---|---|
| **Notes :** | | | | |
| 1. The SB-207266-A, formed according to Description 1 or 2, is equivalent to 40.0mg of pure SB-207266 free base, and is wholly intragranular, being 18.60% by weight of the granules. | | | | |
| 2. The grade of microcrystalline cellulose is Avicel PH102™ (about 100 µm nominal mean particle size), wholly intragranular, 26.46% by weight of the granules. | | | | |
| 3. The grade of calcium hydrogen phosphate dihydrate is Emcompress ™, wholly intragranular, being 54.41% by weight of the granules. | | | | |
| 4. The sodium starch glycollate is Explotab ™, wholly extragranular. | | | | |
| 5. The magnesium stearate is split into 50% intragranular (which is 0.53% by weight of the granules) and 50% extragranular. | | | | |
| 6. Total granules (intragranular ingredients) = 9450 g per 10 kg of tablets = 236.25 mg per 250 mg tablet = 94.5% by weight of the composition | | | | |

### Process :

1. Microcrystalline cellulose was passed through a 710 µm stainless steel screen into a bin blender. SB-207266-A was passed through a 1 mm stainless steel screen into the blender. One half of the quantity of magnesium stearate (50 g) and Calcium hydrogen phosphate dihydrate (5142 g) were similarly passed through the 710 µm stainless steel screen into the blender and the mix blended at 15 rpm for 10 minutes.
2. The blend from step 1 was fed through a roller compactor (Fitzpatrick Chilsonator IR220) equipped with interlocking knurled rolls and with the roll speed set at 5 rpm, the (preliminary) horizontal feed screw speed set at 17 to 20 rpm, the (main) vertical feed screw speed set at 49 to 53 rpm, and the roll force (roll pressure) set to 24 to 25 kN/cm. The compact formed from the roller compactor was screened using a comminuting mill (Fitzmill L1A) with knives forward, with a speed of 5000 rpm and using a screen size of 0.093 inches (2.36 mm). The resultant yield was 9306 g of granule mix with a tapped bulk density of 1.09 g/ml.
3. The granule mix from step 2 (9306 g) was passed into a bin blender. Magnesium stearate (49.3 g) and sodium starch glycollate (493 g) were similarly passed through a 710 µm stainless steel screen into the blender and the mix blended for 5 minutes at 15 rpm.
4. A portion of the compression mix was compacted into tablets using a single punch tablet press (Manesty F3) equipped with 10.5 mm x 5.0 mm oval normal concave punches. The target uncoated tablet weight was 250 mg and the target hardness range 9 to 15 Kp. The tablets were tested for hardness, thickness, weight variation, disintegration and dissolution.

The following data was recorded for the intermediates used in the above process:

| | |
|---|---|
| Bulk Density of powder blend from step 1 prior to roller compaction | 0.47 g/ml |
| Tapped Density of blend from step 1 prior to roller compaction | 0.88 g/ml |
| Bulk Density of granule mix after roller compaction and milling (end of step 2) | 0.73 g/ml |
| Tapped Density of granule mix after roller compaction and milling (end of step 2) | 1.09 g/ml |

The sieve analysis of the roller compacted and milled granule mix (as at the end of step 2) was as follows:

| Sieve Size (µm) | Weight Retained (g) | Cumulative Weight Retained (% w/w) |
|---|---|---|
| 1000 | 0.578 | 5.8 |
| 710 | 0.935 | 15.1 |
| 500 | 1.146 | 26.5 |
| 355 | 0.971 | 36.1 |
| 250 | 1.223 | 48.3 |
| 180 | 1.248 | 60.7 |
| 63 | 2.475 | 85.4 |
| Base | 1.467 | 100.0 |

The following data was recorded for the tablets produced in step 4:

| | |
|---|---|
| Mean Hardness (Kp) | 15.9 |
| Mean thickness (mm) | 4.30 |
| Mean weight (mg) | 244.5 |
| Uniformity of weight | Complied with Ph Eur |
| Disintegration Time (mins) | 7.5 |
| Dissolution Time T75 (mins) | 17.2 |

### Example 13 - 40-mg-dose tablets with no binder, with microcrystalline cellulose and calcium hydrogen phosphate wholly intragranular, and with the lubricant and the disintegrant being part-intragranular and part-extragranular

| **Ingredient** | **Unit Formula (weight per tablet, mg)** | **Batch quantity (g)** | **% by weight of uncoated tablet** | **Notes** |
|---|---|---|---|---|
| SB-207266-A (hydrochloride salt) | 43.95 mg | 1758 g | 17.58 % | 1 |
| Microcrystalline cellulose | 62.50 mg | 2500 g | 25.00 % | 2 |
| Calcium hydrogen phosphate dihydrate | 128.55 mg | 5142 g | 51.42 % | 3 |
| Sodium starch glycollate | 12.50 mg | 500 g | 5.00 % | 4 |
| Magnesium stearate | 2.50 mg | 100 g | 1.00 % | 5 |
| **Total** | **250.0 mg** | **10000 g (10 kg)** | 100 % | |

| | | | | |
|---|---|---|---|---|
| **Notes :** | | | | |
| 1. The SB-207266-A, formed according to Description 1 or 2, is equivalent to 40.0mg of pure SB-207266 free base, and is wholly intragranular. | | | | |
| 2. The grade of microcrystalline cellulose is Avicel PH102 ™ (about 100 µm nominal mean particle size), wholly intragranular. | | | | |
| 3. The grade of calcium hydrogen phosphate dihydrate is Emcompress ™, wholly intragranular. | | | | |
| 4. The sodium starch glycollate is Explotab ™, split into 50% intragranular and 50% extragranular. | | | | |
| 5. The magnesium stearate is split 50% intragranular / 50% extragranular. | | | | |

### Process :

1. Microcrystalline cellulose was passed through a 710 µm stainless steel screen into a bin blender. SB-207266-A was passed through a 1 mm stainless steel screen into the blender. Sodium starch glycollate (250 g), one half of the quantities of magnesium stearate (50 g) and calcium hydrogen phosphate dihydrate (5142 g) were similarly passed through the 710 µm stainless steel screen into the blender and the mix blended at 15 rpm for 10 minutes.
2. The blend from step 1 was fed through a roller compactor (Fitzpatrick Chilsonator IR220) equipped with interlocking knurled rolls and with the roll speed set at 5 rpm, the (preliminary) horizontal feed screw speed set at 20 rpm, the (main) vertical feed screw speed set at 49 to 53 rpm, and the roll force (roll pressure) set to 24 to 26 kN/cm. The compact formed from the roller compactor was screened using a comminuting mill (Fitzmill L1A) with knives forward, with a speed of 5000 rpm and using a screen size of 0.093 inches (2.36 mm). The resultant yield was 9528 g of granule mix with a tapped bulk density of 1.08 g/ml.
3. The granule mix from step 2 (9528 g) was passed into a bin blender. Magnesium stearate (49.2 g) along with the proportional quantities of the remainder of the sodium starch glycollate (245 g) were passed through a 710 µm stainless steel screen and the mix blended for 5 minutes at 15 rpm.
4. A portion of the compression mix was compacted into tablets using a single punch tablet press (Manesty F3) equipped with 10.5 mm x 5.0 mm oval normal concave punches. The target uncoated tablet weight was 250 mg and the target hardness range 9 to 15 Kp. The tablets were tested for hardness, thickness, weight variation, disintegration and dissolution.

The following data was recorded for the intermediates used in the above process:

| | |
|---|---|
| Bulk Density of powder blend from step 1 prior to roller compaction | 0.49 g/ml |
| Tapped Density of blend from step 1 prior to roller compaction | 0.81 g/ml |
| Bulk Density of granule mix after roller compaction and milling (end of step 2) | 0.70 g/ml |
| Tapped Density of granule mix after roller compaction and milling (end of step 2) | 1.08 g/ml |

The sieve analysis of the roller compacted and milled granule mix (as at the end of step 2) was as follows:

| Sieve Size (µm) | Weight Retained (g) | Cumulative Weight Retained (% w/w) |
|---|---|---|
| 1000 | 0.245 | 2.3 |
| 710 | 0.373 | 5.8 |
| 500 | 0.645 | 11.8 |
| 355 | 0.636 | 17.8 |
| 250 | 1.574 | 32.5 |
| 180 | 2.168 | 52.7 |
| 63 | 3.091 | 81.6 |
| Base | 1.964 | 100.0 |

The following data was recorded for the tablets produced in step 4:

| | |
|---|---|
| Mean Hardness (Kp) | 17.7 |
| Mean thickness (mm) | 4.32 |
| Mean weight (mg) | 247.0 |
| Uniformity of weight | Complied with Ph Eur |
| Disintegration Time (mins) | 14.2 |
| Dissolution Time T75 (mins) | 14.7 |

### Example 14 - 80-mg-dose tablets with with no binder and with microcrystalline cellulose partly extragranular

| Ingredient | Unit Formula (weight per tablet, mg) | Batch quantity (g) | % by weight of uncoated tablet | Notes |
|---|---|---|---|---|
| SB-207266-A (hydrochloride salt) | 87.90 mg | 3516 g | 29.30 % | 1 |
| Microcrystalline cellulose (intragranular) | 48.12 mg | 1925 g | 16.04 % | 2 |
| Calcium hydrogen phosphate dihydrate | 98.98 mg | 3959 g | 32.99 % | 3 |
| Sodium starch glycollate | 15.00 mg | 600 g | 5.00 % | 4 |
| Magnesium stearate | 3.00 mg | 120 g | 1.00 % | 5 |
| Microcrystalline cellulose (extragranular) | 47.00 mg | 1880 g | 15.67 % | 6 |
| **Total** | **300.0 mg** | **12000 g (12 kg)** | 100 % | |

| | | | | |
|---|---|---|---|---|
| **Notes :** | | | | |
| 1. The SB-207266-A, formed according to Description 1 or 2, is equivalent to 80.0mg of pure SB-207266 free base, and is wholly intragranular. | | | | |
| 2. The grade of microcrystalline cellulose is Avicel PH102 ™ (about 100 µm nominal mean particle size), and this is the intragranular portion thereof. | | | | |
| 3. The grade of calcium hydrogen phosphate dihydrate is Emcompress ™, wholly intragranular. | | | | |
| 4. The sodium starch glycollate is Explotab ™, wholly extragranular. | | | | |
| 5. The magnesium stearate is split 50% intragranular / 50% extragranular. | | | | |
| 6. The grade of microcrystalline cellulose is Avicel PH102 ™ (about 100 µm nominal mean particle size), and this is the extragranular portion thereof. | | | | |

### Process :

The process is generally the same as or similar to that of Example 11, except that additional microcrystalline cellulose (MCC) is added extragranularly to increase the compressibility of the tablet compression mix (there is about 50% MCC extragranular, about 50% MCC intragranular). Therefore Steps 1, 3 and 4 are modified, and the process is as follows:
1. SB-207266-A (3516 g) was passed through a 1 mm stainless steel screen into a bin blender. The intragranular portion of the microcrystalline cellulose (1925 g), the calcium hydrogen phosphate dihydrate (3959 g) and one half of the quantity of the magnesium stearate (60g) were similarly passed through a 630 µm or 710 µm stainless steel screen into the blender and the mix blended at 15 rpm for 10 minutes.
2. Step 2 is as for Example 11.
3. Most or all of the granule mix from step 2 was passed into a bin blender. Proportional quantities of: sodium starch glycollate (600 g if 100% yield in step 2 and if all the granule mix is used), magnesium stearate (60 g if 100% yield and if all the granule mix is used) and the extragranular portion of the microcrystalline cellulose (1880 g if 100% yield and if all the granule mix is used) were passed through a 630 µm or 710 µm stainless steel screen into the blender, and the mix blended for 5 minutes at 15 rpm.
4. A portion of the compression mix was compacted into tablets using a single punch tablet press (e.g. Manesty F3) equipped with oval normal concave punches. The target uncoated tablet weight was 300 mg.

### Example 15 - film coating of Examples 11 to 14

In a modification of any of Examples 11 to 14, the tablets can be provided with a film coating, e.g. a coating of Opadry White YS-1-7033, preferably of about 6.25 mg / tablet. In one such coating method, the tablet cores are transferred to a suitable coating machine, the cores are rotated and an aqueous dispersion of Opadry is sprayed onto them. Release test samples can be taken randomly from the batch and appropriately labelled.

### Example 16 - SB-207266 dose modification of Examples 11 to 15

In a modification of any of Examples 11 to 15, formulations containing 10 mg, 20 mg, 25 mg, 30 mg, 40 mg, 50 mg, 75 mg, 80 mg, or 120 mg of SB-207266 per tablet (present as the hydrochloride salt, but the dose stated here being calculated as the free base) can be used to make tablets. This is instead of the 40 mg or 80 mg per tablet doses of SB-207266 (calculated as the free base) given in Examples 11-15.

As the amount of SB 207266-A (hydrochloride salt) varies, the formulation can change in one of three alternative ways, options A, B or C:
(A) As the amount of SB 207266-A varies, the amount of calcium hydrogen phosphate dihydrate used can vary accordingly, while maintaining unchanged the total uncoated e.g. pre-coated tablet weight of 250 mg or 300 mg. So, e.g. if the dose increases, the amount of CaHPO₄.2H₂O decreases by the same amount that the amount of SB 207266-A increases.
(B) As the amount of SB 207266-A varies, the amount of calcium hydrogen phosphate dihydrate and the microcrystalline cellulose (the intragranular portion of these) used varies accordingly such that the ratio of the intragranular calcium hydrogen phosphate dihydrate and the intragranular microcrystalline cellulose remains approximately constant at about 2 : 1 (e.g. from 2.2 : 1 to 1.8 : 1, e.g. from 2.1 : 1 to 2.0 : 1, e.g. 2.07 : 1 to 2.04 : 1). The total uncoated e.g. pre-coated tablet weight of 250 mg or 300 mg is maintained unchanged. So the amount of intragranular [CaHPO₄.2H₂O and MCC] decreases at an approximately constant ratio if the SB 207266-A amount increases.
(C) As the amount of SB 207266-A varies, the total uncoated e.g. pre-coated tablet weight of 250 mg or 300 mg increases or decreases by the same amount as the increase or decrease in the amount of SB 207266-A. All excipients remain unchanged.

In all three permutations, these formulations maintain unchanged: the amounts of the other excipients in the Examples 11-15 compositions, and the processes (subject to all necessary changes e.g. to the amounts being made).

For example, where the 80-mg-dose tablet of Example 11 is modified to a 50-mg-dose tablet (calculated as the free base) according to option (B) above, tablets are formed as shown in the following table (**Example 16(B)-50**):

### Example 16(B)-50 composition - 50-mg-dose tablet (calculated as the free base)

| **Ingredient** | **Weight per tablet, mg** | **Batch quantity (g)** | **% by wt. of uncoated tablet** | **Notes** |
|---|---|---|---|---|
| SB-207266-A | 54.93 mg | 2197 g | 21.97 % | 1 |
| Microcrystalline cellulose | 59.12 mg | 2365 g | 23.65 % | 2 |
| Calcium hydrogen phosphate dihydrate | 120.95 mg | 4838 g | 48.38 % | 3 |
| Sodium starch glycollate | 12.50 mg | 500 g | 5.00 % | 4 |
| Magnesium stearate | 2.50 mg | 100 g | 1.00 % | 5 |
| **Total** | **250.0 mg** | **10000 g** | 100 % | 6 |

| | | | | |
|---|---|---|---|---|
| **Notes**: as for Example 11 except: | | | | |
| 1. SB-207266-A, formed according to Description 1 or 2, equivalent to 50.0mg of pure SB-207266 free base, wholly intragranular, 23.25% by weight of granules. | | | | |
| 2. The grade of microcrystalline cellulose is Avicel PH102 ™ (about 100 µm nominal mean particle size), wholly intragranular, 25.03% by weight of the granules. | | | | |
| 3. The grade of calcium hydrogen phosphate dihydrate is Emcompress ™, and is wholly intragranular, being 51.20% by weight of the granules. | | | | |

### Example 17 - Protocol for the treatment or prophylaxis of atrial fibrillation and/or atrial remodelling in humans using orally administered SB 207266

A proposed clinical protocol for the treatment or prophylaxis of atrial fibrillation and/or atrial remodelling using an orally administrable pharmaceutical composition comprising SB 207266 or a salt thereof, which composition can be an orally administrable composition of the present invention, is now described in detail.

This Protocol describes administration of SB 207266 or the salt (hereinafter "SB 207266") to patients with symptomatic persistent atrial fibrillation (AF). The objective is the inhibition of symptomatic recurrences of atrial fibrillation in these patients with persistent AF. Patients with symptomatic persistent AF, of duration ≥ 48 hrs and < 6 months, who require cardioversion (e.g. DC cardioversion) are suitable. Symptoms of persistent AF may for example include palpitations, etc. Patients preferably either have:
- therapeutic anticoagulation (e.g. warfarin or coumarin) for ≥ 3 weeks before commencement of treatment, preferably anticoagulated to an International Normalised Ratio (INR) of at least 2, or
- in the absence of therapeutic anticoagulation for ≥ 3 weeks, they have a transesophageal echocardiography (TEE ) which is negative for clot and have received intravenous heparin until aPTT is stable and in the therapeutic range.

Patients receive SB 207266 preferably after such therapeutic anticoagulation, or after TEE in addition to intravenous (iv) heparin.

SB 207266 (e.g. as free base, but more preferably as the hydrochloride salt SB 207266-A) is generally administered at daily oral doses of 20mg, 50 mg or 80 mg uid (once daily) (calculated as the free base). However, on day 1 of the administration of SB 207266, it is generally administered at a single oral loading dose of 1.5 times (1.5 x) or 2 times (2 x) the dosage allocated for the daily maintenance therapy. Therefore, preferably, for a 1.5 x loading dose, a single oral loading dose of 30 mg, 75 mg or 120 mg (calculated as the free base) is given on day 1, followed by a daily dose of 20 mg, 50 mg or 80 mg respectively on subsequent days (calculated as the free base). Alternatively, for a 2 x loading dose, a single oral loading dose of 40 mg, 100 mg or 160 mg (calculated as the free base) is given on day 1, followed by the a daily dose of 20 mg, 50 mg or 80 mg respectively on subsequent days (calculated as the free base).

Where 1.5 x loading doses are used, these can be administered as three 10, 25 or 40 mg tablets given at the same time on day 1; the daily maintenance dose can be administered as two 10, 25 or 40 mg tablets given at the same time on subsequent days; the 10, 25 and 40 mg tablets used are preferably those described in any of Examples 1 to 16 above. Where 2 x loading doses are used, these can be administered as two 20, 50 or 80 mg tablets given at the same time on day 1; the daily maintenance dose can be administered as one 20, 50 or 80 mg tablet given on subsequent days; the 20, 50 or 80 mg tablets used are preferably those described in any of Examples 1 to 16 above.

About two or about five hours after administration of the first-day 1.5x or 2 x oral loading dose of SB 207266, patients remaining in atrial fibrillation (and/or not pharmacologically cardioverted) preferably then undergo direct current (DC) cardioversion. Any of the following mono or bi-phasic cardioversion algorithms can be followed.

| Shock sequence | Mono-phasic | Bi-Phasic (option 1) | Bi-Phasic (option 2) |
|---|---|---|---|
| | | | |
| 1st Shock | 200 Joules | 170 Joules | 120 Joules |
| 2nd Shock | 250 Joules | 200 Joules | 150 Joules |
| 3rd Shock | 300 Joules | 230 Joules | 170 Joules |

If the patient does not revert to normal sinus rhythmn (NSR) after the 3rd shock using one of the above sequences the doctor may at his discretion proceed with further attempts at different energies. Successful cardioversion is defined as maintenance of NSR for ≥ 1 hour post-cardioversion.

Following a successful DC cardioversion to NSR, administration of SB 207266 to the patient can be continued once daily for 6 months (for example), or for shorter or longer periods. Those patients who spontaneously revert to normal sinus rhythmn (NSR) can also receive SB 207266 once daily for (e.g.) 6 months. Patients who experience a recurrence of AF during this daily treatment can be DC cardioverted back to sinus rhythm and can continue to receive SB 207266.

Patients should preferably continue on anticoagulation therapy (e.g. warfarin or coumarin) for at least the first four weeks following successful cardioversion, and more preferably throughout the period during which SB 207266 is administered. Preferably, the patients are anticoagulated to an International Normalised Ratio (INR) of at least 2 during some or all, e.g. most or all, of the period during which they are on anticoagulation therapy. This reduces the risk of cardiac blood clotting and/or stroke in the event of a recurrence of AF.

The most preferred Protocol is therefore given below:

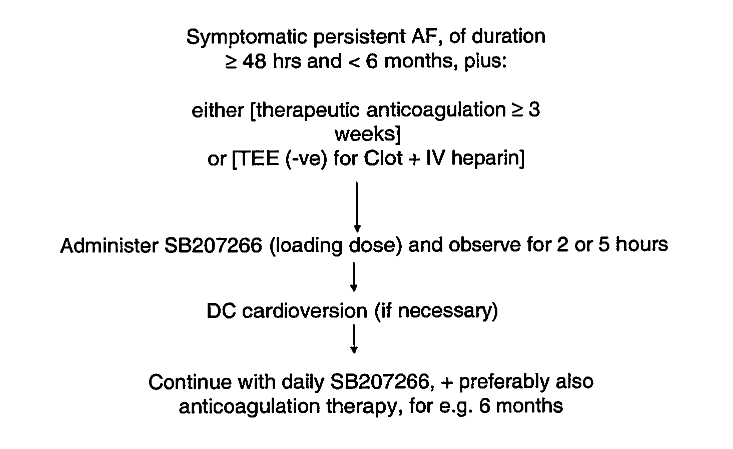

A "symptomatic recurrence" of AF includes or means an episode of palpitations or other symptoms typical for the patient. This can be further established by either a ECG (e.g. 12-lead ECG) recording showing evidence of atrial fibrillation or a rhythm strip recorded on a event recorder device and optionally reviewed by the doctor.

## Claims

1. A process for preparing a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable excipients,
the process comprising forming part or all of the SB 207266 or the salt thereof into granules by a dry granulation process,
and wherein the SB 207266 or the salt thereof is present in the composition in at least 4 weight % by weight of the composition.

2. A process as claimed in claim 1, wherein the dry granulation process is a process in which granules are formed wholly in the absence of an externally applied granulating solvent.

3. A process as claimed in any preceding claim wherein the dry granulation process comprises compression and compaction of the SB 207266 or the salt thereof.

4. A process as claimed in any preceding claim, wherein the process increases the particle size of the SB 207266 or the salt thereof by compression together of particles of drug, optionally also with particles of intragranular excipients, to form larger granules.

5. A process as claimed in any preceding claim, wherein the dry granulation process comprises roller compaction of the SB 207266 or the salt thereof.

6. A process as claimed in claim 5, wherein the rollers (rolls) have textured outer circumferential surfaces.

7. A process as claimed in claim 6, wherein the textured outer circumferential surfaces of the rolls comprise corrugations (peaks and valleys) aligned generally in the direction of the roll axis ("corrugated rolls"), and wherein the corrugated rolls are interlocking knurled rolls.

8. A process as claimed in claim 5, 6 or 7, wherein, during roller compaction, a pre-granulation powder to be compacted is fed into a roll gap by a rotating screw feed which is adjacent and directed towards the rollers (rolls), and wherein the roll gap is a gap between a pair of counterrotating rollers (rolls) having substantially parallel longitudinal axes.

9. A process as claimed in claim 8, wherein the rolls are substantially horizontal and substantially level with each other and wherein the screw feed used is a "vertical screw feed" positioned above and directed towards the rolls.

10. A process as claimed in claim 8 or 9, wherein the speed of the main screw feed adjacent the rolls is from about 30 to about 100 revolutions per minute (rpm).

11. A process as claimed in claim 8, 9 or 10, wherein a preliminary screw feed is used to meter the product (the pre-granulation blend containing the SB 207266 or the salt thereof) from an inlet hopper into a pre-compression (pre-compaction) stage, and wherein the main screw feed adjacent the rolls is downstream from the preliminary screw feed.

12. A process as claimed in claim 11, wherein the preliminary screw feed speed is from about 10 rpm to about 40 rpm.

13. A process as claimed in claim 11 or 12, wherein the ratio of the preliminary screw feed speed to the main screw feed speed is from 1 : 5 to 1 : 1.5.

14. A process as claimed in any of claims 5 to 13, wherein the roll pressure is from about 8000 to about 16500 pounds per linear inch (pli) (i.e. from about 14.0 to about 28.93 kN / cm).

15. A process as claimed in any of claims 5 to 14, wherein the roll speed is about 1.5 rpm to about 10 rpm.

16. A process as claimed in any of claims 5 to 15, wherein the SB-207266 or the salt thereof is present in the granules (i.e. during dry granulation) in at least 22% of the granules (intragranular ingredients), and:
- the preliminary screw feed speed, if such a feed is present, is at least about 25 rpm; and/or
- the main screw feed speed is at least about 56 rpm; and/or
- the roll speed is from about 1.5 rpm to about 4 rpm.

17. A process as claimed in any of claims 5 to 16, wherein the density of the ribbon (that is, the roller-compacted mixture as it exits the rollers) is from about 1.0 to about 1.5 g / cc (g/ml).

18. A process as claimed in any of claims 5 to 17, wherein the compact (flake, ribbon) exiting from the rollers (rolls) is milled to a particle size suitable for use in tablets or capsules.

19. A process as claimed in any of claims 5 to 17, wherein the granules formed by the dry granulation process are milled to a particle size suitable for use in tablets or capsules.

20. A process as claimed in claim 18 or 19, wherein the milling uses a comminuting mill with hammers and/or knives.

21. A process as claimed in claim 20, wherein the mill is a comminuting mill with "knives forward".

22. A process as claimed in any of claims 18 to 21, wherein, during or after milling, the granules are passed through a sieve having a 0.110 inch (2.80 mm) to a 0.032 inch (0.81 mm) hole size.

23. A process as claimed in any of claims 18 to 22, wherein the tapped density of the dry granulated and milled granules is about 0.8 to about 1.2 g/ml.

24. A process as claimed in any preceding claim, wherein, after formation of the granules and optional milling to a suitable size, the granules are then (i) optionally mixed with one or more pharmaceutically acceptable excipients (extragranular excipients) and (ii) compressed into tablets or filled into capsules.

25. A process as claimed in any preceding claim, wherein the pharmaceutical composition is orally administrable.

26. A process as claimed in any preceding claim, wherein the pharmaceutical composition is a tablet(s), or the pharmaceutical composition is or is contained in a capsule.

27. A process as claimed in any preceding claim, wherein 90% or more by weight of the SB 207266 or the salt thereof is present in the granules obtainable or prepared (formed) by the dry granulation process.

28. A process as claimed in any preceding claim, wherein 50% or more by weight or by volume of the granules including the SB 207266 or the salt thereof have a particle size of ≥ 75 microns (micrometres).

29. A process as claimed in claim 28, wherein 50% or more by weight or by volume of the granules including the SB 207266 or the salt thereof have a particle size of ≥ 106 microns (micrometres).

30. A process as claimed in claim 28, wherein 50% or more by weight or by volume of the granules including the SB 207266 or the salt thereof have a particle size of 75 to 1000 microns (micrometres).

31. A process as claimed in any preceding claim, wherein 90% or more by weight or by volume of the granules including the SB 207266 or the salt thereof have a particle size of 10 to 1000 microns (micrometres).

32. A process as claimed in any preceding claim, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises the hydrochloride salt of SB 207266.

33. A process as claimed in claim 32, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof is the hydrochloride salt of SB 207266.

34. A process as claimed in claim 32 or 33, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises the needle-shaped crystalline form of the hydrochloride salt of SB 207266.

35. A process as claimed in claim 32, 33 or 34, wherein the hydrochloride salt of SB 207266 has an infrared (IR) spectrum (nujol mull) substantially as shown in Fig. 4; and/or has an infrared (IR) spectrum (nujol mull) with three, four, five or more of the following peaks: 1628, 1582, 1502, 1531, 1184, 748 cm⁻¹ (with ± about 2 cm⁻¹ peak variation being allowed).

36. A process as claimed in any preceding claim, wherein 50% or more by weight or by volume of the particles of the SB 207266 or the salt thereof have a particle size of ≤ 53 microns (micrometres) before forming into granules.

37. A process as claimed in any preceding claim, wherein the SB 207266 or the salt thereof is present in the granules in at least 6 weight % by weight of the granules.

38. A process as claimed in any preceding claim, wherein the SB 207266 or the salt thereof is present in the composition in at least 6 weight % by weight of the composition.

39. A process as claimed in any preceding claim, wherein the SB 207266 or the salt thereof is present in the composition in up to 70 weight % by weight of the composition.

40. A process as claimed in any preceding claim, wherein the composition includes a lubricant.

41. A process as claimed in claim 40 wherein the lubricant is present in about 0.2 to about 5 weight % by weight of the composition.

42. A process as claimed in claim 40 or 41, wherein the lubricant is present both intragranularly and extragranularly.

43. A process as claimed in claim 40, 41 or 42, wherein the lubricant comprises calcium stearate or magnesium stearate.

44. A process as claimed in any preceding claim, comprising mixing some or all of the SB 207266 or the salt thereof with one or more pharmaceutically acceptable excipients (intragranular excipients) before dry granulation.

45. A process as claimed in claim 44 wherein the one or more intragranular excipients comprise a lubricant.

46. A process as claimed in claim 45, wherein the lubricant is present in about 0.2 to about 5 weight % by weight of the granules.

47. A process as claimed in claim 45 or 46, wherein the lubricant comprises calcium stearate or magnesium stearate.

48. A process as claimed in claim 44, 45, 46 or 47, wherein the one or more intragranular excipients comprise a filler (diluent), that is the granules containing the SB 207266 or the salt thereof also contain a filler (diluent).

49. A process as claimed in claim 48, wherein the filler comprises a pharmaceutically acceptable calcium or magnesium salt which is insoluble, practically insoluble, very slightly soluble or slightly soluble in water and/or ethanol,
and wherein the calcium or magnesium salt is calcium phosphate, dibasic calcium phosphate (calcium hydrogen phosphate), calcium carbonate, magnesium carbonate, magnesium phosphate, or calcium lactate.

50. A process as claimed in claim 48, wherein the filler comprises calcium phosphate, i.e. tribasic calcium phosphate, Ca₃(PO₄)₂.

51. A process as claimed in claim 48 or 50, wherein the filler comprises dibasic calcium phosphate (i.e. dicalcium phosphate, calcium hydrogen phosphate, CaHP04).

52. A process as claimed in claim 48, wherein the filler is dibasic calcium phosphate (i.e. dicalcium phosphate, calcium hydrogen phosphate, CaHPO₄).

53. A process as claimed in claim 51 or 52, wherein the filler comprises calcium hydrogen phosphate dihydrate (CaHPO₄.2H₂O).

54. A process as claimed in any of claims 48 to 53, wherein the filler is a coarse grade filler having a mean or median particle size of 53-300 µm (micrometres).

55. A process as claimed in any of claims 48 to 54, wherein the filler comprises from 10 to 90% by weight of the granules.

56. A process as claimed in any of claims 48 to 55, wherein the filler is present in from 15 to 85% by weight of the composition.

57. A process as claimed in any of claims 48 to 56, wherein the weight ratio of the filler to the SB-207266 or the salt thereof in the granules is at least 1:3.

58. A process as claimed in any of claims 48 to 56, wherein the filler is at least partly present intragranularly (i.e. is present during the dry granulation process), and the intragranular ratio of the filler to the SB-207266 or the salt thereof (i.e. the ratio during dry granulation) is from 1:3 to 10:1.

59. A process as claimed in any preceding claim, wherein the composition includes an excipient which acts as a compression aid.

60. A process as claimed in claim 59, wherein the compression aid comprises microcrystalline cellulose.

61. A process as claimed in claim 60, wherein the compression aid is microcrystalline cellulose.

62. A process as claimed in claim 60 or 61, wherein the microcrystalline cellulose has a nominal mean particle size of about 25 to about 150 µm (micrometres).

63. A process as claimed in claim 59, 60, 61 or 62, wherein the compression aid is present in at least 10 weight % by weight of the composition.

64. A process as claimed in claim 59, 60, 61, 62 or 63, wherein the compression aid is present in at least 10 weight % by weight of the granules.

65. A process as claimed in any of claims 59 to 64, wherein the compression aid is present in 10-50 weight % by weight of the composition.

66. A process as claimed in any of claims 59 to 65, wherein the compression aid is present inside the granules (i.e. intragranularly).

67. A process as claimed in any of claims 59 to 66, wherein the intragranular weight ratio, i.e. the weight ratio during the dry granulation process, of the filler to the compression aid is from 5:1 to 2:3.

68. A process as claimed in any preceding claim, wherein the composition includes no binder.

69. A process as claimed in any preceding claim, wherein the composition includes a disintegrant present in about 1 to about 10 weight % of the composition.

70. A process as claimed in any preceding claim, wherein the granules (i.e. the intragranular ingredients) form from about 75% to about 99% by weight of the composition.

71. A process as claimed in any of claims 1 to 69, wherein the granules (i.e. the intragranular ingredients) form 100% by weight of the composition (i.e. no extragranular excipients).

72. A process as claimed in any of claims 1 to 71, wherein the granules containing the SB 207266 or the salt thereof also contain a filler (diluent), wherein the filler comprises dibasic calcium phosphate (i.e. dicalcium phosphate, calcium hydrogen phosphate, CaHPO₄) and/or calcium phosphate [i.e. tribasic calcium phosphate, Ca₃(PO₄)₂],
and wherein the filler comprises from 10 to 90% by weight of the granules, and the weight ratio of the filler to the SB-207266 or the salt thereof in the granules is at least 1:3.

73. A process for preparing a pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable excipients (carriers), wherein the SB 207266 or the salt thereof is present in the composition in at least 4 weight % by weight of the composition,
the process comprising:
(a) dissolving the SB 207266 or the salt thereof in ethanol or an ethanol-containing solvent to form a solution,
(b) crystallising the SB 207266 or the salt thereof from the solution by addition of a C₅-C₁₀ hydrocarbon and/or a solvent containing a C₅-C₁₀ hydrocarbon, and
(c) forming at least some of the SB 207266 or the salt thereof into granules by a dry granulation process.

74. A process as claimed in claim 73, wherein the ethanol-containing solvent is industrial methylated spirits.

75. A process as claimed in claim 73 or 74, wherein the C₅-C₁₀ hydrocarbon is hexane and/or heptane.

76. A process as claimed in claim 73, 74 or 75, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises the hydrochloride salt of SB 207266.

77. A process as claimed in claim 73, 74, 75 or 76, wherein the dry granulation process and/or the excipient(s) are as described in any of claims 2 to 71.

78. A process as claimed in claim 73, 74, 75, 76 or 77, wherein the granules containing the SB 207266 or the salt thereof also contain a filler (diluent), wherein the filler comprises dibasic calcium phosphate (i.e. dicalcium phosphate, calcium hydrogen phosphate, CaHPO₄) and/or calcium phosphate [i.e. tribasic calcium phosphate, Ca₃(PO₄)₂],
and wherein the filler comprises from 10 to 90% by weight of the granules, and the weight ratio of the filler to the SB-207266 or the salt thereof in the granules is at least 1:3.

79. A pharmaceutical composition obtainable by a process as defined in any of claims 1 to 78.

80. A pharmaceutical composition which has been prepared by a process as defined in any of claims 1 to 78.

81. A pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable excipients,
wherein part or all of the SB 207266 or the salt thereof is present in granules obtainable by a dry granulation process,
and wherein the SB 207266 or the salt thereof is present in the composition in at least 4 weight % by weight of the composition.

82. A pharmaceutical composition comprising N-[(1-ⁿbutyl-4-piperidinyl)methyl]-3,4-dihydro-2H-[1,3]oxazino[3,2-a]indole-10-carboxamide (SB 207266) or a pharmaceutically acceptable salt thereof in combination with one or more pharmaceutically acceptable excipients,
wherein part or all of the SB 207266 or the salt thereof is present in granules prepared by a dry granulation process,
and wherein the SB 207266 or the salt thereof is present in the composition in at least 4 weight % by weight of the composition.

83. A pharmaceutical composition as claimed in claim 81 or 82, wherein the dry granulation process comprises roller compaction of the SB 207266 or the salt thereof.

84. A pharmaceutical composition as claimed in claim 83, wherein, in the process, the compact (flake, ribbon) exiting from the rollers (rolls) is milled to a particle size suitable for use in tablets or capsules.

85. A pharmaceutical composition as claimed in claim 81, 82, 83 or 84, wherein the pharmaceutical composition is a tablet(s), or the pharmaceutical composition is or is contained in a capsule.

86. A pharmaceutical composition as claimed in any of claims 81 to 85, wherein 90% or more by weight of the SB 207266 or the salt thereof is present in the granules obtainable or prepared (formed) by the dry granulation process.

87. A pharmaceutical composition as claimed in any of claims 81 to 86, wherein 50% or more by weight or by volume of the granules including the SB 207266 or the salt thereof have a particle size of ≥ 75 microns (micrometres).

88. A pharmaceutical composition as claimed in any of claims 81 to 87, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises the hydrochloride salt of SB 207266.

89. A pharmaceutical composition as claimed in claim 88, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof is the hydrochloride salt of SB 207266.

90. A pharmaceutical composition as claimed in claim 88 or 89, wherein the N-[(1-ⁿbutyl-4-piperidinyl)methyl]-3,4-dihydro-2H-[1,3]oxazino[3,2-a]indole-10-carboxamide (SB 207266) or the pharmaceutically acceptable salt thereof comprises the needle-shaped crystalline form of the hydrochloride salt of SB 207266.

91. A pharmaceutical composition as claimed in claim 88, 89 or 90, wherein the hydrochloride salt of SB 207266 has an infrared (IR) spectrum (nujol mull) substantially as shown in Fig. 4; and/or has an infrared (IR) spectrum (nujol mull) with three, four, five or more of the following peaks: 1628, 1582, 1502, 1531, 1184, 748 cm⁻¹ (with ± about 2 cm⁻¹ peak variation being allowed).

92. A pharmaceutical composition as claimed in any of claims 81 to 91, wherein the SB 207266 or the salt thereof is present in the granules in at least 6 weight % by weight of the granules.

93. A pharmaceutical composition as claimed in any of claims 81 to 92, wherein the SB 207266 or the salt thereof is present in the composition in at least 6 weight % by weight of the composition.

94. A pharmaceutical composition as claimed in any of claims 81 to 93, wherein the composition includes a lubricant present both intragranularly and extragranularly, and wherein the lubricant is present in 0.2 to 5 weight % by weight of the composition.

95. A pharmaceutical composition as claimed in any of claims 81 to 94, wherein the granules containing the SB 207266 or the salt thereof also contain a filler (diluent),
wherein the filler comprises a pharmaceutically acceptable calcium or magnesium salt which is insoluble, practically insoluble, very slightly soluble or slightly soluble in water and/or ethanol,
wherein the calcium or magnesium salt is calcium phosphate, dibasic calcium phosphate (calcium hydrogen phosphate), calcium carbonate, magnesium carbonate, magnesium phosphate, or calcium lactate,
and wherein the filler comprises from 10 to 90% by weight of the granules, and the weight ratio of the filler to the SB-207266 or the salt thereof in the granules is at least 1:3.

96. A pharmaceutical composition as claimed in any of claims 81 to 94, wherein the granules containing the SB 207266 or the salt thereof also contain a filler (diluent), wherein the filler comprises dibasic calcium phosphate (i.e. dicalcium phosphate, calcium hydrogen phosphate, CaHPO₄) and/or calcium phosphate [i.e. tribasic calcium phosphate, Ca₃(PO₄)₂],
and wherein the filler comprises from 10 to 90% by weight of the granules, and the weight ratio of the filler to the SB-207266 or the salt thereof in the granules is at least 1:3.

97. A pharmaceutical composition as claimed in claim 95 or 96, wherein the filler is a coarse grade filler having a mean or median particle size of 53-300 µm (micrometres).

98. A pharmaceutical composition as claimed in any of claims 81 to 97, wherein the composition includes an excipient which acts as a compression aid, wherein the compression aid is microcrystalline cellulose, wherein the compression aid is present in at least 10 weight % by weight of the composition, and wherein the compression aid is present inside the granules (i.e. intragranularly).

99. A pharmaceutical composition as claimed in any of claims 81 to 98, wherein the process and/or the composition is as defined in any of claims 2 to 78.

## Patentansprüche

1. Verfahren zur Herstellung einer pharmazeutischen Zusammensetzung umfassend N-[(1-ⁿButyl-4-piperidinyl)methyl]-3,4-dihydro-2H-[1,3]oxazino[3,2-a]indol-10-carboxamid (SB 207266) oder ein pharmazeutisch verträgliches Salz davon in Kombination mit einem oder mehreren pharmazeutisch verträglichen Exzipienten, wobei das Verfahren Überführen eines Teils des oder des gesamten SB 207266 oder des Salzes davon in Granulatkörner durch ein Trockengranulierungsverfahren umfasst und wobei der SB 207266 oder das Salz davon in der Zusammensetzung zu mindestens 4 Gew.-%, bezogen auf das Gewicht der Zusammensetzung, vorhanden ist.

2. Verfahren gemäß Anspruch 1, wobei das Trockengranulierungsverfahren ein Verfahren ist, in welchem die Granulatkörner in völliger Abwesenheit eines extern angewendeten Granulierungslösungsmittels gebildet werden.

3. Verfahren gemäß einem der vorstehenden Ansprüche, wobei das Trockengranulierungsverfahren Verdichten und Kompaktieren des SB 207266 oder des Salzes davon umfasst.

4. Verfahren gemäß einem der vorstehenden Ansprüche, wobei das Verfahren die Teilchengröße des SB 207266 oder des Salzes davon durch Verdichten der Wirkstoffteilchen miteinander, gegebenenfalls auch mit Teilchen der intragranularen Exzipienten, vergrößert, um größere Granulatkörner zu erzeugen.

5. Verfahren gemäß einem der vorstehenden Ansprüche, wobei das Trockengranulierungsverfahren das Walzenkompaktieren des SB 207266 oder des Salzes davon umfasst.

6. Verfahren gemäß Anspruch 5, wobei die Walzen textuierte Außenumfangsflächen aufweisen.

7. Verfahren gemäß Anspruch 6, wobei die textuierten Außenumfangsflächen der Walzen Wellungen (Berge und Täler), allgemein ausgerichtet in der Richtung der Walzenachse ("gewellte Walzen"), aufweisen, und wobei die gewellten Walzen ineinandergreifende Rändelwalzen sind.

8. Verfahren gemäß Anspruch 5, 6 oder 7, wobei während der Walzenkompaktierung ein Vorgranulierungspulver, welches zu kompaktieren ist, in einen Walzenspalt mittels einer drehenden Zuführschnecke, welche an die Walzen angrenzt und ihnen zugewandt ist, eingespeist wird und wobei der Walzenspalt eine Lücke zwischen einem Paar gegenläufig rotierender Walzen mit im Wesentlichen parallelen Longitudinalachsen ist.

9. Verfahren gemäß Anspruch 8, wobei die Walzen im Wesentlichen horizontal und miteinander im Wesentlichen auf einer Ebene liegen und wobei die verwendete Zuführschnecke eine "Vertikalzuführschnecke" ist, die oberhalb der Walzen positioniert und ihnen zugewandt ist.

10. Verfahren gemäß Anspruch 8 oder 9, wobei die Geschwindigkeit der Hauptzuführschnecke, die an die Walzen angrenzt, etwa 30 bis etwa 100 Umdrehungen pro Minute (UpM) beträgt.

11. Verfahren gemäß Anspruch 8, 9 oder 10, wobei eine vorgeschaltete Zuführschnecke verwendet wird, um das Produkt (das Vorgranulierungsgemisch, welches das SB 207266 oder das Salz davon enthält) aus einem Einlasstrichter einer Vorverdichtungs-(Vorkompaktierungs)Stufe zuzudosieren, und wobei die Hauptzuführschnecke, die an die Walzen angrenzt, stromabwärts der vorgeschalteten Zuführschnecke liegt.

12. Verfahren gemäß Anspruch 11, wobei die Geschwindigkeit der vorgeschalteten Zuführschnecke etwa 10 UpM bis etwa 40 UpM beträgt.

13. Verfahren gemäß Anspruch 11 oder 12, wobei das Verhältnis der Geschwindigkeit der vorgeschalteten Zuführschnecke zu der Geschwindigkeit der Hauptzuführschnecke 1:5 bis 1:1,5 beträgt.

14. Verfahren gemäß einem der Ansprüche 5 bis 13, wobei der Walzendruck etwa 8000 bis etwa 16500 Pfund per Linearinch (pli) (d.h. etwa 14,0 bis etwa 28,93 kN/cm) beträgt.

15. Verfahren gemäß einem der Ansprüche 5 bis 14, wobei die Walzengeschwindigkeit etwa 1,5 UpM bis etwa 10 UpM beträgt.

16. Verfahren gemäß einem der Ansprüche 5 bis 15, wobei das SB 207266 oder das Salz davon in den Granulatkörnern (d.h. während der Trockengranulierung) in mindestens 22% der Granulatkörner (Intragranularbestandteile) vorhanden ist, und:
- die Geschwindigkeit der vorgeschalteten Zuführschnecke, falls eine derartige Zuführung vorhanden ist, mindestens etwa 25 UpM beträgt; und/oder
- die Geschwindigkeit der Hauptzuführschnecke mindestens etwa 56 UpM beträgt; und/oder
- die Walzengeschwindigkeit etwa 1,5 UpM bis etwa 4 UpM beträgt.

17. Verfahren gemäß einem der Ansprüche 5 bis 16, wobei die Dichte des Bandes (d.h. das walzenkompaktierte Gemisch, wie es die Walzen verlässt) etwa 1,0 bis etwa 1,5 g/cm³ (g/ml) beträgt.

18. Verfahren gemäß einem der Ansprüche 5 bis 17, wobei das kompaktierte Material (Plättchen, Band), welches die Walzen verlässt, zu einer Teilchengröße gemahlen wird, die geeignet ist für die Verwendung in Tabletten oder Kapseln.

19. Verfahren gemäß einem der Ansprüche 5 bis 17, wobei die Granulatkörner, die durch das Trockengranulierungsverfahren gebildet werden, zu einer Teilchengröße gemahlen werden, die geeignet ist zur Verwendung in Tabletten oder Kapseln.

20. Verfahren gemäß Anspruch 18 oder 19, wobei das Mahlen eine pulverisierende Mühle mit Hämmern und/oder Messern verwendet.

21. Verfahren gemäß Anspruch 20, wobei die Mühle eine pulverisierende Mühle mit "Messern vorwärts" ist.

22. Verfahren gemäß einem der Ansprüche 18 bis 21, wobei während oder nach dem Mahlen die Granulatkörner ein Sieb passieren, das eine Lochgröße von 0,110 Inch (2,80 mm) bis 0,032 Inch (0,81 mm) aufweist.

23. Verfahren gemäß einem der Ansprüche 18 bis 22, wobei die Klopfdichte der trockengranulierten und gemahlenen Granulatkörner etwa 0,8 bis etwa 1,2 g/ml beträgt.

24. Verfahren gemäß einem der vorstehenden Ansprüche, wobei nach der Bildung der Granulatkörner und gegebenenfalls Mahlen zu einer geeigneten Größe, die Granulatkörner dann (i) gegebenenfalls mit einem oder mehreren pharmazeutisch verträglichen Exzipienten (Extragranular-Exzipienten) gemischt werden und (ii) zu Tabletten gepresst oder in Kapseln gefüllt werden.

25. Verfahren gemäß einem der vorstehenden Ansprüche, wobei die pharmazeutische Zusammensetzung oral verabreichbar ist.

26. Verfahren gemäß einem der vorstehenden Ansprüche, wobei die pharmazeutische Zusammensetzung eine oder mehrere Tabletten ist, oder die pharmazeutische Zusammensetzung eine Kapsel ist oder in einer enthalten ist.

27. Verfahren gemäß einem der vorstehenden Ansprüche, wobei 90 Gew.-% oder mehr des SB 207266 oder des Salzes davon in den Granulatkörner vorhanden ist, welche erhältlich sind oder hergestellt (gebildet) werden durch das Trockengranulierungsverfahren.

28. Verfahren gemäß einem der vorstehenden Ansprüche, wobei 50 Gew.- oder Vol.-% oder mehr der Granulatkörner, welche das SB 207266 oder das Salz davon enthalten, eine Teilchengröße von ≥75 Mikron (Mikrometern) aufweisen.

29. Verfahren gemäß Anspruch 28, wobei 50 Gew.- oder Vol.-% oder mehr der Granulatkörner, welche das SB 207266 oder das Salz davon enthalten, eine Teilchengröße von ≥106 Mikron (Mikrometern) aufweisen.

30. Verfahren gemäß Anspruch 28, wobei 50 Gew.- oder Vol.-% oder mehr der Granulatkörner, welche das SB 207266 oder das Salz davon enthalten, eine Teilchengröße von 75 bis 1000 Mikron (Mikrometern) aufweisen.

31. Verfahren gemäß einem der vorstehenden Ansprüche, wobei 90 Gew.- oder Vol.-% oder mehr der Granulatkörner, welche das SB 207266 oder das Salz davon enthalten, eine Teilchengröße von 10 bis 1000 Mikron (Mikrometern) aufweisen.

32. Verfahren gemäß einem der vorstehenden Ansprüche, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon das Hydrochloridsalz von SB 207266 umfasst.

33. Verfahren gemäß Anspruch 32, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon das Hydrochloridsalz von SB 207266 ist.

34. Verfahren gemäß Anspruch 32 oder 33, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon die kristalline Form des Hydrochloridsalzes von SB 207266 in Nadelform umfasst.

35. Verfahren gemäß Anspruch 32, 33 oder 34, wobei das Hydrochloridsalz von SB 207266 im Wesentlichen ein Infrarot-(IR)-Spektrum (Nujolfilm) aufweist wie in Abbildung 4 gezeigt; und/oder ein Infrarot-(IR)-Spektrum (Nujolfilm) aufweist mit drei, vier, fünf oder mehr der nachstehenden Signale: 1628, 1582, 1502, 1531, 1184, 748 cm⁻¹ (mit einer erlaubten Signalabweichung von etwa ± 2 cm⁻¹).

36. Verfahren gemäß einem der vorstehenden Ansprüche, wobei 50 Gew.- oder Vol.-% oder mehr der Teilchen des SB 207266 oder des Salzes davon vor dem Überführen in Granulatkörner eine Teilchengröße von ≤53 Mikron (Mikrometern) aufweisen.

37. Verfahren gemäß einem der vorstehenden Ansprüche, wobei das SB 207266 oder das Salz davon in den Granulatkörnern zu mindestens 6 Gew.-%, bezogen auf das Gewicht der Granulatkörner, vorhanden ist.

38. Verfahren gemäß einem der vorstehenden Ansprüche, wobei das SB 207266 oder das Salz davon in der Zusammensetzung zu mindestens 6 Gew.-%, bezogen auf das Gewicht der Zusammensetzung, vorhanden ist.

39. Verfahren gemäß einem der vorstehenden Ansprüche, wobei das SB 207266 oder das Salz davon in der Zusammensetzung zu bis zu 70 Gew.-%, bezogen auf das Gewicht der Zusammensetzung, vorhanden ist.

40. Verfahren gemäß einem der vorstehenden Ansprüche, wobei die Zusammensetzung ein Gleitmittel einschließt.

41. Verfahren gemäß Anspruch 40, wobei das Gleitmittel zu etwa 0,2 bis etwa 5 Gew.-% der Zusammensetzung vorhanden ist.

42. Verfahren gemäß Anspruch 40 oder 41, wobei das Gleitmittel sowohl intragranular als auch extragranular vorhanden ist.

43. Verfahren gemäß Anspruch 40, 41 oder 42, wobei das Gleitmittel Calciumstearat oder Magnesiumstearat umfasst.

44. Verfahren gemäß einem der vorstehenden Ansprüche, umfassend das Mischen von etwas oder des gesamten SB 207266 oder des Salzes davon mit einem oder mehreren pharmazeutisch verträglichen Exzipienten (intragranularen Exzipienten) vor der Trockengranulierung.

45. Verfahren gemäß Anspruch 44, wobei das eine oder die mehreren intragranularen Exzipienten ein Gleitmittel umfassen.

46. Verfahren gemäß Anspruch 45, wobei das Gleitmittel zu etwa 0,2 bis etwa 5 Gew.-%, bezogen auf das Gewicht der Granulatkörner, vorhanden ist.

47. Verfahren gemäß Anspruch 45 oder 46, wobei das Gleitmittel Calciumstearat oder Magnesiumstearat umfasst.

48. Verfahren gemäß Anspruch 44, 45, 46 oder 47, wobei das eine oder die mehreren intragranularen Exzipienten einen Füllstoff umfassen (Verdünnungsmittel), d.h. die Granulatkörner, welche das SB 207266 oder das Salz davon enthalten, enthalten auch einen Füllstoff (Verdünnungsmittel).

49. Verfahren gemäß Anspruch 48, wobei der Füllstoff ein pharmazeutisch verträgliches Calcium- oder Magnesiumsalz umfasst, welches unlöslich oder so gut wie unlöslich, sehr wenig löslich oder wenig löslich in Wasser und/oder Ethanol ist, und wobei das Calcium- oder Magnesiumsalz Calciumphosphat, zweibasisches Calciumphosphat (Calciumhydrogenphosphat), Calciumcarbonat, Magnesiumcarbonat, Magnesiumphosphat oder Calciumlactat ist.

50. Verfahren gemäß Anspruch 48, wobei der Füllstoff Calciumphosphat, d.h. dreibasisches Calciumphosphat, Ca₃(PO₄)₂, umfasst.

51. Verfahren gemäß Anspruch 48 oder 50, wobei der Füllstoff zweibasisches Calciumphosphat (d.h. Dicalciumphosphat, Calciumhydrogenphosphat, CaHPO₄) umfasst.

52. Verfahren gemäß Anspruch 48, wobei der Füllstoff zweibasisches Calciumphosphat (d.h. Dicalciumphosphat, Calciumhydrogenphosphat, CaHPO₄) ist.

53. Verfahren gemäß Anspruch 51 oder 52, wobei der Füllstoff Calciumhydrogenphosphatdihydrat (CaHPO₄·2H₂O) umfasst.

54. Verfahren gemäß einem der Ansprüche 48 bis 53, wobei der Füllstoff ein grobkörniger Füllstoff ist, welcher eine mittlere oder Medianteilchengröße von 53 bis 300 µm (Mikrometern) aufweist.

55. Verfahren gemäß einem der Ansprüche 48 bis 54, wobei der Füllstoff 10 bis 90 Gew.-% der Granulatkörner umfasst.

56. Verfahren gemäß einem der Ansprüche 48 bis 55, wobei der Füllstoff zu 15 bis 85 Gew.-% der Zusammensetzung vorhanden ist.

57. Verfahren gemäß einem der Ansprüche 48 bis 56, wobei das Gewichtsverhältnis des Füllstoffs zu dem SB 207266 oder dem Salz davon in den Granulatkömem mindestens 1:3 beträgt.

58. Verfahren gemäß einem der Ansprüche 48 bis 56, wobei der Füllstoff mindestens teilweise intragranular vorhanden ist, (d.h. er ist während des Trockengranulierungsverfahrens vorhanden) und das intragranulare Verhältnis des Füllstoffs zu dem SB 207266 oder dem Salz davon (d.h. das Verhältnis während der Trockengranulierung) 1:3 bis 10:1 beträgt.

59. Verfahren gemäß einem der vorstehenden Ansprüche, wobei die Zusammensetzung einen Exzipienten einschließt, der als Verdichtungshilfsmittel wirkt.

60. Verfahren gemäß Anspruch 59, wobei das Verdichtungshilfsmittel mikrokristalline Cellulose umfasst.

61. Verfahren gemäß Anspruch 60, wobei das Verdichtungshilfsmittel eine mikrokristalline Cellulose ist.

62. Verfahren gemäß Anspruch 60 oder 61, wobei die mikrokristalline Cellulose eine nominale mittlere Teilchengröße von etwa 25 bis etwa 150 µm (Mikrometern) aufweist.

63. Verfahren gemäß Anspruch 59, 60, 61 oder 62, wobei das Verdichtungshilfsmittel zu etwa 10 Gew.-%, bezogen auf das Gewicht der Zusammensetzung, vorhanden ist.

64. Verfahren gemäß Anspruch 59, 60, 61, 62 oder 63, wobei das Verdichtungshilfsmittel zu mindestens 10 Gew.-%, bezogen auf das Gewicht der Granulatkörner, vorhanden ist.

65. Verfahren gemäß einem der Ansprüche 59 bis 64, wobei das Verdichtungshilfsmittel zu 10 bis 50 Gew.-%, bezogen auf das Gewicht der Zusammensetzung, vorhanden ist.

66. Verfahren gemäß einem der Ansprüche 59 bis 65, wobei das Verdichtungshilfsmittel innerhalb der Granulatkörner (d.h. intragranular) vorhanden ist.

67. Verfahren gemäß einem der Ansprüche 59 bis 66, wobei das intragranulare Gewichtsverhältnis, d.h. das Gewichtsverhältnis während des Trockengranulierungsverfahrens, des Füllstoffs zu dem Verdichtungshilfsmittel 5:1 bis 2:3 beträgt.

68. Verfahren gemäß einem der vorstehenden Ansprüche, wobei die Zusammensetzung kein Bindemittel einschließt.

69. Verfahren gemäß einem der vorstehenden Ansprüche, wobei die Zusammensetzung ein Sprengmittel einschließt, welches zu etwa 1 bis etwa 10 Gew.-% der Zusammensetzung vorhanden ist.

70. Verfahren gemäß einem der vorstehenden Ansprüche, wobei die Granulatkörner (d.h. die intragranularen Bestandteile) etwa 75 bis etwa 99 Gew.-% der Zusammensetzung bilden.

71. Verfahren gemäß einem der Ansprüche 1 bis 69, wobei die Granulatkörner (d.h. die intragranularen Bestandteile) 100 Gew.-% der Zusammensetzung bilden (d.h. keine extragranularen Exzipienten).

72. Verfahren gemäß einem der Ansprüche 1 bis 71, wobei die Granulatkörner, die das SB 207266 oder das Salz davon enthalten, auch einen Füllstoff (Verdünnungsmittel) enthalten, wobei der Füllstoff zweibasisches Calciumphosphat (d.h. Dicalciumphosphat, Calciumhydrogenphosphat, CaHPO₄) umfasst und/oder Calciumphosphat [d.h. dreibasisches Calciumphosphat, Ca₃(PO₄)₂], und wobei der Füllstoff 10 bis 90 Gew.-% der Granulatkörner umfasst und das Gewichtsverhältnis des Füllstoffs zu dem SB 207266 oder dem Salz davon in den Granulatkömem mindestens 1:3 beträgt.

73. Verfahren zur Herstellung einer pharmazeutischen Zusammensetzung umfassend N-[(1-ⁿButyl-4-piperidinyl)methyl]-3,4-dihydro-2H-[1,3]oxazino[3,2-a]indol-10-carboxamid (SB 207266) oder ein pharmazeutisch verträgliches Salz davon in Kombination mit einem oder mehreren pharmazeutisch verträglichen Exzipienten (Trägern), wobei das SB 207266 oder das Salz davon in der Zusammensetzung zu mindestens 4 Gew.-%, bezogen auf das Gewicht der Zusammensetzung, vorhanden ist, wobei das Verfahren umfasst:
(a) Lösen des SB 207266 oder des Salzes davon in Ethanol oder einem Ethanol-enthaltenden Lösungsmittel, um eine Lösung zu erhalten,
(b) Kristallisieren des SB 207266 oder des Salzes davon aus der Lösung durch Zugabe eines C₅-C₁₀ Kohlenwasserstoffs und/oder eines Lösungsmittels, welches einen C₅-C₁₀ Kohlenwasserstoff enthält, und
(c) Überführen von mindestens eines Teils des SB 207266 oder des Salzes davon in Granulatkörner durch ein Trockengranulierungsverfahren.

74. Verfahren gemäß Anspruch 73, wobei das Ethanol-enthaltende Lösungsmittel ein industrieller Brennspiritus ist.

75. Verfahren gemäß Anspruch 73 oder 74, wobei der C₅-C₁₀ Kohlenwasserstoff Hexan und/oder Heptan ist.

76. Verfahren gemäß Anspruch 73, 74 oder 75, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon das Hydrochloridsalz von SB 207266 umfasst.

77. Verfahren gemäß Anspruch 73, 74, 75 oder 76, wobei das Trockengranulierungsverfahren und/oder der(die) Exzipient(en) die in den Ansprüchen 2 bis 71 beschriebenen sind.

78. Verfahren gemäß Anspruch 73, 74, 75, 76 oder 77, wobei die Granulatkörner, welche das SB 207266 oder das Salz davon enthalten, auch einen Füllstoff (Verdünnungsmittel) enthalten, wobei der Füllstoff zweibasisches Calciumphosphat (d.h. Dicalciumphosphat, Calciumhydrogenphosphat, CaHPO₄) umfasst und/oder Calciumphosphat [d.h. dreibasisches Calciumphosphat, Ca₃(PO₄)₂], und wobei der Füllstoff 10 bis 90 Gew.-% der Granulatkörner umfasst und das Gewichtsverhältnis des Füllstoffs zu dem SB 207266 oder dem Salz davon in den Granulatkömem mindestens 1:3 beträgt.

79. Pharmazeutische Zusammensetzung, welche durch ein Verfahren gemäß einem der Ansprüche 1 bis 78 erhältlich ist.

80. Pharmazeutische Zusammensetzung, welche durch ein Verfahren gemäß einem der Ansprüche 1 bis 78 hergestellt wurde.

81. Pharmazeutische Zusammensetzung, umfassend N-[(1-ⁿButyl-4-piperidinyl)methyl]-3,4-dihydro-2H-[1,3]oxazino[3,2-a]indol-10-carboxamid (SB 207266) oder ein pharmazeutisch verträgliches Salz davon in Kombination mit einem oder mehreren pharmazeutisch verträglichen Exzipienten, wobei ein Teil des oder das gesamte SB 207266 oder das Salz davon in Granulatkörnern vorhanden ist, welche durch ein Trockengranulierungsverfahren erhältlich sind, und wobei das SB 207266 oder das Salz davon in der Zusammensetzung zu mindestens 4 Gew.-%, bezogen auf das Gewicht der Zusammensetzung, vorhanden ist.

82. Pharmazeutische Zusammensetzung, umfassend N-[(1-ⁿButyl-4-piperidinyl)methyl]-3,4-dihydro-2H-[1,3]oxazino[3,2-a]indol-10-carboxamid (SB 207266) oder ein pharmazeutisch verträgliches Salz davon in Kombination mit einem oder mehreren pharmazeutisch verträglichen Exzipienten, wobei ein Teil des oder das gesamte SB 207266 oder das Salz davon in Granulatkörnern vorhanden ist, welche durch ein Trockengranulierungsverfahren hergestellt wurden, und wobei das SB 207266 oder das Salz davon in der Zusammensetzung zu mindestens 4 Gew.-%, bezogen auf das Gewicht der Zusammensetzung, vorhanden ist.

83. Pharmazeutische Zusammensetzung gemäß Anspruch 81 oder 82, wobei das Trockengranulierungsverfahren Walzenkompaktieren des SB 207266 oder des Salzes davon umfasst.

84. Pharmazeutische Zusammensetzung gemäß Anspruch 83, wobei in dem Verfahren das kompaktierte Material (Plättchen, Band), welches aus den Walzen austritt, zu einer Teilchengröße gemahlen wird, welche geeignet ist zur Verwendung in Tabletten oder Kapseln.

85. Pharmazeutische Zusammensetzung gemäß Anspruch 81, 82, 83 oder 84, wobei die pharmazeutische Zusammensetzung eine oder mehrere Tabletten ist oder die pharmazeutische Zusammensetzung eine Kapsel ist oder in einer enthalten ist.

86. Pharmazeutische Zusammensetzung gemäß einem der Ansprüche 81 bis 85, wobei 90 Gew.-% oder mehr des SB 207266 oder des Salzes davon in den Granulatkörnern, erhältlich oder hergestellt (gebildet) durch das Trockengranulierungsverfahren, vorhanden ist.

87. Pharmazeutische Zusammensetzung gemäß einem der Ansprüche 81 bis 86, wobei 50 Gew.- oder Vol.-% oder mehr der Granulatkörner, welche das SB 207266 oder das Salz davon enthalten, eine Teilchengröße von ≥75 Mikron (Mikrometern) aufweisen.

88. Pharmazeutische Zusammensetzung gemäß einem der Ansprüche 81 bis 87, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon das Hydrochloridsalz von SB 207266 umfasst.

89. Pharmazeutische Zusammensetzung gemäß Anspruch 88, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon das Hydrochloridsalz von SB 207266 ist.

90. Pharmazeutische Zusammensetzung gemäß Anspruch 88 oder 89, wobei das N-[(1-ⁿButyl-4-piperidinyl)methyl]-3,4-dihydro-2H-[1,3]oxazino[3,2-a]indol-10-carboxamid (SB 207266) oder das pharmazeutisch verträgliche Salz davon die kristalline Form des Hydrochloridsalzes von SB 207266 in Nadelform umfasst.

91. Pharmazeutische Zusammensetzung gemäß Anspruch 88, 89 oder 90, wobei das Hydrochloridsalz von SB 207266 im Wesentlichen ein Infrarot-(IR)-Spektrum (Nujolfilm) aufweist wie in Abbildung 4 gezeigt; und/oder ein Infrarot-(IR)-Spektrum (Nujolfilm) aufweist mit drei, vier, fünf oder mehr der nachstehenden Signale: 1628, 1582, 1502, 1531, 1184, 748 cm⁻¹ (mit einer erlaubten Signalabweichung von etwa ± 2 cm⁻¹).

92. Pharmazeutische Zusammensetzung gemäß einem der Ansprüche 81 bis 91, wobei das SB 207266 oder das Salz davon in den Granulatkörnern zu mindestens 6 Gew.-%, bezogen auf das Gewicht der Granulatkörner, vorhanden ist.

93. Pharmazeutische Zusammensetzung gemäß einem der Ansprüche 81 bis 92, wobei das SB 207266 oder das Salz davon in der Zusammensetzung zu mindestens 6 Gew.-%, bezogen auf das Gewicht der Zusammensetzung, vorhanden ist.

94. Pharmazeutische Zusammensetzung gemäß einem der Ansprüche 81 bis 93, wobei die pharmazeutische Zusammensetzung ein Gleitmittel einschließt, welches sowohl intragranular als auch extragranular vorhanden ist, und wobei das Gleitmittel zu 0,2 bis 5 Gew.-%, bezogen auf das Gewicht der Zusammensetzung, vorhanden ist.

95. Pharmazeutische Zusammensetzung gemäß einem der Ansprüche 81 bis 94, wobei die Granulatkörner, welche das SB 207266 oder das Salz davon enthalten, auch einen Füllstoff (Verdünnungsmittel) enthalten,
wobei der Füllstoff ein pharmazeutisch verträgliches Calcium- oder Magnesiumsalz umfasst, welches unlöslich, so gut wie unlöslich, sehr wenig löslich oder wenig löslich in Wasser und/oder Ethanol ist, wobei das Calcium- oder Magnesiumsalz Calciumphosphat, zweibasisches Calciumphosphat (Calciumhydrogenphosphat), Calciumcarbonat, Magnesiumcarbonat, Magnesiumphosphat oder Calciumlactat ist und
wobei der Füllstoff 10 bis 90 Gew.-% der Granulatkörner umfasst und das Gewichtsverhältnis des Füllstoffs zu dem SB 207266 oder dem Salz davon in den Granulatkörnern mindestens 1:3 beträgt.

96. Pharmazeutische Zusammensetzung gemäß einem der Ansprüche 81 bis 94, wobei die Granulatkörner, welche das SB 207266 oder das Salz davon enthalten, auch einen Füllstoff (Verdünnungsmittel) enthalten, wobei der Füllstoff zweibasisches Calciumphosphat (d.h. Dicalciumphosphat, Calciumhydrogenphosphat, CaHPO₄) und/oder Calciumphosphat [d.h. dreibasisches Calciumphosphat, Ca₃(PO₄)₂] umfasst und wobei der Füllstoff 10 bis 90 Gew.-% der Granulatkörner umfasst und das Gewichtsverhältnis des Füllstoffs zu dem SB 207266 oder dem Salz davon in den Granulatkörnern mindestens 1:3 beträgt.

97. Pharmazeutische Zusammensetzung nach Anspruch 95 oder 96, wobei der Füllstoff ein grobkörniger Füllstoff ist, welcher eine mittlere oder Medianteilchengröße von 53 bis 300 µm (Mikrometer) aufweist.

98. Pharmazeutische Zusammensetzung gemäß einem der Ansprüche 81 bis 97, wobei die Zusammensetzung einen Exzipienten einschließt, welcher als Verdichtungshilfsmittel wirkt, wobei das Verdichtungshilfsmittel eine mikrokristalline Cellulose ist, wobei das Verdichtungshilfsmittel zu mindestens 10 Gew.-%, bezogen auf das Gewicht der Zusammensetzung, vorhanden ist und wobei das Verdichtungshilfsmittel innerhalb der Granulatkörner (d.h. intragranular) vorhanden ist.

99. Pharmazeutische Zusammensetzung gemäß einem der Ansprüche 81 bis 98, wobei das Verfahren und/oder die Zusammensetzung wie in einem der Ansprüche 2 bis 78 definiert ist.

## Revendications

1. Procédé pour la préparation d'une composition pharmaceutique comprenant du N-[(1-ⁿbutyl-4-pipéridinyl)-méthyl]-3,4-dihydro-2H-[1,3]oxazino[3,2-a]indole-10-carboxamide (SB 207266) ou un de ses sels pharmaceutiquement acceptables en association avec un ou plusieurs excipients pharmaceutiquement acceptables,
procédé comprenant la transformation d'une partie ou de la totalité du SB 207266 ou de son sel en granules par un procédé de granulation par voie sèche,
dans lequel le SB 207266 ou son sel est présent dans la composition en une proportion d'au moins 4 % en poids de la composition.

2. Procédé suivant la revendication 1, dans lequel le procédé de granulation par voie sèche est un procédé
dans lequel des granules sont formés totalement en l'absence de solvant de granulation ajouté de l'extérieur.

3. Procédé suivant l'une quelconque des revendications précédentes, dans lequel le procédé de granulation par voie sèche comprend une compression ou un compactage du SB 207266 ou de son sel.

4. Procédé suivant l'une quelconque des revendications précédentes, dans lequel le procédé augmente le diamètre de particule du SB 207266 ou de son sel en comprimant ensemble les particules de médicament, éventuellement également avec des particules d'excipients intragranulaires, pour former des granules plus volumineux.

5. Procédé suivant l'une quelconque des revendications précédentes, dans lequel le procédé de granulation par voie sèche comprend un compactage au rouleau du SB 207266 ou de son sel.

6. Procédé suivant la revendication 5, dans lequel les rouleaux ont des surfaces circonférentielles extérieures texturées.

7. Procédé suivant la revendication 6, dans lequel les surfaces circonférentielles extérieures texturées des rouleaux comprennent des ondulations (saillies et creux) alignées généralement dans la direction de l'axe du rouleau ("rouleaux ondulés"), et dans lequel les rouleaux ondulés sont des rouleaux moletés engrenés.

8. Procédé suivant la revendication 5, 6 ou 7, dans lequel, au cours du compactage au rouleau, une poudre avant granulation à compacter est introduite dans un intervalle des rouleaux par un distributeur à vis rotative qui est adjacent aux, et dirigé vers les, rouleaux, et dans lequel l'intervalle des rouleaux est un intervalle entre deux rouleaux contrarotatifs ayant des axes longitudinaux substantiellement parallèles.

9. Procédé suivant la revendication 8, dans lequel les rouleaux sont substantiellement horizontaux et pratiquement au même niveau l'un par rapport à l'autre et dans lequel le distributeur à vis utilisé est un "distributeur à vis verticale" positionné au-dessus des et dirigé vers les rouleaux.

10. Procédé suivant la revendication 8 ou 9, dans lequel la vitesse du distributeur à vis principal adjacent aux rouleaux est d'environ 30 à environ 100 tours par minute (tr/min).

11. Procédé suivant la revendication 8, 9 ou 10, dans lequel un distributeur à vis préliminaire est utilisé pour doser le produit (le mélange avant granulation comprenant le SB 207266 ou son sel) d'une trémie d'admission dans un étage de précompression (précompactage), et dans lequel le distributeur à vis principal adjacent aux rouleaux est en aval du distributeur à vis préliminaire.

12. Procédé suivant la revendication 11, dans lequel la vitesse du distributeur à vis préliminaire est d'environ 10 tr/min à environ 40 tr/min.

13. Procédé suivant la revendication 11 ou 12, dans lequel le rapport de la vitesse du distributeur à vis préliminaire à la vitesse du distributeur à vis principal est de 1:5 à 1:1,5.

14. Procédé suivant l'une quelconque des revendications 5 à 13, dans lequel la pression du rouleau est d'environ 8000 à environ 16 500 livres par inch linéaire (pli) (c'est-à-dire d'environ 14,0 à environ 28,93 kN/cm).

15. Procédé suivant l'une quelconque des revendications 5 à 14, dans lequel la vitesse des rouleaux est d'environ 1,5 tr/min à environ 10 tr/min.

16. Procédé suivant l'une quelconque des revendications 5 à 15, dans lequel le SB 207266 ou son sel est présent dans les granules (c'est-à-dire au cours de la granulation par voie sèche) dans au moins 22 % des granules (ingrédients intragranulaires), et :
- la vitesse du distributeur à vis préliminaire, si un tel distributeur est présent, est égale au moins à environ 25 tr/min ; et/ou
- la vitesse du distributeur à vis principal est égale à au moins environ 56 tr/min ; et/ou
- la vitesse des rouleaux est d'environ 1,5 tr/min à environ 4 tr/min.

17. Procédé suivant l'une quelconque des revendications 5 à 16, dans lequel la masse volumique du ruban (c'est-à-dire le mélange compacté au rouleau lorsqu'il quitte les rouleaux) est d'environ 1,0 à environ 1,5 g/cm³ (g/ml).

18. Procédé suivant l'une quelconque des revendications 5 à 17, dans lequel le produit compact (paillettes, ruban) quittant les rouleaux est broyé à un diamètre de particule convenable pour l'utilisation dans des comprimés ou capsules.

19. Procédé suivant l'une quelconque des revendications 5 à 17, dans lequel les granules formés par le procédé de granulation par voie sèche sont broyés à un diamètre de particule convenable pour l'utilisation dans des comprimés ou capsules.

20. Procédé suivant la revendication 18 ou 19, dans lequel le broyage utilise un broyeur à comminution avec des marteaux et/ou des couteaux.

21. Procédé suivant la revendication 20, dans lequel le broyeur est un broyeur à comminution avec des "couteaux vers l'avant".

22. Procédé suivant l'une quelconque des revendications 18 à 21, dans lequel, pendant ou après le broyage, les granules sont passés à travers un tamis ayant des ouvertures des mailles de 0,110 inch (2,80 mm) à 0,032 inch (0,81 mm).

23. Procédé suivant l'une quelconque des revendications 18 à 22, dans lequel la masse volumique à l'état tassé des granules granulés et broyés secs est d'environ 0,8 à environ 1,2 g/ml.

24. Procédé suivant l'une quelconque des revendications précédentes, dans lequel, après formation des granules et un broyage facultatif à un diamètre convenable, les granules sont ensuite (i) éventuellement mélangés à un ou plusieurs excipients pharmaceutiquement acceptables (excipients extragranulaires) et (ii) transformés par compression en des comprimés ou utilisés pour remplir des capsules.

25. Procédé suivant l'une quelconque des revendications précédentes, dans lequel la composition pharmaceutique peut être administrée par voie orale.

26. Procédé suivant l'une quelconque des revendications précédentes, dans lequel la composition pharmaceutique consiste en un ou plusieurs comprimés, ou bien la composition pharmaceutique est ou est présente dans une capsule.

27. Procédé suivant l'une quelconque des revendications précédentes, dans lequel une proportion égale ou supérieure à 90 % en poids du SB 207266 ou de son sel est présente dans les granules pouvant être obtenus ou préparés (formés) par le procédé de granulation par voie sèche.

28. Procédé suivant l'une quelconque des revendications précédentes, dans lequel une proportion égale ou supérieure à 50 % en poids ou en volume des granules comprenant le SB 207266 ou son sel a un diamètre de particule supérieur ou égal à 75 micromètres.

29. Procédé suivant la revendication 28, dans lequel une proportion égale ou supérieure à 50 % en poids ou en volume des granules comprenant le SB 207266 ou son sel a un diamètre de particule supérieur ou égal à 106 micromètres.

30. Procédé suivant la revendication 28, dans lequel une proportion égale ou supérieure à 50 % en poids ou en volume des granules comprenant le SB 207266 ou son sel a un diamètre de particule supérieur de 75 à 1000 micromètres.

31. Procédé suivant l'une quelconque des revendications précédentes, dans lequel une proportion égale ou supérieure à 90 % en poids ou en volume des granules comprenant le SB 207266 ou son sel a un diamètre de particule supérieur de 105 à 1000 micromètres.

32. Procédé suivant l'une quelconque des revendications précédentes, dans lequel le N-[(1-ⁿbutyl-4-pipéridinyl)-méthyl]-3,4-dihydro-2H-[1,3]oxazino[3,2-a]indole-10-carboxamide (SB 207266) ou son sel pharmaceutiquement acceptable comprend le chlorhydrate de SB 207266.

33. Procédé suivant la revendication 32, dans lequel le N-[(1-ⁿbutyl-4-pipéridinyl)méthyl]-3,4-dihydro-2H-[1,3]-oxazino[3,2-a]indole-10-carboxamide (SB 207266) ou son sel pharmaceutiquement acceptable est le chlorhydrate de SB 207266.

34. Procédé suivant la revendication 32 ou 33, dans lequel le N-[(1-ⁿbutyl-4-pipéridinyl)méthyl]-3,4-dihydro-2H-[1,3]oxazino[3,2-a]indole-10-carboxamide (SB 207266) ou son sel pharmaceutiquement acceptable comprend la forme cristalline aciculaire du chlorhydrate du SB 207266.

35. Procédé suivant la revendication 32, 33 ou 34,
dans lequel le chlorhydrate du SB 207266 a un spectre à infrarouge (IR) (pâte avec du Nujol) essentiellement tel que représenté sur la figure 4 ; et/ou un spectre à infrarouge (IR) (pâte avec du Nujol) avec trois, quatre, cinq ou plus des pics suivants : 1628, 1582, 1502, 1531, 1184, 748 cm⁻¹ (une variation des pics de ± environ 2 cm⁻¹ étant admise).

36. Procédé suivant l'une quelconque des revendications précédentes, dans lequel une proportion égale ou supérieure à 50 % en poids ou en volume des particules du SB 207266 ou de son sel a un diamètre de particule inférieur ou égal à 53 micromètres avant transformation en granules.

37. Procédé suivant l'une quelconque des revendications précédentes, dans lequel le SB 207266 ou son sel est présent dans les granules en une proportion d'au moins 6 % en poids sur la base du poids des granules.

38. Procédé suivant l'une quelconque des revendications précédentes, dans lequel le SB 207266 ou son sel est présent dans la composition en une proportion d'au moins 6 % en poids sur la base du poids de la composition.

39. Procédé suivant l'une quelconque des revendications précédentes, dans lequel le SB 207266 ou son sel est présent dans la composition en une proportion allant jusqu'à 70 % en poids sur la base du poids de la composition.

40. Procédé suivant l'une quelconque des revendications précédentes, dans lequel la composition comprend un lubrifiant.

41. Procédé suivant la revendication 40, dans lequel le lubrifiant est présent en une proportion d'environ 0,2 à environ 5 % en poids sur la base du poids de la composition.

42. Procédé suivant la revendication 40 ou 41, dans lequel le lubrifiant est présent à la fois de manière intragranulaire et de manière extragranulaire.

43. Procédé suivant la revendication 40, 41 ou 42,
dans lequel le lubrifiant comprend le stéarate de calcium ou le stéarate de magnésium.

44. Procédé suivant l'une quelconque des revendications précédentes, comprenant le mélange d'une partie ou de la totalité du SB 207266 ou de son sel avec un ou plusieurs excipients pharmaceutiquement acceptables (excipients intragranulaires) avant granulation par voie sèche.

45. Procédé suivant la revendication 44, dans lequel le ou les excipients intragranulaires comprennent un lubrifiant.

46. Procédé suivant la revendication 45, dans lequel le lubrifiant est présent en une proportion d'environ 0,2 à environ 5 % en poids sur la base du poids des granules.

47. Procédé suivant la revendication 45 ou 46, dans lequel le lubrifiant comprend le stéarate de calcium ou le stéarate de magnésium.

48. Procédé suivant la revendication 44, 45, 46 ou 47, dans lequel le ou les excipients intragranulaires comprennent une charge (un diluant), ce qui signifie que les granules contenant le SB 207266 ou son sel contiennent également une charge (un diluant).

49. Procédé suivant la revendication 48, dans lequel la charge comprend un sel de calcium ou de magnésium pharmaceutiquement acceptable qui est insoluble, pratiquement insoluble, très légèrement soluble ou légèrement soluble dans l'eau et/ou l'éthanol,
et dans lequel le sel de calcium ou de magnésium est le phosphate de calcium, l'orthophosphate monoacide de calcium (hydrogénophosphate de calcium), le carbonate de calcium, le carbonate de magnésium, le phosphate de magnésium ou le lactate de calcium.

50. Procédé suivant la revendication 48, dans lequel la charge comprend le phosphate de calcium, c'est-à-dire le phosphate tricalcique Ca₃(PO₄)₂.

51. Procédé suivant la revendication 48 ou 50, dans lequel la charge comprend l'orthophosphate monoacide de calcium (c'est-à-dire le phosphate dicalcique, hydrogénophosphate de calcium, CaHPO₄).

52. Procédé suivant la revendication 48, dans lequel la charge est l'orthophosphate monoacide de calcium (c'est-à-dire le phosphate dicalcique, hydrogénophosphate de calcium, CaHPO₄).

53. Procédé suivant la revendication 51 ou 52, dans lequel la charge comprend le dihydrate d'hydrogénophosphate de calcium (CaHPO₄.2H₂O).

54. Procédé suivant l'une quelconque des revendications 48 à 53, dans lequel la charge est une charge de qualité grossière ayant un diamètre moyen ou médian de particule de 53 à 300 µm (micromètres).

55. Procédé suivant l'une quelconque des revendications 48 à 54, dans lequel la charge représente 10 à 90 % en poids des granules.

56. Procédé suivant l'une quelconque des revendications 48 à 55, dans lequel la charge est présente en une proportion de 15 à 85 % en poids de la composition.

57. Procédé suivant l'une quelconque des revendications 48 à 56, dans lequel le rapport pondéral de la charge au SB 207266 ou à son sel dans les granules est égal au moins à 1:3.

58. Procédé suivant l'une quelconque des revendications 48 à 56, dans lequel la charge est au moins partiellement présente de manière intragranulaire (c'est-à-dire est présente au cours du procédé de granulation par voie sèche), et le rapport intragranulaire de la charge au SB 207266 ou à son sel (c'est-à-dire le rapport au cours de la granulation par voie sèche) est de 1:3 à 10:1.

59. Procédé suivant l'une quelconque des revendications précédentes, dans lequel la composition comprend un excipient qui joue le rôle d'adjuvant de compression.

60. Procédé suivant la revendication 59, dans lequel l'adjuvant de compression comprend la cellulose microcristalline.

61. Procédé suivant la revendication 60, dans lequel l'adjuvant de compression est la cellulose microcristalline.

62. Procédé suivant la revendication 60 ou 61, dans lequel la cellulose microcristalline a un diamètre moyen nominal de particule d'environ 25 à environ 150 µm (micromètres) .

63. Procédé suivant la revendication 59, 60, 61 ou 62, dans lequel l'adjuvant de compression est présent en une proportion d'au moins 10 % en poids sur la base du poids de la composition.

64. Procédé suivant la revendication 59, 60, 61, 62 ou 63, dans lequel l'adjuvant de compression est présent en une proportion d'au moins 10 % en poids sur la base du poids des granules.

65. Procédé suivant l'une quelconque des revendications 59 à 64, dans lequel l'adjuvant de compression est présent en une proportion de 10 à 50 % en poids sur la base du poids de la composition.

66. Procédé suivant l'une quelconque des revendications 59 à 65, dans lequel l'adjuvant de compression est présent à l'intérieur des granules (c'est-à-dire de manière intragranulaire).

67. Procédé suivant l'une quelconque des revendications 59 à 66, dans lequel le rapport pondéral intragranulaire, c'est-à-dire le rapport pondéral au cours du procédé de granulation par voie sèche, de la charge à l'adjuvant de compression est de 5:1 à 2:3.

68. Procédé suivant l'une quelconque des revendications précédentes, dans lequel la composition ne comprend aucun liant.

69. Procédé suivant l'une quelconque des revendications précédentes, dans lequel la composition comprend un agent de délitement présent en une proportion d'environ 1 à environ 10 % en poids de la composition.

70. Procédé suivant l'une quelconque des revendications précédentes, dans lequel les granules (c'est-à-dire les ingrédients intragranulaires) représentent environ 75 % à environ 99 % en poids de la composition.

71. Procédé suivant l'une quelconque des revendications 1 à 69, dans lequel les granules (c'est-à-dire les ingrédients intragranulaires) représentent 100 % en poids de la composition (ce qui signifie que les excipients extragranulaires sont absents).

72. Procédé suivant l'une quelconque des revendications 1 à 71, dans lequel les granules contenant le SB 207266 ou son sel contiennent également une charge (un diluant), la charge comprenant de l'orthophosphate monoacide de calcium (c'est-à-dire le phosphate dicalcique, l'hydrogénophosphate de calcium, CaHPO₄) et/ou du phosphate de calcium [c'est-à-dire le phosphate tricalcique, Ca₃(PO₄)₂],
et dans lequel la charge représente 10 à 90 % en poids des granules, et le rapport pondéral de la charge au SB 207266 ou à son sel dans les granules est égal à au moins 1:3.

73. Procédé pour la préparation d'une composition pharmaceutique comprenant du N-[(1-ⁿbutyl-4-pipéridinyl)-méthyl]-3,4-dihydro-2H-[1,3]oxazino[3,2-a]indole-10-carboxamide (SB 207266) ou un de ses sels pharmaceutiquement acceptables en association avec un ou plusieurs excipients (supports) pharmaceutiquement acceptables, dans lequel le SB 207266 ou son sel est présent dans la composition en une proportion d'au moins 4 % en poids sur la base du poids de la composition,
procédé comprenant :
(a) la dissolution du SB 207266 ou de son sel dans de l'éthanol ou un solvant contenant de l'éthanol pour former une solution,
(b) la cristallisation du SB 207266 ou de son sel à partir de la solution par addition d'un hydrocarbure en C₅ à C₁₀ et/ou d'un solvant contenant un hydrocarbure en C₅ à C₁₀, et
(c) la transformation d'au moins une certaine quantité du SB 207266 ou de son sel en des granules par un procédé de granulation par voie sèche.

74. Procédé suivant la revendication 73, dans lequel le solvant contenant de l'éthanol consiste en alcool ordinaire dénaturé.

75. Procédé suivant la revendication 73 ou 74, dans lequel l'hydrocarbure en C₅ à C₁₀ est l'hexane et/ou l'heptane.

76. Procédé suivant la revendication 73, 74 ou 75, dans lequel le N-[(1-ⁿbutyl-4-pipéridinyl)-méthyl]-3,4-dihydro-2H-[1,3]oxazino[3,2-a]indole-10-carboxamide (SB 207266) ou son sel pharmaceutiquement acceptable comprend le chlorhydrate du SB 207266.

77. Procédé suivant la revendication 73, 74, 75 ou 76, dans lequel le procédé de granulation par voie sèche et/ou le ou les excipients sont tels que décrits dans l'une quelconque des revendications 2 à 71.

78. Procédé suivant la revendication 73, 74, 75, 76 ou 77, dans lequel les granules contenant le SB 207266 ou son sel contiennent également une charge (un diluant), la charge comprenant de l'orthophosphate monoacide de calcium (c'est-à-dire le phosphate dicalcique, hydrogénophosphate de calcium, CaHPO₄) et/ou du phosphate de calcium [c'est-à-dire le phosphate tricalcique, Ca₃(PO₄)₂], et dans lequel la charge représente 10 à 90 % en poids des granules, et le rapport pondéral de la charge au SB 207266 ou à son sel dans les granules est égal à au moins 1:3.

79. Composition pharmaceutique pouvant être obtenue par un procédé tel que défini dans l'une quelconque des revendications 1 à 78.

80. Composition pharmaceutique qui a été préparée par un procédé tel que défini dans l'une quelconque des revendications 1 à 78.

81. Composition pharmaceutique comprenant du N-[(1-ⁿbutyl-4-pipéridinyl)méthyl]-3,4-dihydro-2H-[1,3]oxazino[3,2-a]indole-10-carboxamide (SB 207266) ou un de ses sels pharmaceutiquement acceptables en association avec un ou plusieurs excipients pharmaceutiquement acceptables,
dans laquelle une partie ou la totalité du SB 207266 ou de son sel est présente dans les granules pouvant être obtenus par un procédé de granulation par voie sèche,
et dans laquelle le SB 207266 ou son sel est présent dans la composition en une proportion d'au moins 4 % en poids sur la base du poids de la composition.

82. Composition pharmaceutique comprenant du N-[(1-ⁿbutyl-4-pipéridinyl)méthyl]-3,4-dihydro-2H-[1,3]oxazino[3,2-a]indole-10-carboxamide (SB 207266) ou un de ses sels pharmaceutiquement acceptables en association avec un ou plusieurs excipients pharmaceutiquement acceptables,
dans laquelle une partie ou la totalité du SB 207266 ou de son sel est présente dans les granules préparés par un procédé de granulation par voie sèche,
et dans laquelle le SB 207266 ou son sel est présent dans la composition en une proportion d'au moins 4 % en poids sur la base du poids de la composition.

83. Composition pharmaceutique suivant la revendication 81 ou 82, dans laquelle le procédé de granulation par voie sèche comprend un compactage au rouleau du SB 207266 ou de son sel.

84. Composition pharmaceutique suivant la revendication 83, dans laquelle, dans le procédé, le produit compacté (paillettes, ruban) quittant les rouleaux est broyé à un diamètre de particule convenable pour l'utilisation dans des comprimés ou capsules.

85. Composition pharmaceutique suivant la revendication 81, 82, 83 ou 84, la composition pharmaceutique consistant en un ou plusieurs comprimés, ou bien la composition pharmaceutique étant ou étant présente dans une capsule.

86. Composition pharmaceutique suivant l'une quelconque des revendications 81 à 85, dans laquelle une proportion égale ou supérieure à 90 % en poids du SB 207266 ou de son sel est présente dans les granules pouvant être obtenus ou préparés (formés) par le procédé de granulation par voie sèche.

87. Composition pharmaceutique suivant l'une quelconque des revendications 81 à 86, dans laquelle une proportion égale ou supérieure à 50 % en poids ou en volume des granules comprenant le SB 207266 ou son sel a un diamètre de particule supérieur ou égal à 75 micromètres.

88. Composition pharmaceutique suivant l'une quelconque des revendications 81 à 87, dans laquelle le N-[(1-ⁿbutyl-4-pipéridinyl)méthyl]-3,4-dihydro-2H-[1,3]oxazino[3,2-a]indole-10-carboxamide (SB 207266) ou son sel pharmaceutiquement acceptable comprend le chlorhydrate du SB 207266.

89. Composition pharmaceutique suivant la revendication 88, dans laquelle le N-[(1-ⁿbutyl-4-pipéridinyl)méthyl]-3,4-dihydro-2H-[1,3]oxazino[3,2-a]indole-10-carboxamide (SB 207266) ou son sel pharmaceutiquement acceptable est le chlorhydrate du SB 207266.

90. Composition pharmaceutique suivant la revendication 88 ou 89, dans laquelle le N-[(1-ⁿbutyl-4-pipéridinyl)-méthyl]-3,4-dihydro-2H-[1,3]oxazino[3,2-a]indole-10-carboxamide (SB 207266) ou son sel pharmaceutiquement acceptable comprend la forme cristalline aciculaire du chlorhydrate du SB 207266.

91. Composition pharmaceutique suivant la revendication 88, 89 ou 90, dans laquelle le chlorhydrate du SB 207266 a un spectre à infrarouge (IR) (pâte avec du Nujol) essentiellement tel que représenté sur la figure 4 ; et/ou un spectre à infrarouge (IR) (pâte avec du Nujol) avec trois, quatre, cinq ou plus des pics suivants : 1628, 1582, 1502, 1531, 1184, 748 cm⁻¹ (une variation des pics de ± environ 2 cm⁻¹ étant admise).

92. Composition pharmaceutique suivant l'une quelconque des revendications 81 à 91, dans laquelle le SB 207266 ou son sel est présent dans les granules en une proportion d'au moins 6 % en poids sur la base du poids des granules.

93. Composition pharmaceutique suivant l'une quelconque des revendications 81 à 92, dans laquelle le SB 207266 ou son sel est présent dans la composition en une proportion d'au moins 6 % en poids sur la base du poids de la composition.

94. Composition pharmaceutique suivant l'une quelconque des revendications 81 à 93, la composition comprenant un lubrifiant présent à la fois de manière intragranulaire et de manière extragranulaire, dans laquelle le lubrifiant est présent en une proportion de 0,2 à 5 % en poids sur la base du poids de la composition.

95. Composition pharmaceutique suivant l'une quelconque des revendications 81 à 94, dans laquelle les granules contenant le SB 207266 ou son sel contiennent également une charge (un diluant),
dans laquelle la charge comprend un sel de calcium ou de magnésium pharmaceutiquement acceptable qui est insoluble, pratiquement insoluble, très légèrement soluble ou légèrement soluble dans l'eau et/ou l'éthanol,
dans lequel le sel de calcium ou de magnésium est le phosphate de calcium, l'orthophosphate monoacide de calcium (hydrogénophosphate de calcium), le carbonate de calcium, le carbonate de magnésium, le phosphate de magnésium ou le lactate de calcium,
et dans laquelle la charge représente 10 à 90 % en poids des granules, et le rapport pondéral de la charge au SB-207266 ou à son sel dans les granules est égal à au moins 1:3.

96. Composition pharmaceutique suivant l'une quelconque des revendications 81 à 94, dans laquelle les granules contenant le SB 207266 ou son sel contiennent également une charge (un diluant), la charge comprenant de l'orthophosphate monoacide de calcium (c'est-à-dire le phosphate dicalcique, hydrogénophosphate de calcium, CaHPO₄) et/ou du phosphate de calcium [c'est-à-dire le phosphate tricalcique, Ca₃(PO₄)₂],
et dans laquelle la charge représente 10 à 90 % en poids des granules, et le rapport pondéral de la charge au SB-207266 ou à son sel dans les granules est égal au moins à 1:3.

97. Composition pharmaceutique suivant la revendication 95 ou 96, dans laquelle la charge est une charge de qualité grossière ayant un diamètre moyen ou médian de particule de 53 à 300 µm (micromètres).

98. Composition pharmaceutique suivant l'une quelconque des revendications 81 à 97, la composition comprenant un excipient qui joue le rôle d'adjuvant de compression, dans laquelle l'adjuvant de compression est la cellulose microcristalline, dans laquelle l'adjuvant de compression est présent en une proportion d'au moins 10 % en poids sur la base du poids de la composition, et dans laquelle l'adjuvant de compression est présent à l'intérieur des granules (c'est-à-dire de manière intragranulaire).

99. Composition pharmaceutique suivant l'une quelconque des revendications 81 à 98, dans laquelle le procédé et/ou la composition est tel que défini dans l'une quelconque des revendications 2 à 78.
